# EUROPEAN PATENT SPECIFICATION

(11) **EP 3 962 942 B1**
(45) Date of publication and mention of the grant of the patent: **22.10.2025**
(21) Application number: 20728590.9
(22) Date of filing: 30.04.2020
(51) Int. Cl.: C07K 16/24, A61K 39/395, A61K 47/18, A61K 47/26

(54) **ANTI-IL-6 ANTIBODY FORMULATION**
ANTI-IL-6-ANTIKÖRPERFORMULIERUNG
FORMULATIONS D'ANTICORPS ANTI-IL-6

(30) Priority: 01.05.2019 US 201962841662 P; 19.06.2019 EP 19181345
(43) Date of publication of application: 09.03.2022
(60) Divisional application: 25169130.9
(73) Proprietor: Novo Nordisk A/S, 2880 Bagsværd (DK)
(72) Inventor: ZHU, Gaozhong, 2880 Bagsværd (DK); SHAHROKH, Zahra, 2880 Bagsværd (DK); MELVILLE, Mark, 2880 Bagsværd (DK); DEVALARAJA, Madhav, N., 2880 Bagsværd (DK)
(86) International application number: PCT/US2020/030865
(87) International publication number: WO 2020/223565

(56) References cited:
- WO-A1-2014/036071
- WO-A1-2014/066468
- WO-A2-2011/161226
- HADA SHAVRON ET AL: "Evaluation of antioxidants in protein formulation against oxidative stress using various biophysical methods", INTERNATIONAL JOURNAL OF BIOLOGICAL MACROMOLECULES, ELSEVIER BV, NL, vol. 82, 21 October 2015 (2015-10-21), pages 192 - 200, XP029357951, ISSN: 0141-8130, DOI: 10.1016/J.IJBIOMAC.2015.10.048
- NICHOLAS W WARNE ED - LENDLEIN ANDREAS ET AL: "Development of high concentration protein biopharmaceuticals: The use of platform approaches in formulation development", EUROPEAN JOURNAL OF PHARMACEUTICS AND BIOPHARMACEUTICS, ELSEVIER SCIENCE PUBLISHERS B.V., AMSTERDAM, NL, vol. 78, no. 2, 3 March 2011 (2011-03-03), pages 208 - 212, XP028203394, ISSN: 0939-6411, [retrieved on 20110313], DOI: 10.1016/J.EJPB.2011.03.004

## Description

### 1. CROSS REFERENCE TO RELATED APPLICATIONS

This application claims priority to U.S. Provisional Application No. 62/841,662, filed May 1, 2019, and European Patent Application No. 19181345.0, filed June 19, 2019.

### 3. BACKGROUND

The elevation of IL-6 has been implicated in a variety of diseases and conditions, such as autoimmune diseases, inflammatory diseases and cancer. *See* US5856135, WO2004/020633, US20060257407A1, US7291721, and US8198414. Antibodies that reduce IL-6 signaling by binding to and neutralizing IL-6 or by binding to the IL-6 receptor have been approved for treating rheumatoid arthritis and Castleman's disease. More recently, anti-IL6 antibodies have been demonstrated to be useful in the treatment of hepcidin-mediated disorders in genotypically selected patients, *see* US20170029499A1; in treatment of diuretic resistance, *see* WO 2018/144773; and in treatment of inflammatory cardiovascular disease, *see* US 2019/0241650.

WO2014/066468 (MedImmune LLC) relates to an anti-IL-6 antibody formulation. WO 2011/161226 (Hoffmann LaRoche) relates to an antibody formulation. Hada Shavron et al. 2015 relates to protein formulations. The therapeutic use of anti-IL-6 antibodies is facilitated by formulations that retain stability of the antibodies under a variety of conditions. It is important that the therapeutic formulation permits storage without an unacceptable loss of activity of the active antibody, minimizes the accumulation of undesirable products such as aggregates or degraded species (e.g., fragmented, oxidized, deamidated or isomerized species), accommodates appropriate concentrations of the antibody, and does not contain components that are incompatible with therapeutic applications.

There is a need in the art for a stable aqueous pharmaceutical formulation comprising an anti-IL-6 antibody, which is suitable for therapeutic use.

### 4. SUMMARY

We have designed, produced, and tested an anti-IL-6 antibody formulation that have reduced formation of aggregates, acidic species, and oxidized species. The anti-IL-6 antibody formulation can be used for treating IL-6 mediated diseases.

An antibody formulation is described herein. The antibody formulation described herein comprises: about 5 mg/mL to about 120 mg/mL of an anti-IL-6 antibody and about 5 mM to about 15 mM methionine.

Described herein is an anti-IL-6 antibody that comprises a heavy chain variable (VH) domain and a light chain variable (VL) domain, wherein the VH domain comprises: the VH CDR1 sequence of SEQ ID NO: 1; the VH CDR2 sequence of SEQ ID NO: 2; and the VH CDR3 sequence of SEQ ID NO: 3; and wherein the VL domain comprises: the VL CDR1 sequence of SEQ ID NO: 4; the VL CDR2 sequence of SEQ ID NO: 5; and the VL CDR3 sequence of SEQ ID NO: 6. An anti-IL-6 antibody described herein comprises the VH domain amino acid sequence of SEQ ID NO: 7 and the VL domain amino acid sequence of SEQ ID NO: 8. An anti-IL-6 antibody described herein comprises the heavy chain amino acid sequence of SEQ ID NO: 9 and the light chain amino acid sequence of SEQ ID NO: 10.

Also described herein is a formulation that comprises about 7.5 mg/mL to about 30 mg/mL of the anti-IL-6 antibody. A formulation described herein comprises about 7.5 mg/mL of the anti-IL-6 antibody. In some embodiments, the formulation comprises about 15 mg/mL of the anti-IL-6 antibody. In some embodiments, the formulation comprises about 30 mg/mL of the anti-IL-6 antibody.

In some embodiments, the formulation comprises about 10 mM methionine.

Also described herein is formulation that comprises about 0.03% to about 0.1% (w/v) polysorbate 80. Also described herein is a formulation that comprises about 0.05% to about 0.1% polysorbate 80 (w/v). In some embodiments, the formulation comprises about 0.07% (w/v) polysorbate 80.

Also described herein is a formulation that comprises further about 1% to about 40% (w/v) trehalose. In some embodiments, the formulation comprises about 5% (w/v) trehalose.

Also described herein is a formulation that further comprises about 10 mM to about 200 mM arginine. In some embodiments, the formulation comprises about 70 mM arginine.

Also described herein is formulation further comprises about 10 mM to about 100 mM histidine. In some embodiments, the formulation comprises about 20 mM histidine.

Also described herein is a antibody formulation that has a pH of about 5.0 to about 7.0. In some embodiments, the antibody formulation has a pH of about 6.0.

Also described herein is an antibody formulation comprising: about 5 mg/mL to about 120 mg/mL of an anti-IL-6 antibody, about 5 mM to about 15 mM methionine, and about 0.03% to about 0.1% (w/v) polysorbate 80. In some embodiments, the anti-IL-6 antibody has the heavy chain amino acid sequence of SEQ ID NO: 9 and the light chain amino acid sequence of SEQ ID NO: 10.

Also described herein is an antibody formulation comprising: a) about 5 mg/mL to about 120 mg/mL of an anti-IL-6 antibody; b) about 1% to about 40% (w/v) trehalose; c) about 0.03% to about 0.1% (w/v) polysorbate 80; d) about 10 mM to about 200 mM arginine; e) about 5 mM to about 15 mM methionine; and f) about 10 mM to about 100 mM histidine; wherein the antibody formulation has a pH of about 5.0 to about 7.0. In some embodiments, the anti-IL-6 antibody has the heavy chain amino acid sequence of SEQ ID NO: 9 and the light chain amino acid sequence of SEQ ID NO: 10.

Also described herein is an antibody formulation comprising: a) about 5 mg/mL to about 120 mg/mL of an anti-IL-6 antibody, wherein the anti-IL-6 antibody has the heavy chain amino acid sequence of SEQ ID NO: 9 and the light chain amino acid sequence of SEQ ID NO: 10; b) about 5% (w/v) trehalose; c) about 0.07% (w/v) polysorbate 80; d) about 70 mM arginine; e) about 10 mM methionine; and f) about 20 mM histidine; wherein the antibody formulation has a pH of about 6.0.

In some embodiments, the formulation comprises about 7.5 mg/mL of the anti-IL-6 antibody. In some embodiments, the formulation comprises about 15 mg/mL of the anti-IL-6 antibody. In some embodiments, the formulation comprises about 30 mg/mL of the anti-IL-6 antibody.

In some embodiments, the formulation has a viscosity of less than 10 cP at 25°C.

In some embodiments, the formulation has less than 5% soluble aggregates after 20 hours of agitation at 300 rpm, as measured by size exclusion-high-performance liquid chromatography (SEC-HPLC).

In some embodiments, the formulation has less than 50% acidic species after incubation at 45°C for two weeks, as measured by imaging detection capillary isoelectric focusing (icIEF). In some embodiments, the acidic species being measured are generated by deamidation, isomerization, oxidation or degradation.

In some embodiments, the formulation has less than 5% oxidized species after incubation at 45°C for two weeks, as measured by reversed phase-high performance liquid chromatography (RP-HPLC).

In some embodiments, the formulation has less than 50% charged variants after storage at 5 + 3°C for 12 months, as measured by imaging detection capillary isoelectric focusing (icIEF). In some embodiments, the formulation has less than 50% acidic species after storage at 5 + 3°C for 12 months, as measured by imaging detection capillary isoelectric focusing (icIEF).

In some embodiments, the formulation has less than 10% oxidized species after storage at 5 + 3°C for 12 months, as measured by reversed phase-high performance liquid chromatography (RP-HPLC). In some embodiments, the formulation has less than 6% oxidized species after storage at 5 + 3°C for 12 months, as measured by reversed phase-high performance liquid chromatography (RP-HPLC).

In some embodiments, the formulation has less than 50% reduction in potency after storage at 5 + 3°C for 12 months, as measured by IL-6 binding ELISA. In some embodiments, the formulation has less than 30% reduction in potency after storage at 5 + 3°C for 12 months, as measured by IL-6 binding ELISA. In some embodiments, the formulation has less than 50% reduction in potency after storage at 5 + 3°C for 12 months, as measured by HEK Blue cell-based bioassay. In some embodiments, the formulation has less than 30% reduction in potency after storage at 5 + 3°C for 12 months, as measured by HEK Blue cell-based bioassay.

In some embodiments, the formulation is suitable for parenteral administration. In some embodiments, the formulation is suitable for intravenous administration. In some embodiments, the formulation is suitable for subcutaneous administration.

Also described herein is a unit dosage form comprising the antibody formulation. In some embodiments, the unit dosage form comprises about 7.5 mg of the anti-IL-6 antibody. In some embodiments, the unit dosage form comprises about 15 mg of the anti-IL-6 antibody. In some embodiments, the unit dosage form comprises about 30 mg of the anti-IL-6 antibody.

### 5. BRIEF DESCRIPTION OF THE DRAWINGS

These and other features, aspects, and advantages of the present invention will become better understood with regard to the following description, and accompanying drawings, where:
**FIG. 1** shows the effect of L-arginine-HCl on COR-001 formulation viscosity at 25°C with increasing protein concentration.
**FIG. 2** shows the effect of polysorbate 80 on COR-001 formulation aggregation upon agitation. The concentrations of COR-001 were 5 mg/mL, 50 mg/mL, and 120 mg/mL respectively.

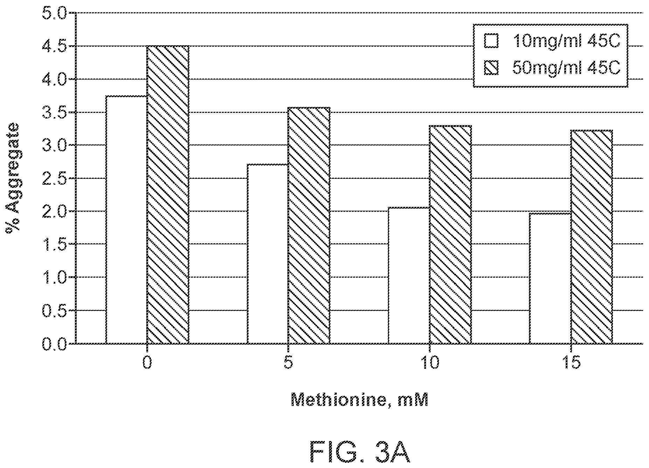

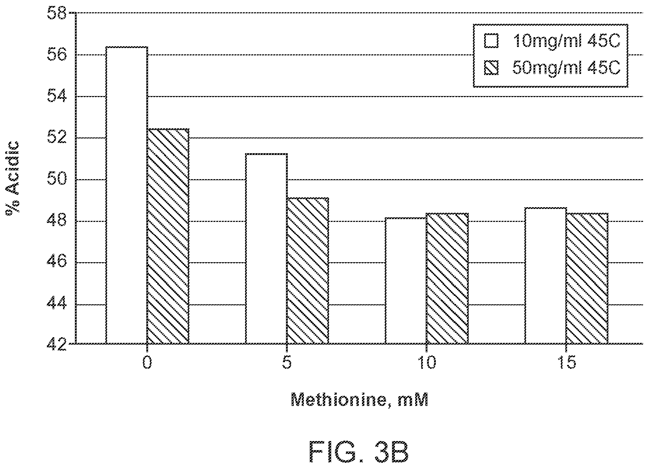

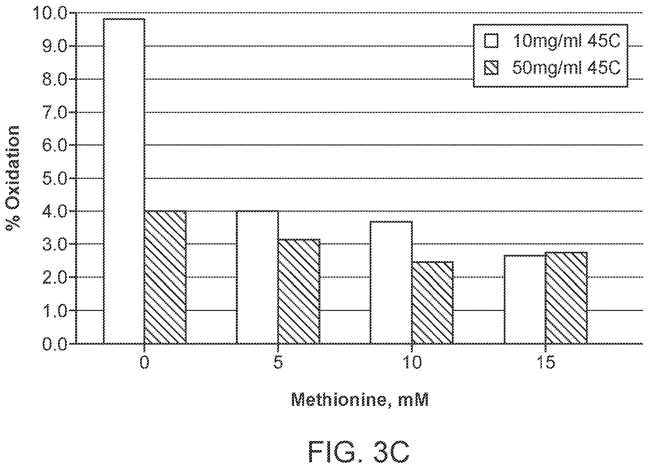

**FIGs. 3A****,** **3B****, and** **3C** show the effect of methionine on COR-001 formulation stability upon thermal stress, with **FIG. 3A** showing the percentage of aggregates of COR-001 formulation at 45°C in the absence or in the presence of 5 mM, 10 mM, or 15 mM methionine as measured by SEC-HPLC, **FIG. 3B** showing the percentage of acidic species of COR-001 formulation at 45°C in the absence or in the presence of 5 mM, 10 mM, or 15 mM methionine as measured by icIEF, and **FIG. 3C** showing the percentage of oxidized species of COR-001 formulation at 45°C in the absence or in the presence of 5 mM, 10 mM, or 15 mM methionine as measured by oxidation testing. The concentrations of COR-001 were 10 mg/mL and 50 mg/mL, respectively.
**FIGs. 4A and 4B** show the pH change over time under accelerated and stress conditions, respectively, with **FIG. 4A** showing the pH change over time under the accelerated condition and **FIG. 4B** showing the pH change over time under the stress condition.
**FIGs. 5A and 5B** show the polysorbate 80 change over time under accelerated and stress conditions, with **FIG. 5A** showing the polysorbate 80 change over time under the accelerated condition and **FIG. 5B** showing polysorbate 80 change over time under the stress condition.
**FIGs. 6A and 6B** show the protein concentration change over time under accelerated and stress conditions, with **FIG. 6A** showing the protein concentration change over time under the accelerated condition and **FIG. 6B** showing the protein concentration change over time under the stress condition.
**FIGs. 7A and 7B** show the change of monomer (non-aggregated bivalent full length IgG antibody) over time under accelerated and stress conditions measured by SEC-UHPLC, with **FIG. 7A** showing monomer% change over time under the accelerated condition and **FIG. 7B** showing monomer% change over time under the stress condition.
**FIGs. 8A and 8B** show the change of HMW over time under accelerated and stress conditions measured by SEC-UHPLC, with **FIG. 8A** showing HMW% change over time under the accelerated condition and **FIG. 8B** showing HMW% change over time under the stress condition.
**FIGs. 9A and 9B** show the change of LMW over time under accelerated and stress conditions measured by SEC-UHPLC, with **FIG. 9A** showing LMW% change over time under the accelerated condition and **FIG. 9B** showing LMW% change over time under the stress condition.
**FIGs. 10A and 10B** show the change of IgG over time under accelerated and stress conditions measured by nonreduced CE-SDS, with **FIG. 10A** showing IgG% change over time under the accelerated condition and **FIG. 10B** showing IgG% change over time under the stress condition.
**FIGs. 11A and 11B** show the change of HHL over time under accelerated and stress conditions measured by nonreduced CE-SDS, with **FIG. 11A** showing HHL% change over time under the accelerated condition and **FIG. 11B** showing HHL% change over time under the stress condition.
**FIGs. 12A and 12B** show the change of HC+LC over time under accelerated and stress conditions measured by reduced CE-SDS, with **FIG. 12A** showing HC+LC% change over time under the accelerated condition and **FIG. 12B** showing HC+LC% change over time under the stress condition.
**FIGs. 13A and 13B** show the change of main species over time under accelerated and stress conditions measured by icIEF, with **FIG. 13A** showing main species% change over time under the accelerated condition and **FIG. 13B** showing main species% change over time under the stress condition.
**FIGs. 14A and 14B** show the change of acidic species over time under accelerated and stress conditions measured by icIEF, with **FIG. 14A** showing acidic species% change over time under the accelerated condition and **FIG. 14B** showing acidic species% change over time under the stress condition.
**FIGs. 15A and 15B** show the change of basic species over time under accelerated and stress conditions measured by icIEF, with **FIG. 15A** showing basic species% change over time under the accelerated condition and **FIG. 15B** showing basic species% change over time under the stress condition.
**FIGs. 16A and 16B** show the change in level of oxidation over time under accelerated and stress conditions measured by RP-HPLC, with **FIG. 16A** showing oxidation% change over time under the accelerated condition and **FIG. 16B** showing oxidation% change over time under the stress condition.
**FIGs. 17A, 17B, and 17C** show the change in level of sub-visible particles over time under the accelerated condition measured by MFI, with **FIG. 17A** showing the change of ≥ 2 micron particles over time, **FIG. 17B** showing the change of ≥ 10 micron particles over time, and **FIG. 17C** showing the change of ≥ 25 micron particles over time.
**FIGs. 18A and 18B** show the change in antibody potency over time under accelerated and stress conditions measured by IL-6 binding ELISA assay, with **FIG. 18A** showing the change in IL-6 binding% compared to reference standard over time under the accelerated condition and **FIG. 18B** showing the change in IL-6 binding% compared to reference standard over time under the stress condition.
**FIGs. 19A and 19B** show the change in antibody potency over time under accelerated and stress conditions measured by HEK Blue cell-based bioassay, with **FIG. 19A** showing the change in IL-6 binding% compared to reference standard over time under the accelerated condition and **FIG. 19B** showing the change in IL-6 binding% compared to reference standard over time under the stress condition.
**FIG. 20** shows the pH change over time under the long term storage condition.
**FIG. 21** shows the polysorbate 80 change over time under the long term storage condition.
**FIG. 22** shows the protein concentration change over time under the long term storage condition.

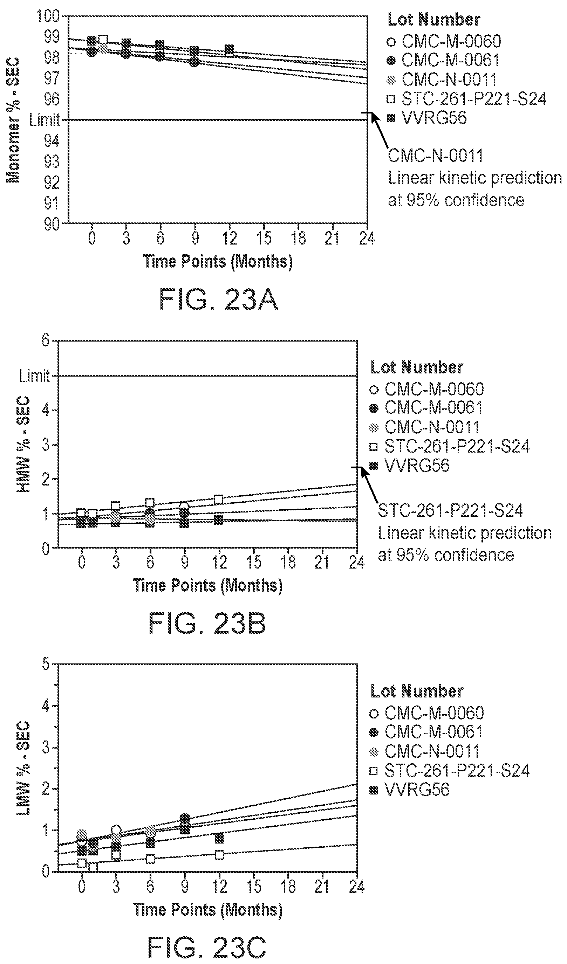

**FIGs. 23A, 23B, and 23C** show changes of monomer (non-aggregated bivalent full length IgG antibody), HMW, and LMW by SEC over time under the long term storage condition, with **FIG. 23A** showing monomer% change over time (the arrow points to the boundary of monomer% at 24 months with linear kinetic prediction at 95% confidence for all lots, with the indicated lot being the one that is the closest to the limit), **FIG. 23B** showing HMW% change over time (the arrow points to the boundary of HMW% at 24 months with linear kinetic prediction at 95% confidence for all lots, with the indicated lot being the one that is the closest to the limit), and **FIG. 23C** showing LMW% change over time.
**FIG. 24** shows an overlay of chromatographic profiles of Lot CMC-M-0061 comparing the changes under the long term storage condition with changes under the accelerated condition (25°C) and the stress condition (40°C) at 3 months.
**FIGs. 25A and 25B** show changes of IgG and HHL by nonreduced CE-SDS over time under the long term storage condition, with **FIG. 25A** showing IgG% change over time (the arrow points to the boundary of IgG% at 24 months with linear kinetic prediction at 95% confidence for all lots, with the indicated lot being the one that is the closest to the limit) and **FIG. 25B** showing HHL% change over time.
**FIG. 26** shows an overlay of electrophoretic profiles of Lot CMC-M-0061 comparing the changes under the long term storage condition (5°C) with changes under the accelerated condition (25°C) and the stress condition (40°C) at 3 months.
**FIG. 27** shows the change of HC+LC by reduced CE-SDS over time under the long term storage condition (the arrow points to the boundary of HC+LC% at 24 months with linear kinetic prediction at 95% confidence for all lots, with the indicated lots being the ones that are beyond the limit).
**FIG. 28** shows an overlay of electrophoretic profiles of Lot CMC-M-0061 comparing the changes under the long term storage condition (5°C) with changes under the accelerated condition (25°C) and the stress condition (40°C) at 3 months.
**FIGs. 29A, 29B, and 29C** show changes of main species, acidic species, and basic species by icIEF over time under the long term storage condition, with **FIG. 29A** showing main species% change over time, **FIG. 29B** showing acidic species% change over time, and **FIG. 29C** showing basic species% change over time.
**FIG. 30** shows an overlay of electrophoretic profiles of Lot CMC-M-0061 comparing the changes under the long term storage condition (5°C) with changes under the accelerated condition (25°C) and the stress condition (40°C) at 3 months.
**FIG. 31** shows the change in level of oxidation over time under the long term storage condition measured by RP-HPLC.
**FIGs. 32A and 32B** show the change in level of sub-visible particles over time under the long term storage condition measured by HIAC, with **FIG. 32A** showing the change of ≥ 10 micron particles over time and **FIG. 32B** showing the change of ≥ 25 micron particles over time.
**FIGs. 33A, 33B, and 33C** show the change in level of sub-visible particles over time under the long term storage condition measured by MFI, with **FIG. 33A** showing the change of ≥ 2 micron particles over time, **FIG. 33B** showing the change of ≥ 10 micron particles over time, and **FIG. 33C** showing the change of ≥ 25 micron particles over time.
**FIGs. 34A and 34B** show the change in antibody potency over time under the long term storage condition, with **FIG. 34A** showing the change in antibody potency measured by IL-6 binding ELISA and **FIG. 34B** showing the change in antibody potency measured by HEK Blue Bioassay.

### 6. DETAILED DESCRIPTION

### 6.1. Definitions

Unless defined otherwise, all technical and scientific terms used herein have the meaning commonly understood by one of ordinary skill in the art to which the invention pertains.

A **"stable"** antibody formulation is one in which the antibody substantially retains its physical stability and/or chemical stability and/or its biological activity upon storage. The storage period is generally selected based on the intended shelf-life of the formulation. Various analytical techniques for measuring protein stability are available in the art. Examples of the analytical techniques are described below. By **"substantially retains"** is intended 85% or greater retention, such as at least 90% retention or at least 95% retention.

A protein **"retains its physical stability"** in a pharmaceutical formulation if it shows no significant physical changes such as aggregation, precipitation and/or denaturation upon visual examination of color and/or clarity, or as measured by UV light scattering or by size exclusion chromatography.

A protein **"retains its chemical stability"** in a pharmaceutical formulation if no significant chemical changes of the protein is shown. Chemical stability can be assessed by detecting and quantifying chemically altered forms of the protein. Chemical alteration may involve size modification (e.g. clipping) which can be evaluated using size exclusion chromatography, SDS-PAGE and/or matrix-assisted laser desorption ionization/time-of-flight mass spectrometry (MALDI/TOF MS), for example. Other types of chemical alteration include charge alteration (e.g. occurring as a result of deamidation, oxidation and/or isomerization) which can be evaluated by ion-exchange chromatography, for example.

An antibody **"retains its biological activity"** in a pharmaceutical formulation if the biological activity of the antibody at a given time is not significantly changed from the biological activity exhibited at the time the pharmaceutical formulation was prepared as determined in an antigen binding assay, for example. **"Biological activity"** of a monoclonal antibody refers to the ability of the antibody to bind to antigen and result in a measurable biological response which can be measured *in vitro* or *in vivo.* Such activity may be antagonistic or agonistic.

A **"histidine buffer"** is a buffer comprising histidine ions. Examples of histidine buffers include histidine chloride, histidine acetate, histidine phosphate, and histidine sulfate solutions. The histidine buffer or histidine-HCl buffer has a pH of about 5.5 to about 6.5, about 5.6 to about 6.4, about 5.7 to about 6.3, about 5.8 to about 6.2, about 5.9 to about 6.1, or about 6.0.

The term **"antibody"** is used in the broadest sense and specifically covers monoclonal antibodies (including full-length monoclonal antibodies), polyclonal antibodies, multispecific antibodies (e.g., bispecific antibodies), and antibody fragments so long as they exhibit the desired biological activity.

**"Antibody fragments"** comprise a portion of a full-length antibody, generally the antigen binding or variable region thereof. Examples of antibody fragments include Fab fragments, Fab' fragments, F(ab')₂ fragments, Fv fragments, scFv (sFv) fragments, scFv-Fc fragments, diabodies, linear antibodies, single-chain antibody molecules, and multispecific antibodies formed from antibody fragments.

By **"interleukin 6 (IL-6)"** or **"IL-6 polypeptide"** is meant a polypeptide or fragment thereof having at least about 85% or greater amino acid identity to the amino acid sequence provided at NCBI Accession No. NP_000591 and having IL-6 biological activity. IL-6 is a pleotropic cytokine with multiple biologic functions. Exemplary IL-6 biological activities include immunostimulatory and pro-inflammatory activities. By **"interleukin 6 (IL-6) nucleic acid"** is meant a polynucleotide encoding an interleukin 6 (IL-6) polypeptide. An exemplary interleukin 6 (IL-6) nucleic acid sequence is provided at NCBI Accession No. NM_000600.

By **"IL-6 antibody"** or **"anti-IL-6 antibody"** is meant an antibody that specifically binds IL-6. Anti-IL-6 antibodies include monoclonal and polyclonal antibodies that are specific for IL-6, and antigen-binding fragments or derivatives thereof. Anti-IL-6 antibodies are described in greater detail in Section 6.2.2.1 below.

Percent **"identity"** between a polypeptide sequence and a reference sequence is defined as the percentage of amino acid residues in the polypeptide sequence that are identical to the amino acid residues in the reference sequence, after aligning the sequences and introducing gaps, if necessary, to achieve the maximum percent sequence identity. Alignment for purposes of determining percent amino acid sequence identity can be achieved in various ways that are within the skill in the art, for instance, using publicly available computer software such as BLAST, BLAST-2, ALIGN, MEGALIGN (DNASTAR), CLUSTALW, CLUSTAL OMEGA, or MUSCLE software. Those skilled in the art can determine appropriate parameters for aligning sequences, including any algorithms needed to achieve maximal alignment over the full length of the sequences being compared. Unless otherwise specified, the percent sequence identity is determined using BLAST algorithms using default parameters.

By **"IL-6 mediated inflammatory disorder"** is meant any disorder in which IL-6 is known or suspected to contribute to the etiology of the disease or any of its symptoms.

By **"subject"** is meant a human or non-human mammal, including, but not limited to, bovine, equine, canine, ovine, feline, and rodent, including murine and rattus, subjects. A **"patient"** is a human subject.

As used herein, the terms **"treat,"** "**treating,**" "**treatment,**" and the like refer to reducing or ameliorating a disorder, and/or signs or symptoms associated therewith, or slowing or halting the progression thereof. It will be appreciated that, although not precluded, treating a disorder or condition does not require that the disorder, condition or symptoms associated therewith be completely eliminated.

In this disclosure, **"comprises," "comprising,"** "**containing,**" "**having**," "**includes**," **"including,"** and linguistic variants thereof have the meaning ascribed to them in U.S. Patent law, permitting the presence of additional components beyond those explicitly recited.

The term **"about"** indicates and encompasses an indicated value and a range above and below that value. The term "about" may indicate the designated value ± 10%, ± 5%, or ± 1%. Where applicable, the term "about" may indicate the designated value(s) ± one standard deviation of that value(s). If not otherwise specified, "about" indicates ± 10% of the designated value.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

### 6.2. Formulation

Described herein is a sterile, stable, aqueous formulation (pharmaceutical composition) comprising an antibody. The formulation may further comprise at least one saccharide. The formulation may further comprise at least one surfactant. The formulation f may urther comprise at least one free amino acid. The formulation may further comprise at least one antioxidant. The formulation may further comprise at least one buffering component. The formulation may comprise an antibody, at least one saccharide, at least one surfactant, at least one free amino acid, at least one antioxidant, and at least one buffering component. Also described herein is a formulation that comprises about 5 mg/mL to about 120 mg/mL of an anti-IL-6 antibody and about 5 mM to about 15 mM methionine.

### 6.2.1. Antibody Concentrations

Described herein is an antibody concentration in the formulation that is at least about 5 mg/mL, at least about 10 mg/mL, at least about 15 mg/mL, at least about 20 mg/mL, at least about 30 mg/mL, at least about 50 mg/mL, at least about 100 mg/mL, at least about 150 mg/mL, at least about 200 mg/mL, at least about 250 mg/mL, or at least about 300 mg/mL. Described herein is an antibody concentration in the formulation that is about 5 mg/mL to about 300 mg/mL, about 10 mg/mL to about 250 mg/mL, about 20 mg/mL to about 200 mg/mL, about 30 mg/mL to about 150 mg/mL, or about 50 mg/mL to about 100 mg/mL. Described herein is an antibody concentration in the formulation that is about 5 mg/mL, about 7.5 mg/mL, about 10 mg/mL, about 15 mg/mL, about 20 mg/mL, about 30 mg/mL, about 50 mg/mL, about 70 mg/mL, about 100 mg/mL, about 120 mg/mL, or about 150 mg/mL.

Described herein is an antibody concentration in the formulation that is at least 5 mg/mL, at least 10 mg/mL, at least 15 mg/mL, at least 20 mg/mL, at least 30 mg/mL, at least 50 mg/mL, at least 100 mg/mL, at least 150 mg/mL, at least 200 mg/mL, at least 250 mg/mL, or at least 300 mg/mL. Described herein is an antibody concentration in the formulation is 5 mg/mL to 300 mg/mL, 10 mg/mL to 250 mg/mL, 20 mg/mL to 200 mg/mL, 30 mg/mL to 150 mg/mL, or 50 mg/mL to 100 mg/mL. Described herein is an antibody concentration in the formulation is 5 mg/mL, 7.5 mg/mL, 10 mg/mL, 15 mg/mL, 20 mg/mL, 30 mg/mL, 50 mg/mL, 70 mg/mL, 100 mg/mL, 120 mg/mL, or 150 mg/mL.

### 6.2.2. Antibodies

The antibodies described herein include, e.g., native antibodies, monoclonal antibodies, polyclonal antibodies, multispecific antibodies (e.g., bispecific antibodies) formed from at least two antibodies, antibody fragments (e.g., antibody fragments that bind to and/or recognize one or more antigens), murine antibodies, chimeric antibodies, humanized antibodies, human antibodies, and antibodies and antibody fragments isolated from antibody phage libraries.

The antibody described herein is directed against one or more antigens. Examples of suitable anti-inflammatory and/or anti-cancer antibodies include, but are not limited to, anti-TNF alpha antibodies such as adalimumab, infliximab, etanercept, golimumab, and certolizumab pegol; anti-IL1β antibodies such as canakinumab; anti-IL12/23 antibodies such as ustekinumab and briakinumab; anti-IL2R antibodies, such as daclizumab; anti-BAFF antibodies such as belimumab; anti-CD20 antibodies such as rituximab; anti-CD22 antibodies such as epratuzumab; anti-CD25 antibodies such as daclizumab; anti-CD30 antibodies such as iratumumab; anti-CD33 antibodies such as gemtuzumab; anti-CD52 antibodies such as alemtuzumab; anti-CD152 antibodies such as ipilimumab; anti-EGFR antibodies such as cetuximab; anti-VEGF antibodies such as bevacizumab; anti-HER2 antibodies such as trastuzumab and pertuzumab; anti-IL-6R antibodies such as tocilizumab, sarilumab, and vobarilizumab; and anti-IL-6 antibodies, such as siltuximab, gerilimzumab (also known as gerilizumab), and ziltivekimab.

### 6.2.2.1. Anti-IL-6 Antibody

Also described herein is an antibody formulation that comprises an anti-IL-6 antibody.

Described herein is an antibody formulation that comprises about 2 mg/mL to about 200 mg/mL of an anti-IL-6 antibody, such as about 2 mg/mL to about 5 mg/mL, about 2 mg/mL to about 10 mg/mL, about 2 mg/mL to about 30 mg/mL, about 2 mg/mL to about 60 mg/mL, about 2 mg/mL to about 120 mg/mL, about 2 mg/mL to about 200 mg/mL, about 5 mg/mL to about 10 mg/mL, about 5 mg/mL to about 30 mg/mL, about 5 mg/mL to about 60 mg/mL, about 5 mg/mL to about 120 mg/mL, about 5 mg/mL to about 200 mg/mL, about 10 mg/mL to about 30 mg/mL, about 10 mg/mL to about 60 mg/mL, about 10 mg/mL to about 120 mg/mL, about 10 mg/mL to about 200 mg/mL, about 30 mg/mL to about 60 mg/mL, about 30 mg/mL to about 120 mg/mL, about 30 mg/mL to about 200 mg/mL, about 60 mg/mL to about 120 mg/mL, about 60 mg/mL to about 200 mg/mL, or about 120 mg/mL to about 200 mg/mL of an anti-IL-6 antibody. Described herein is an antibody formulation that comprises 2 mg/mL to 200 mg/mL of an anti-IL-6 antibody, such as 2 mg/mL to 5 mg/mL, 2 mg/mL to 10 mg/mL, 2 mg/mL to 30 mg/mL, 2 mg/mL to 60 mg/mL, 2 mg/mL to 120 mg/mL, 2 mg/mL to 200 mg/mL, 5 mg/mL to 10 mg/mL, 5 mg/mL to 30 mg/mL, 5 mg/mL to 60 mg/mL, 5 mg/mL to 120 mg/mL, 5 mg/mL to 200 mg/mL, 10 mg/mL to 30 mg/mL, 10 mg/mL to 60 mg/mL, 10 mg/mL to 120 mg/mL, 10 mg/mL to 200 mg/mL, 30 mg/mL to 60 mg/mL, 30 mg/mL to 120 mg/mL, 30 mg/mL to 200 mg/mL, 60 mg/mL to 120 mg/mL, 60 mg/mL to 200 mg/mL, or 120 mg/mL to 200 mg/mL of an anti-IL-6 antibody. Described herein is an antibody formulation that comprises about 5 mg/mL to about 120 mg/mL of an anti-IL-6 antibody. Described herein is an antibody formulation that comprises 5 mg/mL to 120 mg/mL of an anti-IL-6 antibody.

Described herein is an anti-IL-6 antibody that neutralizes the biological activity of IL-6. Described herein is a neutralizing antibody that prevents binding of IL-6 to the IL-6 receptor.

Described herein is a IL-6 antibody that is an anti-IL-6 monoclonal antibody. Described herein is an IL-6 antibody that is a polyclonal composition comprising a plurality of species of anti-IL-6 antibodies, each of the plurality having unique CDRs.

Described herein is an anti-IL-6 antibody that is a Fab, Fab', F(ab')₂, Fv, scFv, (scFv)₂, single chain antibody molecule, dual variable domain antibody, single variable domain antibody, linear antibody, or V domain antibody.

Described herein is an anti-IL-6 antibody that comprises a heavy chain constant region. Described herein is a heavy chain that constant region is Fc, optionally human Fc. Described herein is an anti-IL-6 antibody that comprises a heavy chain constant region of a class selected from IgG, IgA, IgD, IgE, and IgM. Described herein is an anti-IL-6 antibody that comprises a heavy chain constant region of the class IgG and a subclass selected from IgG1, IgG2, IgG3, and IgG4.

Described herein is an antibody that is bispecific or multispecific, with at least one of the antigen-binding portions having specificity for IL-6.

Described herein is an antibody that is fully human. Described herein is an antibody that is humanized. Described herein is an antibody that is chimeric and has non-human V regions and human C region domains. Described herein is an antibody that is murine.

Described herein is an anti-IL-6 antibody that has a K_{D} for binding human IL-6 of less than 100 nM. Described herein is an anti-IL-6 antibody that has a K_{D} for binding human IL-6 of less than 75 nM, 50 nM, 25 nM, 20 nM, 15 nM, or 10 nM. Described herein is an anti-IL-6 antibody that has a K_{D} for binding human IL-6 of less than 5 nM, 4 nM, 3 nM, or 2 nM. Described herein is an anti-IL-6 antibody that has a K_{D} for binding human IL-6 of less than 1 nM, 750 pM, or 500 pM. Described herein is an anti-IL-6 antibody that has a K_{D} for binding human IL-6 of no more than 500 pM, 400 pM, 300 pM, 200 pM, or 100 pM.

Described herein is an anti-IL-6 antibody that has an elimination half-life following intravenous administration of at least 7 days. Described herein is an anti-IL-6 antibody has an elimination half-life of at least 14 days, at least 21 days, or at least 30 days.

Described herein is an anti-IL-6 antibody has a human IgG constant region with at least one amino acid substitution that extends serum half-life as compared to the unsubstituted human IgG constant domain.

The IgG constant domain may comprise substitutions at residues 252, 254, and 256, wherein the amino acid substitution at amino acid residue 252 is a substitution with tyrosine, the amino acid substitution at amino acid residue 254 is a substitution with threonine, and the amino acid substitution at amino acid residue 256 is a substitution with glutamic acid ("YTE"). *See* U.S. Pat. No. 7,083,784. In certain extended half-life embodiments, the IgG constant domain comprises substitutions selected from T250Q/M428L (Hinton et al., J. Immunology 176:346-356 (2006)); N434A (Yeung et al., J. Immunology 182:7663-7671 (2009)); or T307A/E380A/N434A (Petkova et al., International Immunology, 18: 1759-1769 (2006)).

The elimination half-life of the anti-IL-6 antibody may be increased by utilizing the FcRN-binding properties of human serum albumin. The antibody may be conjugated to albumin (Smith et al., Bioconjug. Chem., 12: 750-756 (2001)). The anti-IL-6 antibody may be fused to bacterial albumin-binding domains (Stork et al., Prot. Eng. Design Science 20: 569-576 (2007)). The anti-IL-6 antibody may be fused to an albumin-binding peptide (Nguygen et al., Prot Eng Design Sel 19: 291-297 (2006)). The anti-IL-6 antibody may be bispecific, with one specificity being to IL-6, and one specificity being to human serum albumin (Ablynx, WO 2006/122825 (bispecific Nanobody)).

The elimination half-life of the anti-IL-6 antibody may be increased by PEGylation (Melmed et al., Nature Reviews Drug Discovery 7: 641-642 (2008)); by HPMA copolymer conjugation (Lu et al., Nature Biotechnology 17: 1101-1104 (1999)); by dextran conjugation *(*Nuclear Medicine Communications, 16: 362-369 (1995)); by conjugation with homo-aminoacid polymers (HAPs; HAPylation) (Schlapschy et al., Prot Eng Design Sel 20: 273-284 (2007)); or by polysialylation (Constantinou et al., Bioconjug. Chem. 20: 924-931 (2009)).

Described herein is an anti-IL-6 antibody that comprises all six CDRs of COR-001 (also known as ziltivekimab and MEDI5117), siltuximab, gerilimzumab, sirukumab, clazakizumab, olokizumab, VX30 (VOP-R003; Vaccinex), EB-007 (EBI-029; Eleven Bio), or FM101 (Femta Pharmaceuticals, Lonza). Described herein is an anti-IL-6 antibody that comprises the VH and VL domains of COR-001, siltuximab, gerilimzumab, sirukumab, clazakizumab, olokizumab, VX30 (VOP-R003; Vaccinex), EB-007 (EBI-029; Eleven Bio), or FM101 (Femta Pharmaceuticals, Lonza). Described herein is an anti-IL-6 antibody that is (that is, comprises the heavy and light chains of) COR-001 (ziltivekimab), siltuximab, gerilimzumab, sirukumab, clazakizumab, olokizumab, VX30 (VOP-R003; Vaccinex), EB-007 (EBI-029; Eleven Bio), or FM101 (Femta Pharmaceuticals, Lonza).

### 6.2.2.1.1. COR-001 and Derivatives

In certain preferred embodiments, the anti-IL-6 antibody or antigen-binding portion thereof comprises all six CDRs of COR-001. The COR-001 antibody (also known as ziltivekimab and MEDI5117) is described in WO 2010/088444 and US 2012/0034212. In particular embodiments, the antibody or antigen-binding portion thereof comprises the COR-001 heavy chain V region and light chain V region. In specific embodiments, the antibody is the full-length COR-001 antibody. The COR-001 antibody has the following CDR, VH, VL, heavy chain, and light chain sequences:
COR-001 VH CDR1
   SNYMI (SEQ ID NO: 1)
COR-001 VH CDR2
   DLYYYAGDTYYADSVKG (SEQ ID NO: 2)
COR-001 VH CDR3
   WADDHPPWIDL (SEQ ID NO: 3)
COR-001 VL CDR1
   RASQGISSWLA (SEQ ID NO: 4)
COR-001 VL CDR2
   KASTLES (SEQ ID NO: 5)
COR-001 VL CDR3
   QQSWLGGS (SEQ ID NO: 6)
COR-001 VH 
EVQLVESGGGLVQPGGSLRLSCAASGFTISSNYMIWVRQAPGKGLEWVSDLYYYAGDTYY
ADSVKGRFTMSRDISKNTVYLQMNSLRAEDTAVYYCARWADDHPPWIDLWGRGTLVTVSS
(SEQ ID NO: 7)
COR-001 VL 
DIQMTQSPSTLSASVGDRVTITCRASQGISSWLAWYQQKPGKAPKVLIYKASTLESGVPS
RFSGSGSGTEFTLTISSLQPDDFATYYCQQSWLGGSFGQGTKLEIK (SEQ ID NO: 8)
COR-001 Heavy chain 
EVQLVESGGGLVQPGGSLRLSCAASGFTISSNYMIWVRQAPGKGLEWVSDLYYYAGDTYY
ADSVKGRFTMSRDISKNTVYLQMNSLRAEDTAVYYCARWADDHPPWIDLWGRGTLVTVSS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGG
PSVFLFPPKPKDTLYITREPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 9)
COR-001 Light chain

DIQMTQSPSTLSASVGDRVTITCRASQGISSWLAWYQQKPGKAPKVLIYKASTLESGVPS
RFSGSGSGTEFTLTISSLQPDDFATYYCQQSWLGGSFGQGTKLEIKRTVAAPSVFIFPPS
DEQLKSGTASVVCLLNNFYPREAKVQWKVDNALQSGNSQESVTEQDSKDSTYSLSSTLTL
SKADYEKHKVYACEVTHQGLSSPVTKSFNRGEC (SEQ ID NO: 10)

Described herein is a anti-IL-6 antibody that is the derivative of COR-001. Described herein is a derivative of COR-001 that comprises a VH CDR1 that has at least about 50%, 75%, 80%, 85%, 90%, 95%, 97%, or 99% identity with SEQ ID NO: 1. Described herein is a derivative of COR-001 that comprises a VH CDR2 that has at least about 50%, 75%, 80%, 85%, 90%, 95%, 97% or 99% identity with SEQ ID NO: 2 Described herein is a derivative of COR-001 that comprises a VH CDR3 that has at least about 50%, 75%, 80%, 85%, 90%, 95%, 97% or 99% identity with SEQ ID NO: 3. Described herein is a derivative of COR-001 that comprises a VL CDR1 that has at least about 50%, 75%, 80%, 85%, 90%, 95%, 97% or 99% identity with SEQ ID NO: 4. Described herein is a derivative of COR-001 that comprises a VL CDR2 that has at least about 50%, 75%, 80%, 85%, 90%, 95%, 97% or 99% identity with SEQ ID NO: 5. Described herein is a derivative of COR-001 that comprises a VL CDR3 that has at least about 50%, 75%, 80%, 85%, 90%, 95%, 97% or 99% identity with SEQ ID NO: 6.

Described herein is a derivative of COR-001 that comprises a VH domain that has at least about 50%, 75%, 80%, 85%, 90%, 95%, 97%, or 99% identity with SEQ ID NO: 7. Described herein is a derivative of COR-001 that comprises a VL domain that has at least about 50%, 75%, 80%, 85%, 90%, 95%, 97%, or 99% identity with SEQ ID NO: 8. Described herein is a derivative of COR-001 that comprises a VH domain that has at least about 50%, 75%, 80%, 85%, 90%, 95%, 97%, or 99% identity with SEQ ID NO: 7 and a VL domain that has at least about 50%, 75%, 80%, 85%, 90%, 95%, 97%, or 99% identity with SEQ ID NO: 8.

Described herein is a derivative of COR-001 that comprises a heavy chain that has at least about 50%, 75%, 80%, 85%, 90%, 95%, 97%, or 99% identity with SEQ ID NO: 9. Described herein is a derivative of COR-001 that comprises a light chain that has at least about 50%, 75%, 80%, 85%, 90%, 95%, 97%, or 99% identity with SEQ ID NO: 10. Described herein is a derivative of COR-001 that comprises a heavy chain that has at least about 50%, 75%, 80%, 85%, 90%, 95%, 97%, or 99% identity with SEQ ID NO: 9 and a light chain that has at least about 50%, 75%, 80%, 85%, 90%, 95%, 97%, or 99% identity with SEQ ID NO: 10.

### 6.2.3. Antioxidants

Described herein is a formulation that comprises an antioxidant.

Described herein is a antioxidant that is at a concentration of about 1 mM to about 100 mM, such as about 2 mM to about 80 mM, about 3 mM to about 50 mM, about 5 mM to about 30 mM, or about 10 mM to about 20 mM. Described herein is a antioxidant that is at a concentration of 1 mM to 100 mM, such as 2 mM to 80 mM, 3 mM to 50 mM, 5 mM to 30 mM, or 10 mM to 20 mM.

Described herein is a antioxidant that is a naturally occurring compound. Described herein is a antioxidant that is a synthetic compound. Described herein is a antioxidant that is selected from chelating agents, reducing agents, oxygen scavengers, and chain terminators, such as superoxide dismutase (SOD), vitamin C or E, methionine, cysteine, glutathione, EDTA, sodium thiosulfate, catalase, or platinum. Described herein is a antioxidant that is citric acid, uric acid, ascorbic acid, lipoic acid, glutathione, tocopherol, carotene, lycopene, cysteine, or methionine.

### 6.2.3.1. Methionine

Described herein is a formulation comprises methionine. Described herein is a, the formulation comprises L-methionine.

Described herein is a formulation that comprises at least about 1 mM, at least about 2 mM, at least about 5 mM, at least about 10 mM, at least about 15 mM, at least about 20 mM, at least about 25 mM, at least about 50 mM, or at least about 100 mM methionine. Described herein is a formulation comprises about 1 mM to about 100 mM, about 2 mM to about 80 mM, about 5 mM to about 50 mM, about 10 mM to about 20 mM methionine. Described herein is a formulation comprises about 1 mM, about 2 mM, about 5 mM, about 10 mM, about 15 mM, about 20 mM, about 25 mM, about 50 mM, or about 100 mM methionine. Described herein is a formulation that comprises about 1 mM to about 50 mM methionine. In particular embodiments, the formulation comprises about 5 mM to about 15 mM methionine. In specific embodiments, the formulation comprises about 10 mM methionine.

Described herein is a formulation that comprises at least 1 mM, at least 2 mM, at least 5 mM, at least 10 mM, at least 15 mM, at least 20 mM, at least 25 mM, at least 50 mM, or at least 100 mM methionine. Described herein is a formulation that comprises 1 mM to 100 mM, 2 mM to 80 mM, 5 mM to 50 mM, 10 mM to 20 mM methionine. Described herein is a formulation that comprises 1 mM, 2 mM, 5 mM, 10 mM, 15 mM, 20 mM, 25 mM, 50 mM, or 100 mM methionine. In some embodiments, the formulation comprises 1 mM to 50 mM methionine. In particular embodiments, the formulation comprises 5 mM to 15 mM methionine. In specific embodiments, the formulation comprises 10 mM methionine.

### 6.2.4. Surfactants

Described herein is a composition that comprises a surfactant. The surfactant can lower surface tension of a liquid. In some embodiments, the surfactant that is a nonionic surfactant. Examples of surfactants include polysorbate (polyoxyethylene sorbitan monolaurate, for example, polysorbate 20 and polysorbate 80); TRITON (t-Octylphenoxypolyethoxyethanol); sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g. lauroamidopropyl); myristamidopropyl-, palmidopropyl-, or isostearamidopropyl-dimethylamine; sodium methyl cocoyl-, or disodium methyl oleyl-taurate; sorbitan monopalmitate; the MONAQUAT series (Mona Industries, Inc., Paterson, N.J.); polyethyl glycol (PEG), polypropylene glycol (PPG), and copolymers of poloxyethylene and poloxypropylene glycol (e.g. Pluronics/Poloxamer, PF68 etc.). The surfactant may be polysorbate. The polysorbate may be polysorbate 20, polysorbate 40, polysorbate 60, or polysorbate 80.

Described herein is a composition that comprises a surfactant at a concentration of about 0.001% to about 1%, such as about 0.001% to about 0.1%, about 0.005% to about 0.2%, about 0.01% to about 0.2%, or about 0.05% to about 0.1% (w/v). Described herein is a composition that comprises a surfactant at a concentration of 0.001% to 1%, such as 0.001% to 0.1%, 0.005% to 0.2%, 0.01% to 0.2%, or 0.05% to 0.1% (w/v).

### 6.2.4.1. Polysorbate 80

In some embodiments, the formulation comprises polysorbate 80 (PS80).

Described herein is a formulation that comprises at least about 0.001%, at least about 0.002%, at least about 0.003%, at least about 0.005%, at least about 0.01%, at least about 0.02%, at least about 0.05%, at least about 0.1%, at least about 0.2%, at least about 0.5%, or at least about 1% (w/v) polysorbate 80. Described herein is a formulation that comprises about 0.001% to about 1%, about 0.002% to about 0.5%, about 0.005% to about 0.2%, or about 0.01% to about 0.1% (w/v) polysorbate 80. Described herein is a formulation that comprises about 0.001%, about 0.002%, about 0.003%, about 0.005%, about 0.01%, about 0.02%, about 0.05%, about 0.07%, about 0.1%, about 0.2%, about 0.5%, or about 1% (w/v) polysorbate 80. Described herein is a formulation that comprises about 0.005% to about 0.5% (w/v) polysorbate 80. Described herein is a formulation that comprises about 0.03% to about 0.1% (w/v) polysorbate 80. Described herein is a formulation that comprises about 0.05% to about 0.1% (w/v) polysorbate 80. Described herein is a formulation that comprises about 0.07% (w/v) polysorbate 80.

Described herein is a formulation that comprises at least 0.001%, at least 0.002%, at least 0.003%, at least 0.005%, at least 0.01%, at least 0.02%, at least 0.05%, at least 0.1%, at least 0.2%, at least 0.5%, or at least 1% (w/v) polysorbate 80. Described herein is a formulation that comprises 0.001% to 1%, 0.002% to 0.5%, 0.005% to 0.2%, or 0.01% to 0.1% (w/v) polysorbate 80. Described herein is a formulation that comprises 0.001%, 0.002%, 0.003%, 0.005%, 0.01%, 0.02%, 0.05%, 0.07%, 0.1%, 0.2%, 0.5%, or 1% (w/v) polysorbate 80. Described herein is a formulation that comprises 0.005% to 0.5% (w/v) polysorbate 80. Described herein is a formulation that comprises 0.03% to 0.1% (w/v) polysorbate 80. Described herein is a formulation comprises 0.05% to 0.1% (w/v) polysorbate 80. In particular embodiments, the formulation comprises 0.07% (w/v) polysorbate 80.

### 6.2.4.2. Polysorbate 60

Described herein is a formulation that comprises polysorbate 60 (PS60).

Described herein is a formulation that comprises at least about 0.001%, at least about 0.002%, at least about 0.003%, at least about 0.005%, at least about 0.01%, at least about 0.02%, at least about 0.05%, at least about 0.1%, at least about 0.2%, at least about 0.5%, or at least about 1% (w/v) polysorbate 60. In some embodiments, the formulation comprises about 0.001% to about 1%, about 0.002% to about 0.5%, about 0.005% to about 0.2%, or about 0.01% to about 0.1% (w/v) polysorbate 60. In some embodiments, the formulation comprises about 0.001%, about 0.002%, about 0.003%, about 0.005%, about 0.01%, about 0.02%, about 0.05%, about 0.07%, about 0.1%, about 0.2%, about 0.5%, or about 1% (w/v) polysorbate 60. In certain embodiments, the formulation comprises about 0.03% to about 0.1% (w/v) polysorbate 60. In certain embodiments, the formulation comprises about 0.05% to about 0.1% (w/v) polysorbate 60. In particular embodiments, the formulation comprises about 0.07% (w/v) polysorbate 60.

Described herein is a formulation comprises at least 0.001%, at least 0.002%, at least 0.003%, at least 0.005%, at least 0.01%, at least 0.02%, at least 0.05%, at least 0.1%, at least 0.2%, at least 0.5%, or at least 1% (w/v) polysorbate 60. Described herein is a formulation comprises 0.001% to 1%, 0.002% to 0.5%, 0.005% to 0.2%, or 0.01% to 0.1% (w/v) polysorbate 60. Described herein is a formulation comprises 0.001%, 0.002%, 0.003%, 0.005%, 0.01%, 0.02%, 0.05%, 0.07%, 0.1%, 0.2%, 0.5%, or 1% (w/v) polysorbate 60. In certain embodiments, the formulation comprises 0.03% to 0.1% (w/v) polysorbate 60. In certain embodiments, the formulation comprises 0.05% to 0.1% (w/v) polysorbate 60. In particular embodiments, the formulation comprises 0.07% (w/v) polysorbate 60.

### 6.2.4.3. Polysorbate 40

Described herein is a formulation comprises polysorbate 40 (PS40).

Described herein is a formulation comprises at least about 0.001%, at least about 0.002%, at least about 0.003%, at least about 0.005%, at least about 0.01%, at least about 0.02%, at least about 0.05%, at least about 0.1%, at least about 0.2%, at least about 0.5%, or at least about 1% (w/v) polysorbate 40. Described herein is a formulation comprises about 0.001% to about 1%, about 0.002% to about 0.5%, about 0.005% to about 0.2%, or about 0.01% to about 0.1% (w/v) polysorbate 40. Described herein is a formulation comprises about 0.001%, about 0.002%, about 0.003%, about 0.005%, about 0.01%, about 0.02%, about 0.05%, about 0.07%, about 0.1%, about 0.2%, about 0.5%, or about 1% (w/v) polysorbate 40. In certain embodiments, the formulation comprises about 0.03% to about 0.1% (w/v) polysorbate 40. In certain embodiments, the formulation comprises about 0.05% to about 0.1% (w/v) polysorbate 40. In particular embodiments, the formulation comprises about 0.07% (w/v) polysorbate 40.

Described herein is a formulation comprises at least 0.001%, at least 0.002%, at least 0.003%, at least 0.005%, at least 0.01%, at least 0.02%, at least 0.05%, at least 0.1%, at least 0.2%, at least 0.5%, or at least 1% (w/v) polysorbate 40. Described herein is a formulation comprises 0.001% to 1%, 0.002% to 0.5%, 0.005% to 0.2%, or 0.01% to 0.1% (w/v) polysorbate 40. Described herein is a formulation comprises 0.001%, 0.002%, 0.003%, 0.005%, 0.01%, 0.02%, 0.05%, 0.07%, 0.1%, 0.2%, 0.5%, or 1% (w/v) polysorbate 40. In certain embodiments, the formulation comprises 0.03% to 0.1% (w/v) polysorbate 40. In certain embodiments, the formulation comprises 0.05% to 0.1% (w/v) polysorbate 40. In particular embodiments, the formulation comprises 0.07% (w/v) polysorbate 40.

### 6.2.4.4. Polysorbate 20

Described herein is a formulation comprises polysorbate 20 (PS20).

Described herein is a formulation comprises at least about 0.001%, at least about 0.002%, at least about 0.003%, at least about 0.005%, at least about 0.01%, at least about 0.02%, at least about 0.05%, at least about 0.1%, at least about 0.2%, at least about 0.5%, or at least about 1% (w/v) polysorbate 20. Described herein is a formulation comprises about 0.001% to about 1%, about 0.002% to about 0.5%, about 0.005% to about 0.2%, or about 0.01% to about 0.1% (w/v) polysorbate 20. Described herein is a formulation comprises about 0.001%, about 0.002%, about 0.003%, about 0.005%, about 0.01%, about 0.02%, about 0.05%, about 0.07%, about 0.1%, about 0.2%, about 0.5%, or about 1% (w/v) polysorbate 20. In certain embodiments, the formulation comprises about 0.03% to about 0.1% (w/v) polysorbate 20. In certain embodiments, the formulation comprises about 0.05% to about 0.1% (w/v) polysorbate 20. In particular embodiments, the formulation comprises about 0.07% (w/v) polysorbate 20.

Described herein is a formulation comprises at least 0.001%, at least 0.002%, at least 0.003%, at least 0.005%, at least 0.01%, at least 0.02%, at least 0.05%, at least 0.1%, at least 0.2%, at least 0.5%, or at least 1% (w/v) polysorbate 20. Described herein is a formulation comprises 0.001% to 1%, 0.002% to 0.5%, 0.005% to 0.2%, or 0.01% to 0.1% (w/v) polysorbate 20. Described herein is a formulation comprises 0.001%, 0.002%, 0.003%, 0.005%, 0.01%, 0.02%, 0.05%, 0.07%, 0.1%, 0.2%, 0.5%, or 1% (w/v) polysorbate 20. In certain embodiments, the formulation comprises 0.03% to 0.1% (w/v) polysorbate 20. In certain embodiments, the formulation comprises 0.05% to 0.1% (w/v) polysorbate 20. In particular embodiments, the formulation comprises 0.07% (w/v) polysorbate 20.

### 6.2.5. Saccharides

Described herein is a composition comprises a saccharide and derivatives thereof, including monosaccharides, disaccharides, trisaccharides, polysaccharides, sugar alcohols, reducing sugars, nonreducing sugars, and the like. Examples of saccharides include glucose, mannose, sucrose, trehalose, lactose, fructose, maltose, dextran, dextrin, erythritol, glycerol, arabitol, sylitol, sorbitol, mannitol, mellibiose, melezitose, raffinose, mannotriose, stachyose, maltose, lactulose, maltulose, glucitol, maltitol, lactitol, isomaltulose, and the like. Described herein is a saccharide is a disaccharide. The disaccharide may be trehalose or sucrose. The disaccharide may be at a concentration from about 1% to about 40%, from about 2% to about 20%, or from about 2% to about 10% (w/v). In various embodiments, the disaccharide is at a concentration from 1% to 40%, from 2% to 20%, or from 2% to 10% (w/v).

### 6.2.5.1. Trehalose

Described herein is a formulation comprises trehalose.

The formulation may comprise about 1% to about 50% (w/v) trehalose (as trehalose dihydrate). Described herein is a formulation comprises at least about 1%, at least about 2%, at least about 3%, at least about 5%, at least about 10%, at least about 15%, at least about 20%, at least about 30%, or at least about 40% (w/v) trehalose. Described herein is a formulation comprises about 1% to about 40%, about 2% to about 30%, about 3% to about 20%, about 5% to about 15%, or about 5% to about 10% (w/v) trehalose. In certain embodiments, the formulation comprises about 1%, about 2%, about 3%, about 5%, about 10%, about 15%, about 20%, about 30%, or about 40% (w/v) trehalose. In specific embodiments, the formulation comprises about 5% (w/v) trehalose.

The formulation may comprise 1% to 50% (w/v) trehalose (as trehalose dihydrate). Described herein is a formulation comprises at least 1%, at least 2%, at least 3%, at least 5%, at least 10%, at least 15%, at least 20%, at least 30%, or at least 40% (w/v) trehalose. Described herein is a formulation comprises 1% to 40%, 2% to 30%, 3% to 20%, 5% to 15%, or 5% to 10% (w/v) trehalose. The formulation comprises 1%, 2%, 3%, 5%, 10%, 15%, 20%, 30%, or 40% (w/v) trehalose. The formulation comprises 5% (w/v) trehalose.

### 6.2.5.2. Sucrose

Described herein is a formulation that comprises sucrose.

Described herein is a formulation that comprises at least about 1%, at least about 2%, at least about 3%, at least about 5%, at least about 10%, at least about 15%, at least about 20%, at least about 30%, or at least about 40% (w/v) sucrose. Described herein is a formulation that comprises about 1% to about 40%, about 2% to about 30%, about 3% to about 20%, about 5% to about 15%, or about 5% to about 10% (w/v) sucrose. The formulation may comprise about 1%, about 2%, about 3%, about 5%, about 10%, about 15%, about 20%, about 30%, or about 40% (w/v) sucrose. The formulation may comprise about 5% (w/v) sucrose.

Described herein is a formulation that comprises at least 1%, at least 2%, at least 3%, at least 5%, at least 10%, at least 15%, at least 20%, at least 30%, or at least 40% (w/v) sucrose. Described herein is a formulation that comprises 1% to 40%, 2% to 30%, 3% to 20%, 5% to 15%, or 5% to 10% (w/v) sucrose. The formulation may comprise 1%, 2%, 3%, 5%, 10%, 15%, 20%, 30%, or 40% (w/v) sucrose. The formulation may comprise 5% (w/v) sucrose.

### 6.2.6. Free Amino Acids

Described herein is a formulation comprises at least one free amino acid. Described herein is a formulation comprises 1 free amino acid. Described herein is a formulation comprises 2 free amino acids. Described herein is a formulation comprises 3 free amino acids. The free amino acid can be in the L-form, the D-form or a mixture of these forms.

The at least one free amino acid may be glycine, glutamine, asparagine, histidine, arginine, or lysine. The free amino acid may be at a concentration of about 1 mM to about 400 mM, such as about 2 mM to about 300 mM, about 5 mM to about 200mM, or about 10 mM to about 100 mM. The free amino acid may be at a concentration of 1 mM to 400 mM, such as 2 mM to 300 mM, 5 mM to 200mM, or 10 mM to 100 mM.

Described herein is a formulation that comprises methionine and at least one additional free amino acid selected from glycine, glutamine, asparagine, histidine, arginine, and lysine. In certain embodiments, the formulation comprises methionine and arginine. In certain embodiments, the formulation comprises methionine, arginine, and histidine.

### 6.2.6.1. Arginine

Described herein is a formulation that comprises arginine. Described herein is a formulation that comprises L-arginine.

The formulation may comprise about 5 mM to about 500 mM arginine (as arginine-HCl). Described herein is a formulation that comprises at least about 5 mM, at least about 10 mM, at least about 20 mM, at least about 30 mM, at least about 50 mM, at least about 70 mM, at least about 80 mM, at least about 100 mM, at least about 150 mM, at least about 200 mM, or at least about 400 mM arginine. The formulation may comprise about 5 mM to about 400 mM, about 10 mM to about 200 mM, about 20 mM to about 150 mM, about 30 mM to about 100 mM, or about 50 mM to about 80 mM arginine. The formulation may comprise about 5 mM, about 10 mM, about 20 mM, about 30 mM, about 50 mM, about 70 mM, about 80 mM, about 100 mM, about 150 mM, about 200 mM, or about 400 mM arginine. The formulation may comprise about 10 mM to about 200 mM arginine. The formulation may comprise about 70 mM arginine.

The formulation may comprise 5 mM to 500 mM arginine (as arginine-HCl). Described herein is a formulation comprises at least 5 mM, at least 10 mM, at least 20 mM, at least 30 mM, at least 50 mM, at least 70 mM, at least 80 mM, at least 100 mM, at least 150 mM, at least 200 mM, or at least 400 mM arginine. The formulation may comprise 5 mM to 400 mM, 10 mM to 200 mM, 20 mM to 150 mM, 30 mM to 100 mM, or 50 mM to 80 mM arginine. The formulation may comprise 5 mM, 10 mM, 20 mM, 30 mM, 50 mM, 70 mM, 80 mM, 100 mM, 150 mM, 200 mM, or 400 mM arginine. The formulation may comprise 10 mM to 200 mM arginine. In specific embodiments, the formulation comprises 70 mM arginine.

### 6.2.7. Buffering Agents

Described herein is a formulation comprises at least one buffering agent (buffering component). Typically, the buffering agent, when present, is used to adjust the pH of the formulation to about 4.0 to about 8.0, about 4.5 to about 7.5, about 5.0 to about 7.0, about 5.5 to about 6.5, about 5.7 to about 6.3, about 5.9 to about 6.1, or about 6.0.

The at least one buffering agent may be selected from acetate, succinate, gluconate, histidine, citrate, phosphate, maleate, cacodylate, 2-[N-morpholino]ethanesulfonic acid (MES), bis (2-hydroxyethyl)iminotris [hydroxymethyl]methane (Bis-Tris), N-[2-acetamido]-2-iminodiacetic acid (ADA), glycylglycine and other organic acid buffers. The buffering agent may be histidine, citrate, phosphate, glycine, or acetate. The buffering component may be at a concentration from about 1 mM to about 200 mM, from about 1 mM to about 50 mM, or from about 5 mM to about 20 mM. The buffering component may be at a concentration from 1 mM to 200 mM, from 1 mM to 50 mM, or from 5 mM to 20 mM. The buffering component may be at a concentration of about 10 mM, about 15 mM, about 20 mM, or about 25 mM. The buffering component may be at a concentration of 10 mM, 15 mM, 20 mM, or 25 mM.

### 6.2.7.1. Histidine

Described herein is a formulation that comprises histidine. In some embodiments, the formulation comprises L-histidine.

Described herein is a composition that comprises at least about 1 mM, at least about 5 mM, at least about 10 mM, at least about 15 mM, at least about 20 mM, at least about 30 mM, at least about 50 mM, at least about 100 mM, at least about 150 mM, or at least about 200 mM histidine. Described herein is a composition that comprises about 1 mM to about 200 mM, about 5 mM to about 150 mM, about 10 mM to about 100 mM, about 15 mM to about 50 mM, or about 20 mM to about 30 mM histidine. Described herein is a composition that comprises about 1 mM, about 5 mM, about 10 mM, about 15 mM, about 20 mM, about 30 mM, about 50 mM, about 100 mM, about 150 mM, or about 200 mM histidine. Described herein is a formulation that comprises about 10 mM to about 100 mM histidine. In specific embodiments, the formulation comprises about 20 mM histidine.

Described herein is a composition that comprises at least 1 mM, at least 5 mM, at least 10 mM, at least 15 mM, at least 20 mM, at least 30 mM, at least 50 mM, at least 100 mM, at least 150 mM, or at least 200 mM histidine. Described herein is a composition that comprises 1 mM to 200 mM, 5 mM to 150 mM, 10 mM to 100 mM, 15 mM to 50 mM, or 20 mM to 30 mM histidine. Described herein is a composition that comprises 1 mM, 5 mM, 10 mM, 15 mM, 20 mM, 30 mM, 50 mM, 100 mM, 150 mM, or 200 mM histidine. Described herein is a formulation that comprises 10 mM to 100 mM histidine. In specific embodiments, the formulation comprises 20 mM histidine.

### 6.2.7.2. pH

Described herein is a formulation that has a pH of about 4.0 to about 8.0, such as about 4.5 to about 7.5, about 5.0 to about 7.0, about 5.5 to about 6.5, about 5.7 to about 6.3, or about 5.9 to about 6.1. Described herein is a formulation that has a pH of about 4.0, about 4.5, about 5.0, about 5.5, about 5.7, about 5.9, about 6.0, about 6.1, about 6.3, about 6.5, about 7.0, about 7.5, or about 8.0. Described herein is a formulation that has a pH of about 5.0 to about 7.0. Described herein is a formulation that has a pH of about 5.0 to about 7.0. Described herein is a formulation has a pH of about 5.5 to about 6.5. Described herein is a formulation that has a pH of about 5.7 to about 6.3. In specific embodiments, the formulation has a pH of about 6.0.

Described herein is a formulation that has a pH of 4.0 to 8.0, such as 4.5 to 7.5, 5.0 to 7.0, 5.5 to 6.5, 5.7 to 6.3, or 5.9 to 6.1. Described herein is a formulation that has a pH of 4.0, 4.5, 5.0, 5.5, 5.7, 5.9, 6.0, 6.1, 6.3, 6.5, 7.0, 7.5, or 8.0. Described herein is a formulation has a pH of 5.0 to 7.0. In certain embodiments, the formulation has a pH of 5.5 to 6.5. Described herein is a formulation that has a pH of 5.7 to 6.3. In specific embodiments, the formulation has a pH of 6.0.

### 6.2.8. Preservatives

Described herein is a formulation that further comprises at least one preservative. The at least one preservative may be selected from ctadecyldimethylbenzyl ammonium chloride, hexamethonium chloride, benzalkonium chloride (a mixture of alkylbenzyldimethylammonium chlorides in which the alkyl groups are long-chain compounds), and benzethonium chloride. Other types of preservatives include aromatic alcohols such as phenol, butyl and benzyl alcohol, alkyl parabens such as methyl or propyl paraben, catechol, resorcinol, cyclohexanol, 3-pentanol, and m-creSol.

### 6.2.9. Currently Preferred Anti-IL-6 Antibody Formulations

Described herein is a antibody formulation that comprises about 5 mg/mL to about 120 mg/mL of an anti-IL-6 antibody and about 1 mM to about 100 mM methionine, such as about 1 mM to about 5 mM methionine, about 1 mM to about 10 mM methionine, about 1 mM to about 15 mM methionine, about 1 mM to about 30 mM methionine, about 1 mM to about 50 mM methionine, about 1 mM to about 100 mM methionine, about 5 mM to about 10 mM methionine, about 5 mM to about 15 mM methionine, about 5 mM to about 30 mM methionine, about 5 mM to about 50 mM methionine, about 5 mM to about 100 mM methionine, about 10 mM to about 15 mM methionine, about 10 mM to about 30 mM methionine, about 10 mM to about 50 mM methionine, about 10 mM to about 100 mM methionine, about 15 mM to about 30 mM methionine, about 15 mM to about 50 mM methionine, about 15 mM to about 100 mM methionine, about 30 mM to about 50 mM methionine, about 30 mM to about 100 mM methionine, or about 50 mM to about 100 mM methionine. Described herein is an antibody formulation that comprises about 5 mg/mL to about 120 mg/mL of an anti-IL-6 antibody and about 5 mM to about 15 mM methionine. Described herein is an antibody formulation that comprises about 5 mg/mL to about 120 mg/mL of an anti-IL-6 antibody and about 10 mM methionine.

Described herein is an antibody formulation that comprises 5 mg/mL to 120 mg/mL of an anti-IL-6 antibody and 1 mM to 100 mM methionine, such as 1 mM to 5 mM methionine, 1 mM to 10 mM methionine, 1 mM to 15 mM methionine, 1 mM to 30 mM methionine, 1 mM to 50 mM methionine, 1 mM to 100 mM methionine, 5 mM to 10 mM methionine, 5 mM to 15 mM methionine, 5 mM to 30 mM methionine, 5 mM to 50 mM methionine, 5 mM to 100 mM methionine, 10 mM to 15 mM methionine, 10 mM to 30 mM methionine, 10 mM to 50 mM methionine, 10 mM to 100 mM methionine, 15 mM to 30 mM methionine, 15 mM to 50 mM methionine, 15 mM 100 mM methionine, 30 mM to 50 mM methionine, 30 mM to 100 mM methionine, or 50 mM to 100 mM methionine; a antibody formulation that comprises 5 mg/mL to 120 mg/mL of an anti-IL-6 antibody and 5 mM to 15 mM methionine;and a antibody formulation that comprises 5 mg/mL to 120 mg/mL of an anti-IL-6 antibody and 10 mM methionine.

Described herein is a antibody formulation that comprises about 5 mg/mL to about 120 mg/mL of an anti-IL-6 antibody, about 1 mM to about 50 mM methionine, and about 0.005% to about 0.5% (w/v) polysorbate 80. Described herein is a antibody formulation that comprises about 5 mg/mL to about 120 mg/mL of an anti-IL-6 antibody, about 5 mM to about 15 mM methionine, and about 0.03% to about 0.1% (w/v) polysorbate 80. Described herein is a antibody formulation that comprises about 5 mg/mL to about 120 mg/mL of an anti-IL-6 antibody, about 5 mM to about 15 mM methionine, and about 0.05% to about 0.1% (w/v) polysorbate 80. The antibody formulation may comprise about 5 mg/mL to about 120 mg/mL of an anti-IL-6 antibody, about 10 mM methionine, and about 0.07% (w/v) polysorbate 80.

Described herein is an antibody formulation that comprises 5 mg/mL to 120 mg/mL of an anti-IL-6 antibody, 1 mM to 50 mM methionine, and 0.005% to 0.5% (w/v) polysorbate 80. Described herein is an antibody formulation that comprises 5 mg/mL to 120 mg/mL of an anti-IL-6 antibody, 5 mM to 15 mM methionine, and 0.03% to 0.1% (w/v) polysorbate 80. Described herein is an antibody formulation that comprises 5 mg/mL to 120 mg/mL of an anti-IL-6 antibody, 5 mM to 15 mM methionine, and 0.05% to 0.1% (w/v) polysorbate 80. The antibody formulation may comprise 5 mg/mL to 120 mg/mL of an anti-IL-6 antibody, 10 mM methionine, and 0.07% (w/v) polysorbate 80.

Described herein is an antibody formulation that comprises: a) about 5 mg/mL to about 120 mg/mL of an anti-IL-6 antibody; b) about 1% to about 40% (w/v) trehalose; c) about 0.005% to about 0.5% (w/v) polysorbate 80; d) about 10 mM to about 200 mM arginine; e) about 1 mM to about 50 mM methionine; and f) about 10 mM to about 100 mM histidine; wherein the antibody formulation has a pH of about 5.0 to about 7.0. Described herein is an antibody formulation that comprises: a) 5 mg/mL to 120 mg/mL of an anti-IL-6 antibody; b) 1% to 40% (w/v) trehalose; c) 0.005% to 0.5% (w/v) polysorbate 80; d) 10 mM to 200 mM arginine; e) 1 mM to 50 mM methionine; and f) 10 mM to 100 mM histidine; wherein the antibody formulation has a pH of 5.0 to 7.0.

Described herein is an antibody formulation that comprises: a) about 5 mg/mL to about 120 mg/mL of an anti-IL-6 antibody; b) about 1% to about 40% (w/v) trehalose; c) about 0.02% to about 0.1% (w/v) polysorbate 80; d) about 10 mM to about 200 mM arginine; e) about 1 mM to about 100 mM methionine; and f) about 10 mM to about 100 mM histidine; wherein the antibody formulation has a pH of about 5.0 to about 7.0. Described herein is an antibody formulation that comprises: a) 5 mg/mL to 120 mg/mL of an anti-IL-6 antibody; b) 1% to 40% (w/v) trehalose; c) 0.02% to 0.1% (w/v) polysorbate 80; d) 10 mM to 200 mM arginine; e) 1 mM to 100 mM methionine; and f) 10 mM to 100 mM histidine; wherein the antibody formulation has a pH of 5.0 to 7.0.

Described herein is an antibody formulation that comprises: a) about 5 mg/mL to about 120 mg/mL of an anti-IL-6 antibody; b) about 1% to about 40% (w/v) trehalose; c) about 0.03% to about 0.1% (w/v) polysorbate 80; d) about 10 mM to about 200 mM arginine; e) about 5 mM to about 15 mM methionine; and f) about 10 mM to about 100 mM histidine; wherein the antibody formulation has a pH of about 5.0 to about 7.0. Described herein is an antibody formulation that comprises: a) 5 mg/mL to 120 mg/mL of an anti-IL-6 antibody; b) 1% to 40% (w/v) trehalose; c) 0.03% to 0.1% (w/v) polysorbate 80; d) 10 mM to 200 mM arginine; e) 5 mM to 15 mM methionine; and f) 10 mM to 100 mM histidine; wherein the antibody formulation has a pH of 5.0 to 7.0.

In certain embodiments, the anti-IL-6 antibody comprises a VH CDR1 sequence of SEQ ID NO: 1; a VH CDR2 sequence of SEQ ID NO: 2; a VH CDR3 sequence of SEQ ID NO: 3; a VL CDR1 sequence of SEQ ID NO: 4; a VL CDR2 sequence of SEQ ID NO: 5; and a VL CDR3 sequence of SEQ ID NO: 6. In certain embodiments, the anti-IL-6 antibody comprises a VH domain amino acid sequence of SEQ ID NO: 7 and a VL domain amino acid sequence of SEQ ID NO: 8. In certain embodiments, wherein the anti-IL-6 antibody comprises a heavy chain amino acid sequence of SEQ ID NO: 9 and a light chain amino acid sequence of SEQ ID NO: 10.

In specific embodiments, the anti-IL-6 antibody is the full-length COR-001 antibody. Described herein is a antibody formulation that comprises about 5 mg/mL to about 50 mg/mL COR-001 antibody, such as about 5 mg/mL, about 7.5 mg/mL, about 10 mg/mL, about 15 mg/mL, about 20 mg/mL, about 30 mg/mL, or about 50 mg/mL COR-001 antibody. Described herein is a antibody formulation that comprises 5 mg/mL to 50 mg/mL COR-001 antibody, such as 5 mg/mL, 7.5 mg/mL, 10 mg/mL, 15 mg/mL, 20 mg/mL, 30 mg/mL, or 50 mg/mL COR-001 antibody; an antibody formulation that comprises about 7.5 mg/mL to about 30 mg/mL of COR-001 antibody; an antibody formulation that comprises 7.5 mg/mL to 30 mg/mL of COR-001 antibody; an antibody formulation that comprises about 7.5 mg/mL COR-001 antibody; and antibody formulation that comprises 7.5 mg/mL COR-001 antibody. In certain embodiments, the antibody formulation comprises about 15 mg/mL COR-001 antibody. In certain embodiments, the antibody formulation comprises 15 mg/mL COR-001 antibody. In certain embodiments, the antibody formulation comprises about 30 mg/mL COR-001 antibody. In certain embodiments, the antibody formulation comprises 30 mg/mL COR-001 antibody.

Described herein is an antibody formulation comprises: a) about 5 mg/mL to about 120 mg/mL of COR-001 antibody; b) about 5% (w/v) trehalose; c) about 0.07% (w/v) polysorbate 80; d) about 70 mM arginine; e) about 10 mM methionine; and f) about 20 mM histidine; wherein the antibody formulation has a pH of about 6.0. Described herein is a antibody formulation that comprises: a) 5 mg/mL to 120 mg/mL of COR-001 antibody; b) 5% (w/v) trehalose; c) 0.07% (w/v) polysorbate 80; d) 70 mM arginine; e) 10 mM methionine; and f) 20 mM histidine; wherein the antibody formulation has a pH of 6.0.

Described herein is an antibody formulation that comprises: a) about 7.5 mg/mL to about 30 mg/mL of COR-001 antibody; b) about 5% (w/v) trehalose; c) about 0.07% (w/v) polysorbate 80; d) about 70 mM arginine; e) about 10 mM methionine; and f) about 20 mM histidine; wherein the antibody formulation has a pH of about 6.0. Described herein is an antibody formulation that comprises: a) 7.5 mg/mL to 30 mg/mL of COR-001 antibody; b) 5% (w/v) trehalose; c) 0.07% (w/v) polysorbate 80; d) 70 mM arginine; e) 10 mM methionine; and f) 20 mM histidine; wherein the antibody formulation has a pH of 6.0.

### 6.2.9.1. Viscosity

In some embodiments, the viscosity of the formulation is less than 50 cP at 25°C, such as less than 40 cP, less than 30 cP, less than 20 cP, less than 10 cP, or less than 5 cP at 25°C. In specific embodiments, the formulation has a viscosity of less than 10 cP at 25°C. In various embodiments, the viscosity of the formulation is 1 cP, 2 cP, 3 cP, 4 cP, 5 cP, 10 cP, 15 cP, 20 cP, 25 cP, 30 cP, 35 cP, or 40 cP at 25°C.

### 6.2.9.2. Aggregation

In some embodiments, the formulation reduces the aggregation of the antibody. In certain embodiments, the formulation reduces the formation of soluble aggregates. In certain embodiments, the formulation reduces the formation of insoluble aggregates. In certain embodiments, the formulation reduces the formation of soluble aggregates compared to a formulation with less polysorbate 80, as measured by size exclusion-high-performance liquid chromatography (SEC-HPLC). In certain embodiments, the formulation reduces the formation of insoluble aggregates compared to a formulation with less polysorbate 80, as measured by visual appearance check. In certain embodiments, the formulation reduces the formation of soluble aggregates compared to a formulation without methionine, as measured by SEC-HPLC. In some embodiments, the formulation has less than 15% soluble aggregates after 20 hours of agitation at 300 rpm, as measured by SEC-HPLC. In some embodiments, the formulation has less than 10% soluble aggregates after 20 hours of agitation at 300 rpm, as measured by SEC-HPLC. In some embodiments, the formulation has less than 5% soluble aggregates after 20 hours of agitation at 300 rpm, as measured by SEC-HPLC. In some embodiments, the formulation has less than 2% soluble aggregates after 20 hours of agitation at 300 rpm, as measured by SEC-HPLC.

### 6.2.9.3. Charged Variants

In some embodiments, the formulation reduces the percentage of charged variants, measured as the peaks eluted earlier or later than the main species by imaged capillary isoelectric focusing (icIEF). In certain embodiments, the formulation reduces the percentage of charged variants compared to a formulation without methionine, as measured by icIEF. Charged variants comprise acidic species and basic species.

In some embodiments, the formulation has less than 60% charged variants after storage at 5 + 3°C for 12 months, as measured by icIEF. In some embodiments, the formulation has less than 50% charged variants after storage at 5 + 3°C for 12 months, as measured by icIEF. In some embodiments, the formulation has less than 60% acidic species after storage at 5 + 3°C for 12 months, as measured by icIEF. In some embodiments, the formulation has less than 50% acidic species after storage at 5 + 3°C for 12 months, as measured by icIEF.

In some embodiments, the formulation has less than 60% charged variants after storage at 25 ± 2°C for 6 months, as measured by icIEF. In some embodiments, the formulation has less than 50% charged variants after storage at 25 ± 2°C for 6 months, as measured by icIEF. In some embodiments, the formulation has less than 60% acidic species after storage at 25 ± 2°C for 6 months, as measured by icIEF. In some embodiments, the formulation has less than 50% acidic species after storage at 25 ± 2°C for 6 months, as measured by icIEF.

In some embodiments, the formulation has less than 60% charged variants after storage at 40 ± 2°C for 1 month, as measured by icIEF. In some embodiments, the formulation has less than 50% charged variants after storage at 40 ± 2°C for 1 month, as measured by icIEF. In some embodiments, the formulation has less than 60% acidic species after storage at 40 ± 2°C for 1 month, as measured by icIEF. In some embodiments, the formulation has less than 50% acidic species after storage at 40 ± 2°C for 1 month, as measured by icIEF.

In some embodiments, the formulation reduces the percentage of acidic species, measured as peaks eluted earlier than the main species measured by imaged capillary isoelectric focusing (icIEF). In various embodiments, the measured acidic species are generated by deamidation, isomerization, oxidation or other degradations. In certain embodiments, the formulation reduces the percentage of acidic species compared to a formulation without methionine, as measured by icIEF. In some embodiments, the formulation has less than 60% acidic species after incubation at 45°C for two weeks, as measured by icIEF. In some embodiments, the formulation has less than 50% acidic species after incubation at 45°C for two weeks, as measured by icIEF. In some embodiments, the formulation has less than 40% acidic species after incubation at 45°C for two weeks, as measured by icIEF. In some embodiments, the formulation has less than 30% acidic species after incubation at 45°C for two weeks, as measured by icIEF.

### 6.2.9.4. Oxidation

In some embodiments, the formulation reduces the oxidation of the antibody. In some embodiments, the formulation reduces the oxidation, including oxidation of residue Met431, compared to a formulation without methionine, as measured by reversed phase-high performance liquid chromatography (RP-HPLC). In some embodiments, the formulation has less than 15% oxidized species after incubation at 45°C for two weeks, as measured by RP-HPLC. In some embodiments, the formulation has less than 10% oxidized species after incubation at 45°C for two weeks, as measured by RP-HPLC. In some embodiments, the formulation has less than 5% oxidized species after incubation at 45°C for two weeks, as measured by RP-HPLC. In some embodiments, the formulation has less than 4% oxidized species after incubation at 45°C for two weeks, as measured by RP-HPLC. In some embodiments, the formulation has less than 3% oxidized species after incubation at 45°C for two weeks, as measured by RP-HPLC.

In some embodiments, the formulation has less than 10% oxidized species after storage at 5 + 3°C for 12 months, as measured by RP-HPLC. In various embodiments, the formulation has less than 10%, less than 9%, less than 8%, less than 7%, less than 6%, less than 5%, less than 4%, less than 3%, less than 2%, or less than 1% oxidized species after storage at 5 + 3°C for 12 months, as measured by RP-HPLC. In certain embodiments, the formulation has less than 6% oxidized species after storage at 5 + 3°C for 12 months, as measured by RP-HPLC. In certain embodiments, the formulation has less than 5% oxidized species after storage at 5 + 3°C for 12 months, as measured by RP-HPLC.

In some embodiments, the formulation has less than 10% oxidized species after storage at 25 ± 2°C for 6 months, as measured by RP-HPLC. In various embodiments, the formulation has less than 10%, less than 9%, less than 8%, less than 7%, less than 6%, less than 5%, less than 4%, less than 3%, less than 2%, or less than 1% oxidized species after storage at 25 ± 2°C for 6 months, as measured by RP-HPLC. In certain embodiments, the formulation has less than 6% oxidized species after storage at 25 ± 2°C for 6 months, as measured by RP-HPLC. In certain embodiments, the formulation has less than 5% oxidized species after storage at 25 ± 2°C for 6 months, as measured by RP-HPLC.

In some embodiments, the formulation has less than 10% oxidized species after storage at 40 ± 2°C for 1 month, as measured by RP-HPLC. In various embodiments, the formulation has less than 10%, less than 9%, less than 8%, less than 7%, less than 6%, less than 5%, less than 4%, less than 3%, less than 2%, or less than 1% oxidized species after storage at 40 ± 2°C for 1 month, as measured by RP-HPLC. In certain embodiments, the formulation has less than 6% oxidized species after storage at 40 ± 2°C for 1 month, as measured by RP-HPLC. In certain embodiments, the formulation has less than 5% oxidized species after storage at 40 ± 2°C for 1 month, as measured by RP-HPLC.

### 6.2.9.5. Potency

In some embodiments, the formulation preserves the potency of the antibody. In various embodiments, the potency of an anti-IL-6 antibody is measured by IL-6 binding ELISA or HEK Blue cell-based bioassay.

In some embodiments, the formulation has less than 50% reduction in potency after storage at 5 + 3°C for 12 months, as measured by IL-6 binding ELISA. In various embodiments, the formulation has less than 50%, less than 40%, less than 30%, less than 20%, or less than 10% reduction in potency after storage at 5 + 3°C for 12 months, as measured by IL-6 binding ELISA. In certain embodiments, the formulation has less than 30% reduction in potency after storage at 5 + 3°C for 12 months, as measured by IL-6 binding ELISA. In certain embodiments, the formulation has less than 20% reduction in potency after storage at 5 + 3°C for 12 months, as measured by IL-6 binding ELISA. In certain embodiments, the formulation has less than 10% reduction in potency after storage at 5 + 3°C for 12 months, as measured by IL-6 binding ELISA.

In some embodiments, the formulation has less than 50% reduction in potency after storage at 5 + 3°C for 12 months, as measured by HEK Blue cell-based bioassay. In various embodiments, the formulation has less than 50%, less than 40%, less than 30%, less than 20%, or less than 10% reduction in potency after storage at 5 + 3°C for 12 months, as measured by HEK Blue cell-based bioassay. In certain embodiments, the formulation has less than 30% reduction in potency after storage at 5 + 3°C for 12 months, as measured by HEK Blue cell-based bioassay. In certain embodiments, the formulation has less than 20% reduction in potency after storage at 5 + 3°C for 12 months, as measured by HEK Blue cell-based bioassay. In certain embodiments, the formulation has less than 10% reduction in potency after storage at 5 + 3°C for 12 months, as measured by HEK Blue cell-based bioassay.

In some embodiments, the formulation has less than 50% reduction in potency after storage at 25 ± 2°C for 6 months, as measured by IL-6 binding ELISA. In various embodiments, the formulation has less than 50%, less than 40%, less than 30%, less than 20%, or less than 10% reduction in potency after storage at 25 ± 2°C for 6 months, as measured by IL-6 binding ELISA. In certain embodiments, the formulation has less than 30% reduction in potency after storage at 25 ± 2°C for 6 months, as measured by IL-6 binding ELISA. In certain embodiments, the formulation has less than 20% reduction in potency after storage at 25 ± 2°C for 6 months, as measured by IL-6 binding ELISA. In certain embodiments, the formulation has less than 10% reduction in potency after storage at 25 ± 2°C for 6 months, as measured by IL-6 binding ELISA.

In some embodiments, the formulation has less than 50% reduction in potency after storage at 25 ± 2°C for 6 months, as measured by HEK Blue cell-based bioassay. In various embodiments, the formulation has less than 50%, less than 40%, less than 30%, less than 20%, or less than 10% reduction in potency after storage at 25 ± 2°C for 6 months, as measured by HEK Blue cell-based bioassay. In certain embodiments, the formulation has less than 30% reduction in potency after storage at 25 ± 2°C for 6 months, as measured by HEK Blue cell-based bioassay. In certain embodiments, the formulation has less than 20% reduction in potency after storage at 25 ± 2°C for 6 months, as measured by HEK Blue cell-based bioassay. In certain embodiments, the formulation has less than 10% reduction in potency after storage at 25 ± 2°C for 6 months, as measured by HEK Blue cell-based bioassay.

In some embodiments, the formulation has less than 50% reduction in potency after storage at 40 ± 2°C for 1 month, as measured by IL-6 binding ELISA. In various embodiments, the formulation has less than 50%, less than 40%, less than 30%, less than 20%, or less than 10% reduction in potency after storage at 40 ± 2°C for 1 month, as measured by IL-6 binding ELISA. In certain embodiments, the formulation has less than 30% reduction in potency after storage at 40 ± 2°C for 1 month, as measured by IL-6 binding ELISA. In certain embodiments, the formulation has less than 20% reduction in potency after storage at 40 ± 2°C for 1 month, as measured by IL-6 binding ELISA. In certain embodiments, the formulation has less than 10% reduction in potency after storage at 40 ± 2°C for 1 month, as measured by IL-6 binding ELISA.

In some embodiments, the formulation has less than 50% reduction in potency after storage at 40 ± 2°C for 1 month, as measured by HEK Blue cell-based bioassay. In various embodiments, the formulation has less than 50%, less than 40%, less than 30%, less than 20%, or less than 10% reduction in potency after storage at 40 ± 2°C for 1 month, as measured by HEK Blue cell-based bioassay. In certain embodiments, the formulation has less than 30% reduction in potency after storage at 40 ± 2°C for 1 month, as measured by HEK Blue cell-based bioassay. In certain embodiments, the formulation has less than 20% reduction in potency after storage at 40 ± 2°C for 1 month, as measured by HEK Blue cell-based bioassay. In certain embodiments, the formulation has less than 10% reduction in potency after storage at 40 ± 2°C for 1 month, as measured by HEK Blue cell-based bioassay.

### 6.3. Administration of the Formulation

The suitable routes of administration for the antibody formulations described herein include are but not limited to, parenterally (such as by subcutaneous, intravenous, intramuscular, intradermal, or intrasternal injection or infusion (e.g., as sterile injectable aqueous or nonaqueous solutions or suspensions, etc.)), and topically (such as in the form of a cream or ointment). In certain embodiments, the formulations are suitable for parenteral injection. In certain embodiments, the formulations are suitable for intravenous injection. In certain embodiments, the formulations are suitable for subcutaneous injection.

In some embodiments, the formulations are suitable for administration in single or multiple doses.

### 6.4. Dosage Form

In another aspect, provided herein are dosage forms containing one or more unit doses of a pharmaceutical composition comprising an anti-IL-6 antibody.

In various embodiments, the dosage form is a pre-filled syringe. In various embodiments, the dosage form is an auto-inject pen.

In various embodiments, the dosage form comprises one or more unit doses of a formulation as described hereinabove in Section 6.2. In typical embodiments, the formulation is a liquid formulation. In other embodiments, the formulation is a dry formulation, including but not limited to a lyophilate. In particular embodiments, the dosage form comprises a dry formulation and a measured quantity of aqueous diluent.

In some embodiments, the unit dosage form comprises a formulation comprising a) about 5 mg/mL to about 120 mg/mL of an anti-IL-6 antibody; b) about 1% to about 40% (w/v) trehalose; c) about 0.03% to about 0.1% (w/v) polysorbate 80; d) about 10 mM to about 200 mM arginine; e) about 5 mM to about 15 mM methionine; and f) about 10 mM to about 100 mM histidine; wherein the formulation has a pH of about 5.0 to about 7.0. In some embodiments, the unit dosage form comprises a formulation comprising a) 5 mg/mL to 120 mg/mL of an anti-IL-6 antibody; b) 1% to 40% (w/v) trehalose; c) 0.03% to 0.1% (w/v) polysorbate 80; d) 10 mM to 200 mM arginine; e) 5 mM to 15 mM methionine; and f) 10 mM to 100 mM histidine; wherein the formulation has a pH of 5.0 to 7.0.

In some embodiments, the unit dosage form comprises a formulation comprising a) about 7.5 mg/mL to about 30 mg/mL of COR-001; b) about 5% (w/v) trehalose; c) about 0.07% (w/v) polysorbate 80; d) about 70 mM arginine; e) about 10 mM methionine; and f) about 20 mM histidine; wherein the formulation has a pH of about 6.0. In certain embodiments, the unit dosage form comprises about 7.5 mg of COR-001. In certain embodiments, the unit dosage form comprises about 15 mg of COR-001. In certain embodiments, the unit dosage form comprises about 30 mg of COR-001. In some embodiments, the unit dosage form comprises a formulation comprising a) 7.5 mg/mL to 30 mg/mL of COR-001; b) 5% (w/v) trehalose; c) 0.07% (w/v) polysorbate 80; d) 70 mM arginine; e) 10 mM methionine; and f) 20 mM histidine; wherein the formulation has a pH of 6.0.

In certain embodiments, the dosage form comprises a plurality of 7.5 mg unit doses of COR-001. In certain embodiments, the dosage form comprises a single 7.5 mg unit dose of COR-001. In certain embodiments, the dosage form comprises a plurality of 15 mg unit doses of COR-001. In certain embodiments, the dosage form comprises a single 15 mg unit dose of COR-001. In certain embodiments, the unit dosage form comprises a plurality of 30 mg unit doses of COR-001. In certain embodiments, the unit dosage form comprises a single 30 mg unit dose of COR-001.

### 6.5. Methods of Treatment

Disclosed herein is an anti-IL-6 antibody formulation as described herein for use in methods of treating a disease or disorder in a patient .

In some embodiments of the present disclosure, the patient has an IL-6 mediated inflammatory disorder.

In various embodiments of the present disclosure, the patient has elevated pre-treatment levels of C-reactive protein (CRP). In some embodiments, the patient has a pre-treatment CRP level of at least 2 mg/L. In some embodiments, the patient has a pre-treatment CRP level of at least 2 mg/L, 2.5 mg/L, 3 mg/L, 3.5 mg/L, 4 mg/L, 4.5 mg/L, or 5 mg/L. In some embodiments, the patient has a pre-treatment CRP level of at least 7.5 mg/L, 10 mg/L, 12.5 mg/L, or 15 mg/L.

In some embodiments of the present disclosure, the IL-6 mediated inflammatory disorder is a hepcidin-mediated disorder. Hepcidin-mediated disorders are described in US 2017/0029499, incorporated herein by reference in its entirety.

In some embodiments of the present disclosure, the IL-6 mediated inflammatory disorder is not a hepcidin-mediated disorder.

In some embodiments of the present disclosure, the patient has a non-autoimmune IL-6 mediated inflammatory disorder. In particular embodiments, the patient has an IL-6 mediated inflammatory disorder other than rheumatoid arthritis, giant cell arteritis, polyarticular juvenile idiopathic arthritis, or systemic juvenile idiopathic arthritis.

In various embodiments of the present disclosure, the patient has kidney disease. In some embodiments, the kidney disease is chronic kidney disease (CKD). In some embodiments, the patient has KDOQI stage 1-5 chronic kidney disease. In some embodiments, the patient has KDOQI stage 3-5 chronic kidney disease. In some embodiments, the patient is on dialysis. In some embodiments, the patient is not on dialysis. In certain embodiment, the patient has KDOQI stage 3-5 chronic kidney disease, wherein the patient is not on dialysis. In certain embodiment, the patient has KDOQI stage 3-5 chronic kidney disease, wherein the patient is on dialysis. In some embodiments, the patient has cardiorenal syndrome (CRS). In certain embodiments, the patient has CRS Type 4. In some embodiments, the patient has been treated with dialysis.

In particular embodiments of the present disclosure, the patient has KDOQI stage 3-5 chronic kidney disease and has a CRP level of 2 mg/L or more. In certain embodiments, the formulation is administered to reduce the risk of cardiovascular morbidity and mortality in adult KDOQI stage 3-5 chronic kidney disease patients with inflammation.

In various embodiments of the present disclosure, the patient has cardiovascular disease.

In particular embodiment of the present disclosure s, the patient has atherosclerotic heart disease. In particular embodiments, the patient has atherosclerosis and has a CRP level of 2 mg/L or more. In certain embodiments, the formulation is administered to reduce the risk of cardiovascular morbidity and mortality in adult atherosclerotic cardiovascular disease patients with inflammation.

In some embodiments of the present disclosure, the patient has had a previous myocardial infarction. In some embodiments, the patient has not had a previous myocardial infarction.

In particular embodiments of the present disclosure, the patient has had a previous myocardial infarction and has a CRP level of 2 mg/L or more.

In some embodiments of the present disclosure, the cardiovascular disease is congestive heart failure (CHF). In certain embodiments, the patient has congestive heart failure (CHF) with reduced ejection fraction. In certain embodiments, the patient has congestive heart failure (CHF) with mid-range ejection fraction. In certain embodiments, the patient has congestive heart failure (CHF) with preserved ejection fraction. In some embodiments, the cardiovascular disease is acute coronary syndrome. In certain embodiments, the anti-IL-6 antibody formulation is administered at a dose sufficient to reduce nonfatal myocardial infarction, nonfatal stroke, and/or cardiovascular death. In some embodiments, the anti-IL-6 antibody formulation is administered at a dose sufficient to reduce the risk of heart failure. In some embodiments, the anti-IL-6 antibody formulation is administered at a dose sufficient to increase cardiac function. In some embodiments, the anti-IL-6 antibody formulation is administered at a dose sufficient to reduce fibrosis after acute myocardial infarction. In some embodiments, the anti-IL-6 antibody formulation is administered at a dose sufficient to reduce the risk of cardiovascular morbidity and mortality.

In some embodiments of the present disclosure, the cardiovascular disease is heart failure that is not diuretic resistant. In some other embodiments, the cardiovascular disease is heart failure that is diuretic resistant. Diuretic resistant heart failure is described in WO 2018/144773, the disclosure of which is incorporated herein by reference in its entirety.

In various embodiments of the present disclosure, the patient has anemia. In some embodiments, the patient has anemia of chronic disease. In some embodiments, the patient has iron-refractory iron-deficiency anemia (IRIDA).

In some embodiments of the present disclosure, the patient has diabetes. In some embodiments, the patient has liver disease. In some embodiments, the patient has osteoporosis. In some embodiments, the patient has depression. In some embodiments, the patient has asthma. In some embodiments, the patient has neuroinflammatory disorder, such as Alzheimer's disease, Parkinson's disease, multiple sclerosis, and amyotrophic lateral sclerosis (ALS). In some embodiments, the patient has age-related macular degeneration (AMD). In various embodiments, the patient has cancer such as solid tumors, small cell lung cancer, non-small cell lung cancer, hematological cancer, multiple myeloma, leukemia, chronic lymphocytic leukemia (CLL), chronic myeloid leukemia (CML), lymphomas, and Hodgkin's lymphoma. In some embodiments, the patient has skin disease. In some embodiments, the anti-IL-6 antibody formulation prevents aging in the patient.

### 6.6. Examples

Below are examples of specific embodiments for carrying out the present disclosure. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

The practice of the present disclosure will employ, unless otherwise indicated, conventional methods of protein chemistry, biochemistry, recombinant DNA techniques and pharmacology, within the skill of the art. Such techniques are explained fully in the literature.

### Example 1: Minimizing Viscosity At High Protein Concentrations

The final ultrafiltration/diafiltration step in the manufacturing process of COR-001 requires a transient overconcentration exceeding 100 mg/mL. To minimize the risk of filtration flux decay and membrane fouling, a viscosity lower than 10 cP is desired.

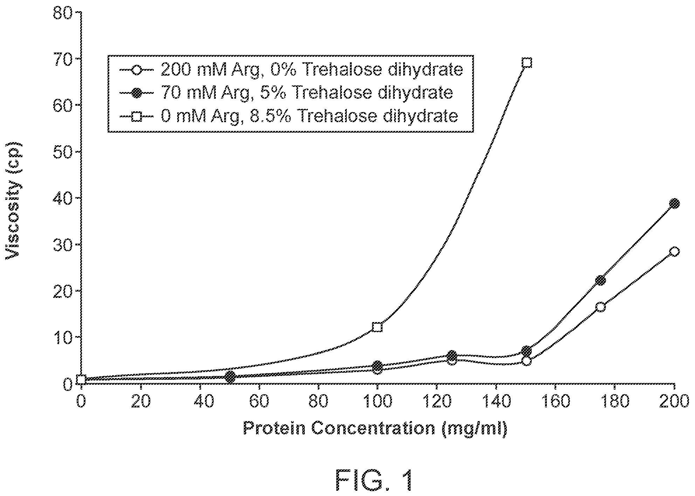

To reduce viscosity, several excipients were assessed and L-arginine salts (e.g. L-arginine-HCl) were identified to be most effective. As shown in FIG. 1, viscosity remained below 10 cP for up to 150 mg/ml COR-001 in the presence of 70 mM or 200 mM L-arginine-HCl.

In an effort to leverage the thermal stabilization imparted by trehalose, a combination of L-arginine-HCl and trehalose dihydrate that rendered an isotonic composition (i.e. 5% (w/v) of trehalose dihydrate) was selected. As shown in FIG. 1, 100 mg/mL COR-001 in 70 mM L-arginine-HCl and 5% trehalose dihydrate had a viscosity of 4 cP. 70 mM L-arginine-HCl and 5% trehalose dihydrate was selected for COR-001 liquid formulation.

### Example 2: Minimizing Agitation-Induced Aggregation

Proteins in liquid formulation are susceptible to agitation-induced aggregation during handling and transportation. In an attempt to prevent agitation-induced protein aggregation, surfactants (e.g. polysorbate) were assessed for inclusion in the COR-001 formulation.

A polysorbate 80 (PS80) range of 0% to 0.10% (w/v) was assessed at 5, 50, and 120 mg/mL COR-001 in 5% trehalose dihydrate, 70 mM arginine-HCL, 10 mM methionine, 20 mM histidine, pH 6.0. After these formulations (1.2 mL in 3 mL glass vials) were exposed under extreme shaking stress condition (20 hours of agitation at 300 rpm under ambient condition), appearance, OD340, soluble aggregates by SEC-HPLC and sub-visible particle counts by MFI were analyzed.

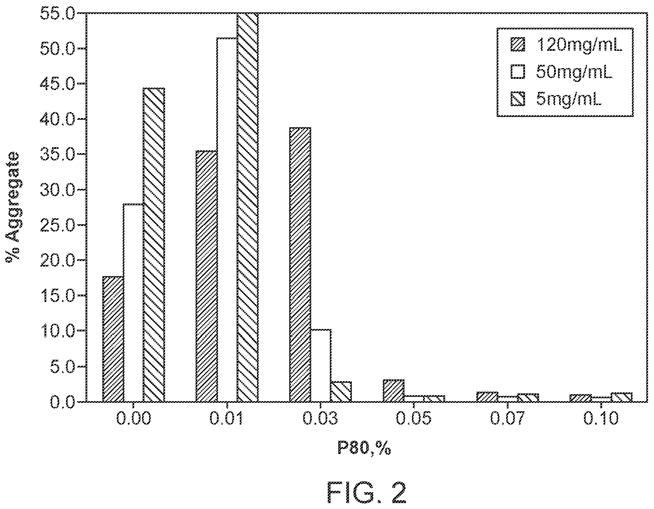

Without PS80, precipitates were observed in all formulations after agitation. With increasing PS80 level, the less turbid the product solution appeared; when PS80 level reached 0.05% or above, no particle or opalescence were observed after agitation. This appearance trend also correlated well with the OD340 and SEC results. FIG. 2 shows the effect of PS80 level on the formation of soluble aggregates by SEC. The results indicate that PS80 at 0.03% can reduce the soluble aggregates for protein concentrations at or below 50 mg/mL and PS80 at 0.05% can reduce the soluble aggregates for protein concentrations ranging from 5 to 120 mg/mL under the stress shaking model. A level of 0.07% was selected for PS80 in COR-001 liquid formulation.

### Example 3: Minimizing Oxidation Over Long Term Storage

To assess the long-term storage stability of COR-001 in the selected liquid formulation (5% trehalose dihydrate, 70 mM L-arginine-HCl, 0.07% PS80, 20 mM L-histidine, pH 6.0), a range of protein concentrations (20, 50, and 120 mg/mL) were prepared and filled at 1.2 mL into 3 mL Type I glass vials closed with 13 mm rubber stoppers. The results of long-term storage at 2-8°C are summarized in Table 1.

| **Table 1: COR-001 Formulation Stability after Long-term Storage at 2 to 8°C** | | | | | | |
|---|---|---|---|---|---|---|
| **Protein Concentration** | **20 mg/mL^{a}** | | **50 mg/mL^{a}** | | **120 mg/mL** | |
| **Timepoint** | **T0** | **12 months** | **T0** | **12 months** | **T0** | **9 months** |
| SEC | | | | | | |
| % Monomer | 99.4 | 98.9 | 99.5 | 99.3 | 98.7 | 97.4 |
| Non-reduced CE-SDS | | | | | | |
| % IgG | 97.6 | 97.2 | 96.2 | 95.2 | 95.2 | 94.2 |
| icIEF, | | | | | | |
| % Main species | 63.5 | 51.7 | 64.3 | 59.1 | 59.8 | 50.7 |
| % Acidic species | 29.1 | 42.5 | 28.7 | 34.1 | 34.0 | 43.2 |
| % Basic species | 7.4 | 5.8 | 6.9 | 6.8 | 6.2 | 6.1 |
| Tryptic map/LC-MS | | | | | | |
| Met431 % oxidation^{b} | 1.4 | 45.5 | 3.2 | 16.2 | 2.6 | 18.5 |
| IL-6 binding ELISA | | | | | | |
| (% of assay control) ^{c} | 61 | 84 | 70 | 89 | 91 | 79 |

| | | | | | | |
|---|---|---|---|---|---|---|
| ^{a} PS80 was 0.05% instead of 0.07%; ^{b} Oxidation level was determined by tryptic map/LC-MS; ^{c} Relative binding results are reported as percentage of a Phase I DS lot. T0 test results are those from frozen aliquots. | | | | | | |

After 9-12 months, there were minimal changes in purity as observed by SE-HPLC and nonreduced CE-SDS. However, a 5-10% decrease in the % main isoform was observed by icIEF, with a concomitant increase in the acidic species. A significant increase in oxidation (16-46%) was observed at all three protein concentrations by tryptic map/LC-MS analysis. No apparent trend in the potency of COR-001 was observed by IL-6 binding ELISA, indicating that the highly oxidized COR-001 had no impact on the potency, consistent with the oxidation site being primarily on the heavy chain Met431 and not in the CDRs.

Although no loss of apparent potency was observed, oxidation products are undesirable.

To assess whether addition of a third amino acid, methionine, to the formulation containing the two amino acids, arginine and histidine, could reduce or prevent oxidation on long term storage, 10 mg/mL and 50 mg/mL COR-001 each were spiked with L-methionine ranged from 0 to 15 mM (5% trehalose dihydrate, 70 mM L-arginine-HCl, 0.07% PS80, 20 mM L-histidine, pH 6.0), and then incubated under the accelerated condition of 45°C for 2 weeks. The samples were tested by appearance, SEC, CE-SDS, and icIEF as well as oxidation assay (RP-HPLC) to measure oxidized species. No apparent effect of various L-methionine levels included was observed by appearance and CE-SDS testing. However, with increasing L-methionine level, less changes were observed in SEC, icIEF, and oxidation testing. As shown in FIGs. 3A, 3B, and 3C, after 2 weeks under the accelerated condition of 45°C, comparing to the formulations without adding L-methionine, L-methionine reduced the aggregation, charge change and oxidation level for both 10 mg/mL and 50 mg/mL COR-001. At 10 mM L-methionine, these change levels essentially reached a plateau, indicating that 10 mM L-methionine is sufficient to stabilize COR-001 under thermal stress conditions. 10 mM methionine was selected for COR-001 liquid formulation.

### Example 4: Effect Of Protein Concentration On Storage Stability

To assess the effect of protein concentration on COR-001 stability upon storage, two different protein concentrations (10 and 50 mg/mL) in the selected liquid formulation (5% trehalose dihydrate, 70 mM L-arginine-HCl, 0.07% PS80, 10 mM L-methionine, 20 mM L-histidine, pH 6.0), were prepared and were filled at 1.0 mL into 3 mL Type I glass vials closed with 13 mm rubber stoppers. The samples after 3 months storage at 5 ± 3°C, 25 ± 2°C, and 40 ±2°C were tested. The results are summarized in Table 2.

| **Table 2: Effect of Protein Concentration on COR-001 Stability after 3 Months** | | | | | | |
|---|---|---|---|---|---|---|
| **Testing** | **5±3°C** | | **25±2°C** | | **40±2°C** | |
| | **50 mg/mL** | **10 mg/mL** | **50 mg/mL** | **10 mg/mL** | **50 mg/mL** | **10 mg/mL** |
| Appearance - Color | <BY7 | <BY7 | <BY7 | <BY7 | <BY6 | BY7 |
| Appearance - Clarity (NTU) | 30 - 50 | 6 - 18 | 30 - 50 | 6-18 | 60 - 80 | 18-30 |
| Visible particles | 0 | 0 | 0 | 0 | 0 | 0 |
| pH | 5.9 | 5.9 | 6.0 | 6.0 | 5.9 | 6.0 |
| Protein concentration | 53.3 | 10.7 | 55.4 | 10.1 | 53.0 | 10.8 |
| SEC | | | | | | |
| % Monomer | 98.6 | 98.7 | 97.7 | 97.9 | 93.0 | 94.2 |
| % HMW | 1.2 | 1.0 | 1.7 | 1.1 | 3.9 | 2.8 |
| % LMW | 0.2 | 0.4 | 0.6 | 1.0 | 3.1 | 3.1 |
| Nonreduced CE-SDS | | | | | | |
| % IgG | 92.3 | 92.7 | 89.8 | 90.7 | 78.4 | 83.6 |
| % HHL | 5.0 | 4.8 | 6.0 | 5.3 | 5.5 | 5.5 |
| Reduced CE-SDS | | | | | | |
| % HC+LC | 97.1 | 97.8 | 96.9 | 97.4 | 89.4 | 92.7 |
| icIEF | | | | | | |
| % Main | 58.8 | 58.8 | 50.4 | 46.3 | 24.6 | 21.6 |
| % Acidic | 34.7 | 34.8 | 42.9 | 47.1 | 68.4 | 72.0 |
| % Basic | 6.5 | 6.6 | 6.6 | 6.6 | 7.1 | 6.5 |
| RP-HPLC | | | | | | |
| % Oxidized | 2.7 | 2.5 | 4.0 | 4.7 | 3.6 | 5.4 |
| IL-6 binding ELISA | | | | | | |
| % of Ref Std | 95 | 83 | 90 | 81 | 81 | 78 |
| Cell based bioassay | | | | | | |
| % of Ref Std | 76 | 86 | 79 | 104 | 97 | 97 |

50 mg/mL had lower clarity than 10 mg/mL due to higher protein concentration. No visible particles were observed after 3 months at all storage conditions. Comparing to 50 mg/mL samples, 10 mg/mL samples at accelerated temperatures (25 ± 2°C and 40 ±2°C) had less changes tested by SEC and CE-SDS assays, but more changes tested by icIEF and oxidation assays. However, the differences of these changes were considered small given the assay variability of these methods. No apparent changes were observed in the potency assays after 3 months at all storage conditions. Overall, the results indicate that there are no apparent differences in COR-001 stability at a concentration range of 10 mg/mL to 50 mg/mL.

### Example 5: Stability Of Different Lots of COR-001

To assess the long-term storage stability of COR-001 in the selected liquid formulation, different lots of COR-001 were formulated into the liquid formulation buffer (70 mM L-arginine hydrochloride, 5 w/v% trehalose dihydrate, 10 mM L-methionine, 0.07 w/v% polysorbate 80, 20 mM L-histidine, pH 6.0), and filled at 1.3 mL each into a container closure system (2R Type I glass vial with 13 mm Flurotec^{®} coated butyl rubber stopper). The COR-001 protein concentration of each lot is summarized in Table 3.

| **Table 3** | |
|---|---|
| **Lot Number** | **Protein Concentration** |
| STC-261-P221-S24 | 30 mg/mL |
| VVRG56 | 30 mg/mL |
| CMC-M-0060 | 30 mg/mL |
| CMC-M-0061 | 15 mg/mL |
| CMC-N-0011 | 7.5 mg/mL |

Lot STC-261-P221-S24 was prepared in a development lab and is intended for development use only. Lot VVRG56 is also intended for development use. Lot CMC-M-0060, lot CMC-M-0061, and lot CMC-N-0011 are the clinical drug product lots.

The studies include an evaluation under the long term storage condition of 5 ± 3°C, the accelerated condition of 25 ± 2°C/60% ± 5% relative humidity (RH), and the stress condition of 40 ± 2°C/75% ± 5% RH. All the stability containers were placed in inverted orientation to simulate the worst case scenario of contact with the stopper surface.

### A. Stability Results under Accelerated and Stress Conditions

The stabilities of each lot of COR-001 prepared in the formulation stored under accelerated and stress conditions are summarized in the Tables 4 to 13 below, with Tables 4 to 8 showing the results under the accelerated condition for up to 12 months and Tables 9 to 13 showing the results under the stress condition for up to 3 months.

| **Table 4: Stability Data for COR-001 Drug Product Lot STC-261-P221-S24 (30 mg/mL) at 25 ± 2°C** | | | | | |
|---|---|---|---|---|---|
| **Test** | **Acceptance Criteria ^{a}** | **Time points (Months)** | | | |
| | | **0** | **1** | **3** | **6** |
| Appearance | Report result (visible particles) | 0 | 0 | 0 | 0 |
| Clarity | Report result | < IV | < IV | < IV | > IV |
| Color | Report result | < BY7 | < BY7 | < BY6 | < BY6 |
| pH | 5.5 - 6.5 | 6.2 | 6.2 | 5.9 | 5.9 |
| Protein concentration | Report result | 29.6 | 29.8 | 28.7 | 28.5 |
| | Monomer peak: ≥ 95.0% | 98.8 | 98.2 | 97.5 | 97.4 |
| SEC | HMW species: ≤ 5.0% | 1.0 | 1.4 | 1.6 | 1.7 |
| | LMW species: report result | 0.2 | 0.5 | 0.9 | 0.8 |
| Nonreduced CE-SDS | %Main peak: Report result | 93.9 | 93.7 | 93.1 | 90.7 |
| | %HHL peak: Report result | 4.2 | 3.8 | 4.0 | 5.5 |
| Reduced CE-SDS | % LC + % HC: ≥ 95.0% | 97.8 | 97.8 | 97.5 | 97.8 |
| icIEF | %Main: Report result | 60.2 | 49.5 | 46.6 | 44.5 |
| | %Acidic: Report result | 33.1 | 43.5 | 47.0 | 50.7 |
| | %Basic: Report result | 6.8 | 6.8 | 6.5 | 4.9 |
| Oxidation | Report % oxidation | 1.7 | 4.2 | 4.3 | 5.2 |
| IL-6 binding ELISA | 50% - 150% of Reference Standard | 97 | 110 | 97 | 82 |
| Cell based bioassay | 50% - 150% of Reference Standard | 106 | 92 | 95 | 121 |

| | | | | | |
|---|---|---|---|---|---|
| ^{a} The acceptance criteria listed here are for comparison purposes only. | | | | | |

| **Table 5: Stability Data for COR-001 Drug Product Lot VVRG56 (30 mg/mL) at 25 ± 2°C** | | | | | | |
|---|---|---|---|---|---|---|
| **Test** | **Acceptance Criteria ^{a}** | **Time points (Months)** | | | | |
| | | **0** | **1** | **3** | **6** | **12** |
| Appearance | Report result | b | b | b | b | b |
| Clarity | Report result | ≤ II | ≤ III | ≤ III | ≤ III | ≤ III |
| Color | Report result | ≤ BY7 | ≤ BY7 | ≤ BY7 | ≤ BY7 | ≤ BY6 |
| pH | Report result | 6.2 | 6.2 | 6.2 | 6.2 | 6.1 |
| Protein concentration | 30.0 ± 5 mg/mL | 30.8 | 30.7 | 30.9 | 31.0 | 30.7 |
| SE-UHPLC | Monomer peak: ≥ 95.0% | 98.8 | 98.3 | 97.6 | 96.5 | 95.8 |
| | HMW species: ≤ 5.0% | 0.7 | 0.8 | 1.1 | 1.2 | 1.4 |
| | LMW species: report result | 0.5 | 0.9 | 1.3 | 2.3 | 2.9 |
| Nonreduced CE-SDS | % IgG: ≥ 85.0% | 95.5 | 96.6 | 95.0 | 95.0 | 92.4 |
| | % HHL: report result | 2.7 | 1.6 | 2.1 | 2.0 | 2.7 |
| Reduced CE-SDS | % LC + % HC: ≥ 95.0% | 98.4 | 97.3 | 97.4 | 97.7 | 96.8 |
| icIEF | %Main: Report result | 73.5 | 68.4 | 63.3 | 59.5 | 49.8 |
| | %Acidic: Report result | 18.0 | 22.7 | 28.5 | 31.3 | 41.7 |
| | %Basic: Report result | 8.5 | 8.8 | 8.2 | 9.2 | 8.5 |
| Oxidation | Report % oxidation | 2.7 | 3.2 | 4.5 | 5.8 | NA |
| IL-6 binding ELISA | 50% - 150% of Reference Standard | 103 | 98 | 84 | 100 | 95 |
| Cell based bioassay | 50% - 150% of Reference Standard | 97 | 85 | 99 | 109 | 98 |

| | | | | | | |
|---|---|---|---|---|---|---|
| ^{a} The acceptance criteria listed here are for comparison purposes only. ^{b} Clear to opalescent, colorless to slightly brown yellow solution, essentially free of visible particles. | | | | | | |

| **Table 6: Stability Data for COR-001 Drug Product Lot CMC-M-0060 (30 mg/mL) at 25 ± 2°C** | | | | | |
|---|---|---|---|---|---|
| **Test** | **Acceptance Criteria ^{a}** | **Time points (Months)** | | | |
| | | **0** | **1** | **3** | **6** |
| Appearance | Clear to opalescent, colorless to slightly brown yellow solution essentially free of visible particles | b | b | b | b |
| Clarity | ≤ Reference IV | ≤ III | ≤ II | ≤ II | ≤ II |
| Color | ≤ Reference BY4 | ≤ BY7 | ≤ BY6 | ≤ BY6 | ≤ BY6 |
| pH | 5.7 - 6.3 | 6.1 | 6.1 | 6.1 | 6.1 |
| Polysorbate 80 | Report result (w/v%) | 0.07 | 0.06 | 0.05 | 0.05 |
| Protein concentration | 30.0 ± 4.5 mg/mL | 30.8 | 30.4 | 30.7 | 30.5 |
| SE-UHPLC | Monomer peak: ≥ 95.0% | 98.3 | 97.7 | 96.7 | 96.1 |
| | HMW species: ≤ 5.0% | 0.9 | 1.2 | 1.5 | 1.5 |
| | LMW species: report result | 0.7 | 1.2 | 1.9 | 2.4 |
| Nonreduced CE-SDS | % IgG: ≥ 85.0% | 96.0 | 94.9 | 93.4 | 92.3 |
| | % HHL: report result | 2.4 | 2.6 | 3.1 | 3.3 |
| Reduced CE-SDS | % LC + % HC: ≥ 95.0% | 97.8 | 96.9 | 96.8 | 96.6 |
| icIEF | %Main: Report result | 65.7 | 62.5 | 55.7 | 46.6 |
| | %Acidic: Report result | 28.0 | 31.2 | 37.7 | 46.1 |
| | %Basic: Report result | 6.3 | 6.3 | 6.6 | 7.4 |
| Oxidation | Report % oxidation | 2.4 | 3.8 | 4.2 | 4.1 |
| IL-6 binding ELISA | 50% - 150% of Reference Standard | 91 | 93 | 90 | 92 |
| Cell based bioassay | 50% - 150% of Reference Standard | 87 | 85 | 107 | 93 |
| Sub-visible particulates (HIAC) | ≤ 600 particles/container for ≥25µm | 0 | 1 | NT^{c} | NT^{c} |
| | ≤ 6000 particles/container for ≥10µm | 19 | 15 | | |
| Sub-visible particulates (MFI) | Report results | | | | |
| | #particles/container for ≥25 µm | 4 | 3 | 1 | 1 |
| | #particles/container for ≥10 µm | 39 | 51 | 14 | 162 |
| | #particles/container for ≥2 µm | 2322 | 4402 | 3975 | 9299 |
| Endotoxin | ≤ 15 EU/mL | <2.5 | <2.5 | NT^{c} | NT^{c} |
| CCIT | Pass (No blue coloration) | Pass | Pass | NT^{c} | NT^{c} |

| | | | | | |
|---|---|---|---|---|---|
| ^{a} The acceptance criteria listed here are for comparison purposes only. ^{b} Clear to opalescent, colorless to slightly brown yellow solution, essentially free of visible particles. ^{c} NT = Not Tested | | | | | |

| **Table 7: Stability Data for COR-001 Drug Product Lot CMC-M-0061 (15 mg/mL) at 25 ± 2°C** | | | | | |
|---|---|---|---|---|---|
| **Test** | **Acceptance Criteria ^{a}** | **Time points (Months)** | | | |
| | | **0** | **1** | **3** | **6** |
| Appearance | Clear to opalescent, colorless to slightly brown yellow solution essentially free of visible particles | b | b | b | b |
| Clarity | ≤ Reference IV | ≤ III | < II | < I | < I |
| Color | ≤ Reference BY4 | < BY7 | < BY6 | < BY7 | < BY7 |
| pH | 5.7 - 6.3 | 6.2 | 6.1 | 6.1 | 6.1 |
| Polysorbate 80 | Report result (w/v%) | 0.07 | 0.05 | 0.04 | 0.04 |
| Protein concentration | 15.0 ± 2.3 mg/mL | 15.0 | 15.2 | 15.3 | 15.2 |
| SE-UHPLC | Monomer peak: ≥ 95.0% | 98.3 | 96.8 | 96.3 | 95.6 |
| | HMW species: ≤ 5.0% | 0.9 | 1.4 | 1.5 | 1.6 |
| | LMW species: report result | 0.8 | 1.8 | 2.2 | 2.8 |
| Nonreduced CE-SDS | % IgG: ≥ 85.0% | 95.9 | 94.6 | 93.1 | 91.9 |
| | % HHL: report result | 2.3 | 2.6 | 3.1 | 3.3 |
| Reduced CE-SDS | % LC + % HC: ≥ 95.0% | 98.0 | 97.5 | 96.5 | 96.5 |
| icIEF | %Main: Report result | 66.2 | 56.5 | 51.8 | 42.3 |
| | %Acidic: Report result | 27.8 | 37.9 | 42.2 | 50.6 |
| | %Basic: Report result | 6.0 | 5.6 | 6.0 | 7.1 |
| Oxidation | Report % oxidation | 2.5 | 4.7 | 4.4 | 5.1 |
| IL-6 binding ELISA | 50% - 150% of Reference Standard | 96 | 96 | 91 | 88 |
| Cell based bioassay | 50% - 150% of Reference Standard | 105 | 87 | 98 | 91 |
| Sub-visible particulates (HIAC) | ≤ 600 particles/container for ≥25µm | 1 | 0 | NT^{c} | NT^{c} |
| | ≤ 6000 particles/container for ≥10µm | 26 | 10 | | |
| Sub-visible particulates (MFI) | Report results | | | | |
| | #particles/container for ≥25 µm | 2 | 3 | 2 | 18 |
| | #particles/container for ≥10 µm | 25 | 68 | 22 | 360 |
| | #particles/container for ≥2 µm | 3730 | 6597 | 4331 | 14408 |
| Endotoxin | ≤ 15 EU/mL | <2.5 | <2.5 | NT^{c} | NT^{c} |
| CCIT | Pass (No blue coloration) | Pass | Pass | NT^{c} | NT^{c} |

| | | | | | |
|---|---|---|---|---|---|
| ^{a} The acceptance criteria listed here are for comparison purposes only. ^{b} Clear to opalescent, colorless to slightly brown yellow solution, essentially free of visible particles. ^{c} NT = Not Tested | | | | | |

| **Table 8: Stability Data for COR-001 Drug Product Lot CMC-N-0011 (7.5 mg/mL) at 25 ± 2°C** | | | | | |
|---|---|---|---|---|---|
| **Test** | **Acceptance Criteria ^{a}** | **Time points (Months)** | | | |
| | | **0** | **1** | **3** | **6** |
| Appearance | Clear to opalescent, colorless to slightly brown yellow solution essentially free of visible particles | b | b | b | b |
| Clarity | ≤ Reference IV | ≤ I | ≤ I | ≤ I | Water |
| Color | ≤ Reference BY4 | ≤ BY7 | ≤ BY7 | ≤ BY7 | ≤ BY7 |
| pH | 5.7 - 6.3 | 6.2 | 6.1 | 6.1 | 6.1 |
| Polysorbate 80 | Report result (w/v%) | 0.07 | 0.06 | 0.06 | 0.06 |
| Protein concentration | 7.5 ± 1.1 mg/mL | 7.5 | 7.6 | 7.6 | 7.7 |
| SE-UHPLC | Monomer peak: ≥ 95.0% | 98.3 | 98.1 | 97.0 | 96.4 |
| | HMW species: ≤ 5.0% | 0.8 | 0.9 | 1.2 | 1.0 |
| | LMW species: report result | 0.9 | 1.0 | 1.8 | 2.6 |
| Nonreduced CE-SDS | % IgG: ≥ 85.0% | 95.5 | 94.7 | 93.8 | 92.7 |
| | % HHL: report result | 2.6 | 2.7 | 3.1 | 3.1 |
| Reduced CE-SDS | % LC + % HC: ≥ 95.0% | 97.3 | 96.8 | 97.4 | 96.5 |
| icIEF | %Main: Report result | 66.1 | 58.4 | 50.9 | 43.5 |
| | %Acidic: Report result | 27.9 | 33.5 | 43.1 | 49.1 |
| | %Basic: Report result | 6.0 | 8.1 | 6.0 | 7.4 |
| Oxidation | Report % oxidation | 2.0 | 4.8 | 5.0 | 5.4 |
| IL-6 binding ELISA | 50% - 150% of Reference Standard | 94 | 95 | 102 | 81 |
| Cell based bioassay | 50% - 150% of Reference Standard | 101 | 118 | 94 | 104 |
| Sub-visible particulates (HIAC) | ≤ 600 particles/container for ≥25µm | 0 | 1 | NT^{c} | NT^{c} |
| | ≤ 6000 particles/container for ≥10µm | 9 | 8 | | |
| Sub-visible particulates (MFI) | Report results | | | | |
| | #particles/container for ≥25 µm | 2 | 6 | 1 | 2 |
| | #particles/container for ≥10 µm | 32 | 161 | 25 | 21 |
| | #particles/container for ≥2 µm | 2600 | 9873 | 4257 | 2079 |
| Endotoxin | ≤ 15 EU/mL | <2.5 | <2.5 | NT^{c} | NT^{c} |
| CCIT | Pass (No blue coloration) | Pass | Pass | NT^{c} | NT^{c} |

| | | | | | |
|---|---|---|---|---|---|
| ^{a} The acceptance criteria listed here are for comparison purposes only. ^{b} Clear to opalescent, colorless to slightly brown yellow solution, essentially free of visible particles. ^{c} NT = Not Tested | | | | | |

| **Table 9: Stability Data for COR-001 Drug Product Lot STC-261-P221-S24 (30 mg/mL) at 40 ± 2°C** | | | | |
|---|---|---|---|---|
| **Test** | **Acceptance Criteria ^{a}** | **Time points (Months)** | | |
| | | **0** | **1** | **3** |
| Appearance | Report result (visible particles) | 0 | 0 | 0 |
| Clarity | Report result | < IV | < IV | > IV |
| Color | Report result | < BY7 | < BY6 | < BY5 |
| pH | 5.5 - 6.5 | 6.2 | 6.2 | 5.9 |
| Protein concentration | Report result | 29.6 | 30.7 | 27.9 |
| SEC | Monomer peak: ≥ 95.0% | 98.8 | 96.4 | 93.9 |
| | HMW species: ≤ 5.0% | 1.0 | 2.5 | 3.6 |
| | LMW species: report result | 0.2 | 1.1 | 2.5 |
| Nonreduced CE-SDS | %Main peak: Report result | 93.9 | 91.4 | 86.9 |
| | %HHL peak: Report result | 4.2 | 4.0 | 5.6 |
| Reduced CE-SDS | % LC + % HC: ≥ 95.0% | 97.8 | 96.9 | 93.9 |
| icIEF | %Main: Report result | 60.2 | 36.4 | 20.7 |
| | %Acidic: Report result | 33.1 | 56.0 | 73.0 |
| | %Basic: Report result | 6.8 | 7.6 | 6.4 |
| Oxidation | Report % oxidation | 1.7 | 4.5 | 4.5 |
| IL-6 binding ELISA | 50% - 150% of Reference Standard | 97 | 85 | 78 |
| Cell based bioassay | 50% - 150% of Reference Standard | 106 | 123 | 82 |

| | | | | |
|---|---|---|---|---|
| ^{a} The acceptance criteria listed here are for comparison purposes only. | | | | |

| **Table 10: Stability Data for COR-001 Drug Product Lot VVRG56 (30 mg/mL) at 40 ± 2°C** | | | | |
|---|---|---|---|---|
| **Test** | **Acceptance Criteria ^{a}** | **Time points (Months)** | | |
| | | **0** | **1** | **3** |
| Appearance | Report result | b | b | b |
| Clarity | Report result | ≤ II | ≤ III | ≤ III |
| Color | Report result | ≤ BY7 | ≤ BY7 | ≤ BY6 |
| pH | Report result | 6.2 | 6.2 | 6.2 |
| Protein concentration | 30.0 ± 5.0 mg/mL | 30.8 | 31.0 | 31.0 |
| SE-UHPLC | Monomer peak: ≥ 95.0% | 98.8 | 95.7 | 92.5 |
| | HMW species: ≤ 5.0% | 0.7 | 1.6 | 2.5 |
| | LMW species: report result | 0.5 | 2.8 | 5.0 |
| Nonreduced CE-SDS | % IgG: ≥ 85.0% | 95.5 | 92.1 | 87.0 |
| | % HHL: report result | 2.7 | 2.9 | 4.1 |
| Reduced CE-SDS | % LC + % HC: ≥ 95.0% | 98.4 | 97.1 | 94.9 |
| icIEF | %Main: Report result | 73.5 | 46.8 | 29.0 |
| | %Acidic: Report result | 18.0 | 42.1 | 64.8 |
| | %Basic: Report result | 8.5 | 11.1 | 6.2 |
| Oxidation | Report % oxidation | 2.7 | 4.7 | 4.5 |
| IL-6 binding ELISA | 50% - 150% of Reference Standard | 103 | 95 | 94 |
| Cell based bioassay | 50% - 150% of Reference Standard | 97 | 86 | 79 |

| | | | | |
|---|---|---|---|---|
| ^{a} The acceptance criteria listed here are for comparison purposes only. ^{b} Clear to opalescent, colorless to slightly brown yellow solution, essentially free of visible particles. | | | | |

| **Table 11: Stability Data for COR-001 Drug Product Lot CMC-M-0060 (30 mg/mL) at 40 ± 2°C** | | | | |
|---|---|---|---|---|
| **Test** | **Acceptance Criteria ^{a}** | **Time points (Months)** | | |
| | | **0** | **1** | **3** |
| Appearance | Clear to opalescent, colorless to slightly brown yellow solution essentially free of visible particles | b | b | b |
| Clarity | ≤ Reference IV | ≤ III | ≤ II | ≤ III |
| Color | ≤ Reference BY4 | ≤ BY7 | ≤ BY6 | ≤ BY5 |
| pH | 5.7 - 6.3 | 6.1 | 6.1 | 6.1 |
| Polysorbate 80 | Report result (w/v%) | 0.07 | 0.05 | 0.05 |
| Protein concentration | 30.0 ± 4.5 mg/mL | 30.8 | 30.7 | 30.6 |
| SE-UHPLC | Monomer peak: ≥ 95.0% | 98.3 | 95.0 | 91.3 |
| | HMW species: ≤ 5.0% | 0.9 | 2.1 | 2.6 |
| | LMW species: report result | 0.7 | 2.8 | 6.1 |
| Nonreduced CE- SDS | % IgG: ≥ 85.0% | 96.0 | 91.6 | 85.0 |
| | % HHL: report result | 2.4 | 3.2 | 4.7 |
| Reduced CE-SDS | % LC + % HC: ≥ 95.0% | 97.8 | 95.9 | 93.6 |
| icIEF | %Main: Report result | 65.7 | 48.6 | 26.7 |
| | %Acidic: Report result | 28.0 | 44.7 | 69.3 |
| | %Basic: Report result | 6.3 | 6.6 | 5.3 |
| Oxidation | Report % oxidation | 2.4 | 4.4 | 4.0 |
| IL-6 binding ELISA | 50% - 150% of Reference Standard | 91 | 89 | 86 |
| Cell based bioassay | 50% - 150% of Reference Standard | 87 | 94 | 106 |

| | | | | |
|---|---|---|---|---|
| ^{a} The acceptance criteria listed here are for comparison purposes only. ^{b} Clear to opalescent, colorless to slightly brown yellow solution, essentially free of visible particles. | | | | |

| **Table 12: Stability Data for COR-001 Drug Product Lot CMC-M-0061 (15 mg/mL) at 40 ± 2°C** | | | | |
|---|---|---|---|---|
| **Test** | **Acceptance Criteria ^{a}** | **Time points (Months)** | | |
| | | **0** | **1** | **3** |
| Appearance | Clear to opalescent, colorless to slightly brown yellow solution essentially free of visible particles | b | b | b |
| Clarity | ≤ Reference IV | < III | < II | < I |
| Color | ≤ Reference BY4 | < BY7 | < BY6 | < BY6 |
| pH | 5.7 - 6.3 | 6.2 | 6.1 | 6.1 |
| Polysorbate 80 | Report result (w/v%) | 0.07 | 0.04 | 0.04 |
| Protein concentration | 15.0 ± 2.3 mg/mL | 15.0 | 15.4 | 15.4 |
| SE-UHPLC | .Monomer peak: ≥ 95.0% | 98.3 | 93.7 | 90.0 |
| | HMW species: ≤ 5.0% | 0.9 | 2.6 | 3.1 |
| | LMW species: report result | 0.8 | 3.7 | 6.9 |
| Nonreduced CE- SDS | % IgG: ≥ 85.0% | 95.9 | 91.0 | 83.7 |
| | % HHL: report result | 2.3 | 3.4 | 4.9 |
| Reduced CE-SDS | % LC + % HC: ≥ 95.0% | 98.0 | 95.5 | 93.4 |
| icIEF | %Main: Report result | 66.2 | 43.4 | 21.6 |
| | %Acidic: Report result | 27.8 | 50.7 | 73.5 |
| | %Basic: Report result | 6.0 | 5.9 | 4.9 |
| Oxidation | Report % oxidation | 2.5 | 4.4 | 4.4 |
| IL-6 binding ELISA | 50% - 150% of Reference Standard | 96 | 85 | 97 |
| Cell based bioassay | 50% - 150% of Reference Standard | 105 | 85 | 89 |

| | | | | |
|---|---|---|---|---|
| ^{a} The acceptance criteria listed here are for comparison purposes only. ^{b} Clear to opalescent, colorless to slightly brown yellow solution, essentially free of visible particles. | | | | |

| **Table 13: Stability Data for COR-001 Drug Product Lot CMC-N-0011 (7.5 mg/mL) at 40 ± 2°C** | | | | |
|---|---|---|---|---|
| **Test** | **Acceptance Criteria ^{a}** | **Time points (Months)** | | |
| | | **0** | **1** | **3** |
| Appearance | Clear to opalescent, colorless to slightly brown yellow solution essentially free of visible particles | b | b | b |
| Clarity | ≤ Reference IV | ≤ I | ≤ I | ≤ I |
| Color | ≤ Reference BY4 | ≤ BY7 | ≤ BY7 | ≤ BY6 |
| pH | 5.7 - 6.3 | 6.2 | 6.1 | 6.1 |
| Polysorbate 80 | Report result (w/v%) | 0.07 | 0.05 | 0.05 |
| Protein concentration | 7.5 ± 1.1 mg/mL | 7.5 | 7.7 | 7.7 |
| SE-UHPLC | Monomer peak: ≥ 95.0% | 98.3 | 95.5 | 91.6 |
| | HMW species: ≤ 5.0% | 0.8 | 2.0 | 2.8 |
| | LMW species: report result | 0.9 | 2.5 | 5.6 |
| Nonreduced CE-SDS | % IgG: ≥ 85.0% | 95.5 | 91.8 | 86.3 |
| | % HHL: report result | 2.6 | 3.4 | 4.8 |
| Reduced CE-SDS | % LC + % HC: ≥ 95.0% | 97.3 | 95.3 | 94.7 |
| icIEF | %Main: Report result | 66.1 | 44.3 | 20.2 |
| | %Acidic: Report result | 27.9 | 47.4 | 73.4 |
| | %Basic: Report result | 6.0 | 8.3 | 6.4 |
| Oxidation | Report % oxidation | 2.0 | 4.9 | 4.8 |
| IL-6 binding ELISA | 50% - 150% of Reference Standard | 94 | 92 | 99 |
| Cell based bioassay | 50% - 150% of Reference Standard | 101 | 91 | 89 |

| | | | | |
|---|---|---|---|---|
| ^{a} The acceptance criteria listed here are for comparison purposes only. ^{b} Clear to opalescent, colorless to slightly brown yellow solution, essentially free of visible particles. | | | | |

There were no apparent changes in appearance up to 12 months under the accelerated condition (Tables 4 to 8) and up to 3 months under the stress condition (Tables 9 to 13).

There were no apparent changes in clarity up to 12 months under the accelerated condition (Tables 4 to 8) and up to 3 months under the stress condition (Tables 9 to 13). All results were below Reference IV (turbidity standard per EU method), except for the development lot STC-261-P221, which was prepared in a development lab. The clarity of this lot was above reference IV at 6 months under the accelerated condition (Table 4) and 3 months under the stress condition (Table 9).

There was no apparent trend in color up to 12 months under the accelerated condition (Tables 4 to 8). Under the stress condition, there was a slight increase trend in color over 3 months storage (Tables 9 to 13), but all results were below Reference BY4.

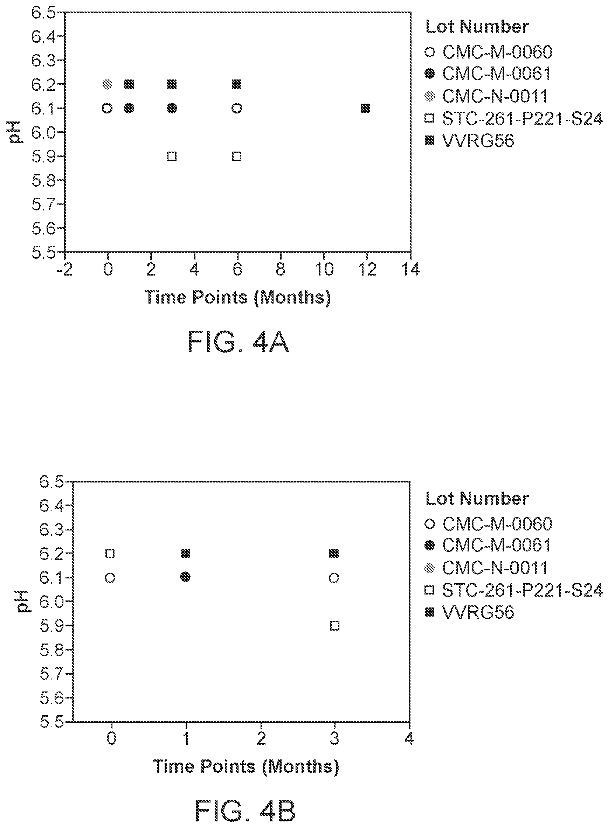

There were no apparent changes in pH up to 12 months under the accelerated condition and 3 months under the stress condition (FIGs. 4A and 4B), except for the development lot STC-261-P221-S24 that had a small drop at 3 months, which is likely due to assay variability.

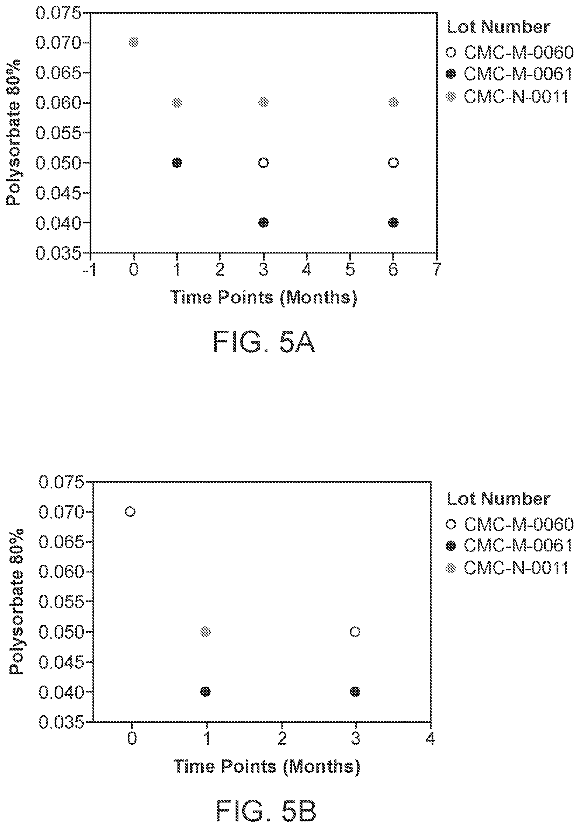

Polysorbate 80 level decreased over time in all three clinical lots tested under accelerated and stress conditions (FIGs. 5A and 5B). Under the accelerated condition, the decrease of polysorbate 80 level reached to a plateau of about 0.04% at 3 months for all three lots without apparent correlation between protein concentration and plateau level (FIG. 5A). Similarly, under the stress condition, the polysorbate 80 level reached to a plateau of about 0.04% at 1 month for all three lots (FIG. 5B). Noticeably, the plateau levels between accelerated and stress conditions are similar, which suggests the decrease of polysorbate 80 is independent of temperature.

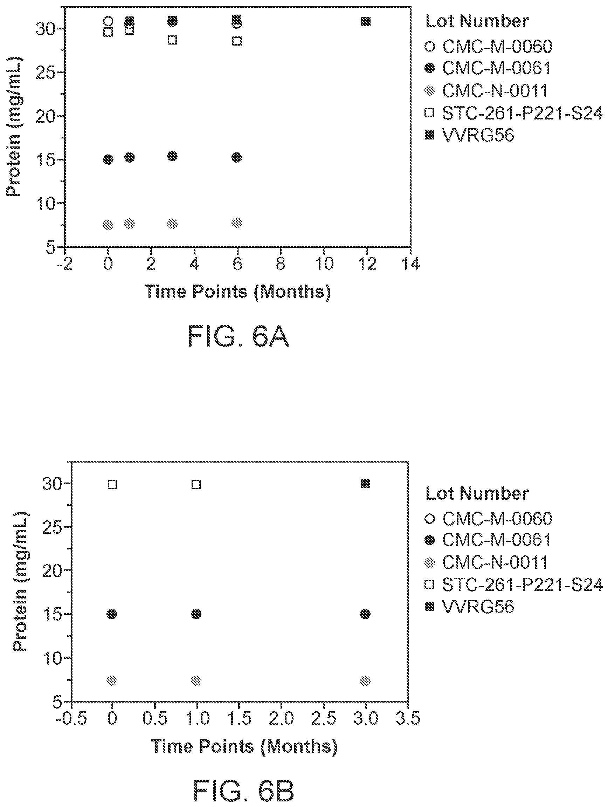

There were no apparent changes in protein concentrations up to 12 months under the accelerated condition and 3 months under the stress condition except the development lot STC-261-P221-S24 (FIGs. 6A and 6B), which was tested with an A280 method by sample dilution while other lots were tested by Solo VPE without dilution. Thus, the apparent change for the development lot was likely due to assay dilution variation.

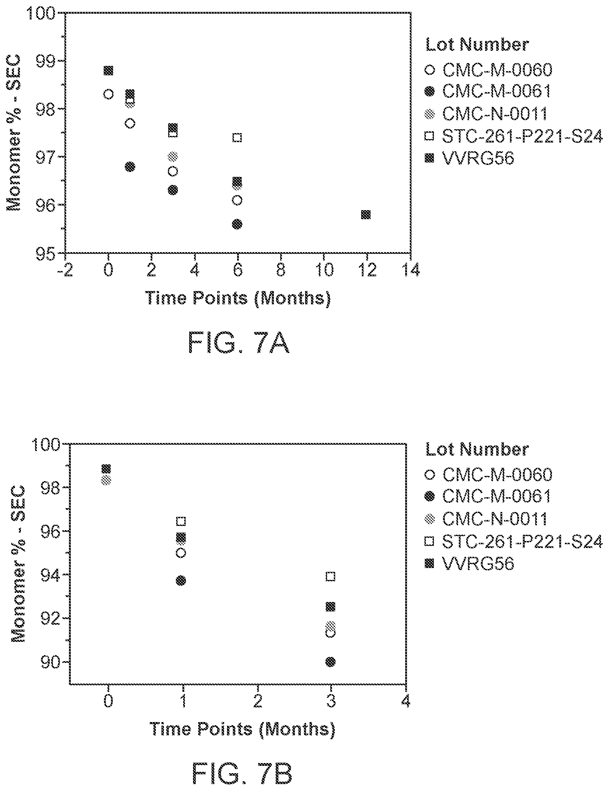

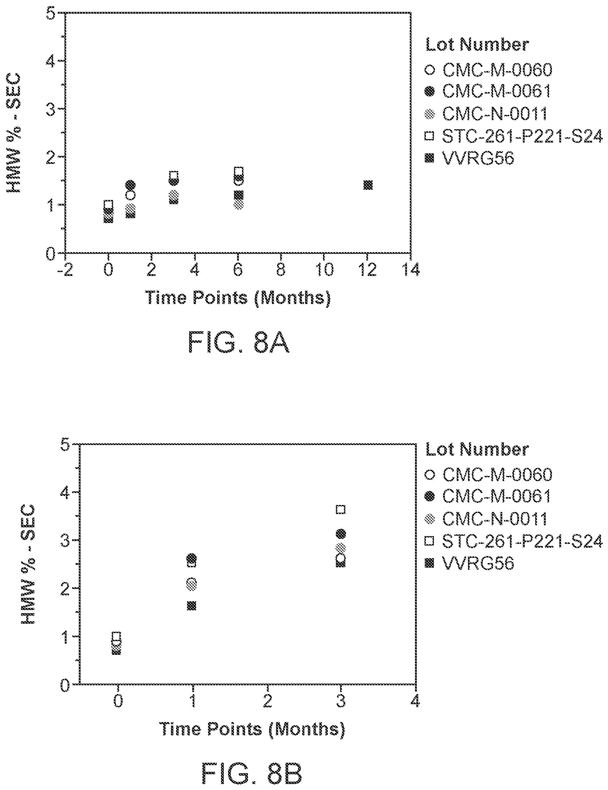

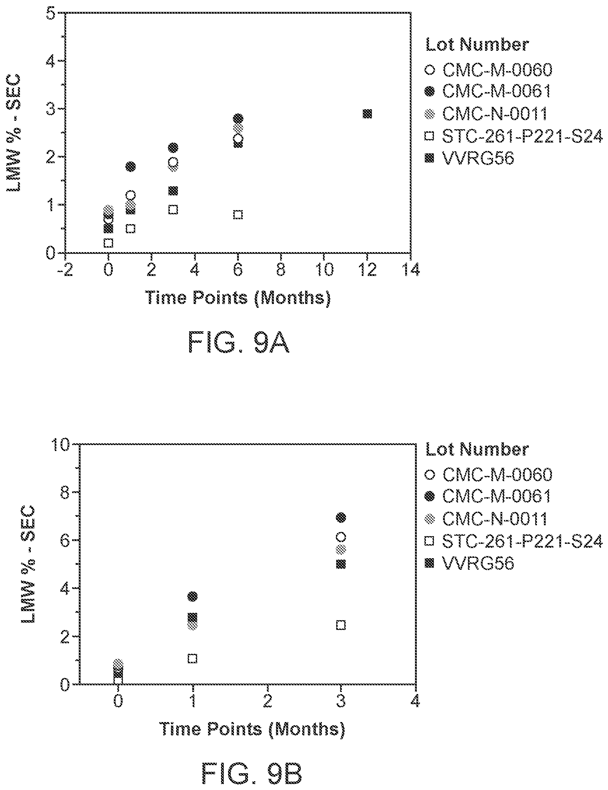

The levels of monomer (non-aggregated bivalent full length IgG antibody), high molecular weight (HMW) species, and low molecular weight (LMW) species were measured by size exclusion chromatography - ultra high performance liquid chromatography (SEC-UHPLC). The monomer% decreased over time, mainly due to increase of HMW% and LMW%, with more changes under the stress condition. The kinetics for the monomer% appeared to be linear with some variations from lot to lot without apparent trend correlating with the protein concentrations (FIGs. 7A and 7B; FIGs. 8A and 8B; FIGs. 9A and 9B).

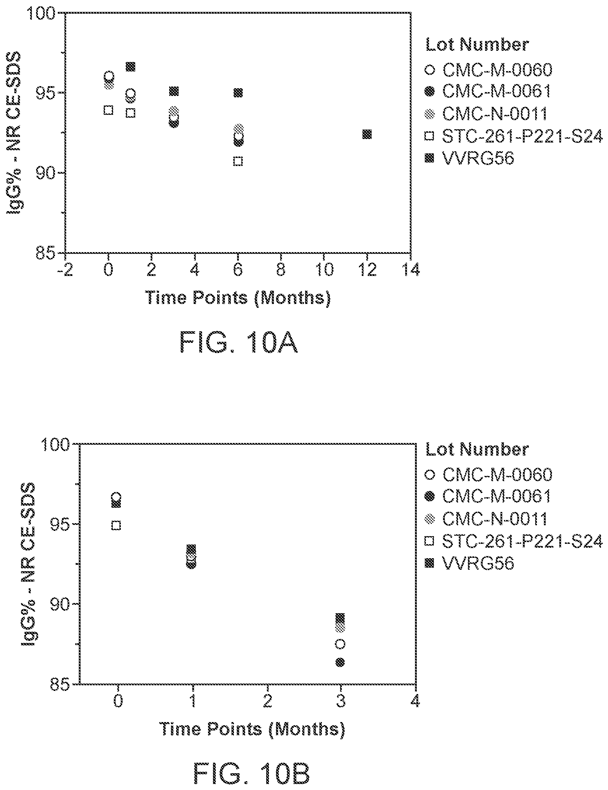

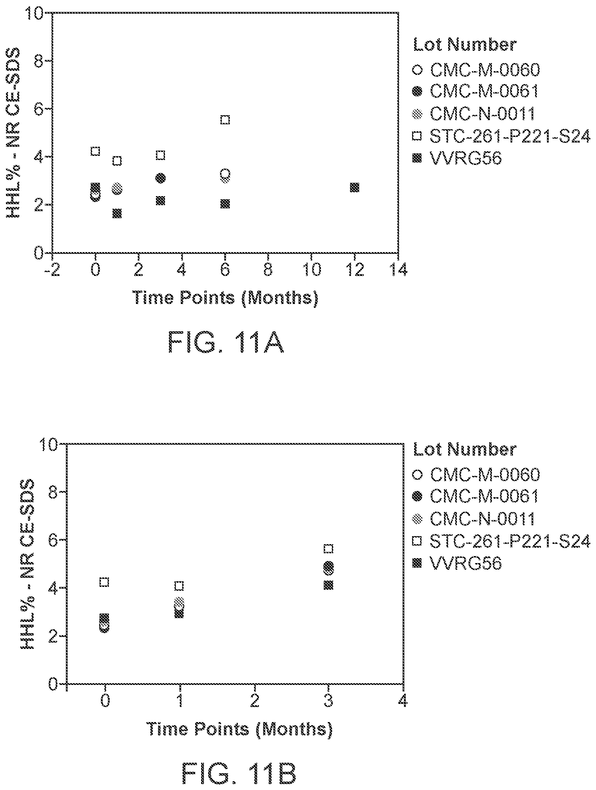

The levels of immunoglobulin gamma (IgG) and the combination of two heavy chains and one light chain (HHL) were measured by non-reduced CE-SDS. Under the accelerated condition, the IgG% decreased over time without apparent trend in HHL% (FIG. 10A and FIG. 11A), suggesting that the decrease was due to the increase of other fragments. Under the stress condition, IgG% decreased faster than the accelerated condition, and HHL% also increased over time (FIG. 10B and FIG. 11B). Both IgG% and HHL% changes under the stress condition appeared to be linear with some variation from lot to lot. But no apparent trend correlating with the protein concentrations was observed.

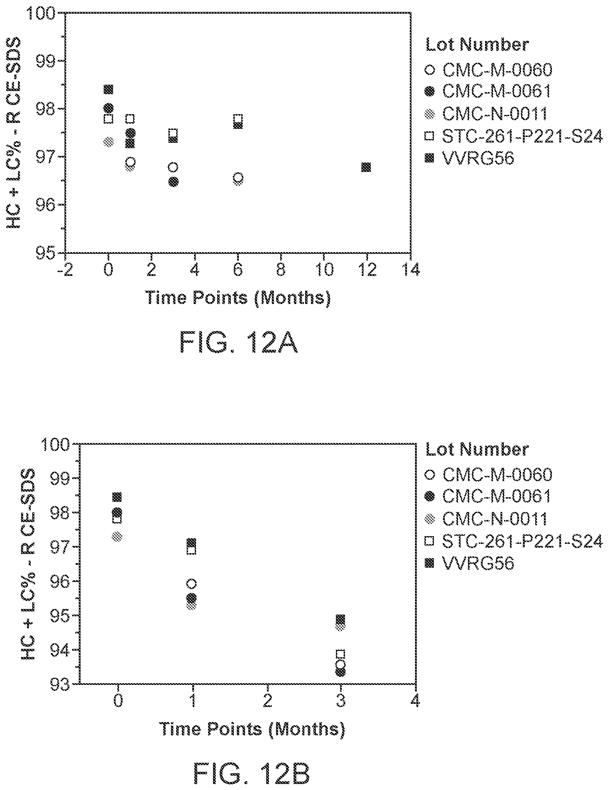

The level of heavy chain + light chain (HC+LC) was measured by reduced CE-SDS. Under the accelerated condition, the decrease of HC+LC% appeared to reach to a plateau after 3 months for all lots (FIG. 12A). Under the stress condition, the HC+LC% decrease appeared to be linear with some variation from lot to lot. But no apparent trend correlating with the protein concentrations was observed (FIG. 12B).

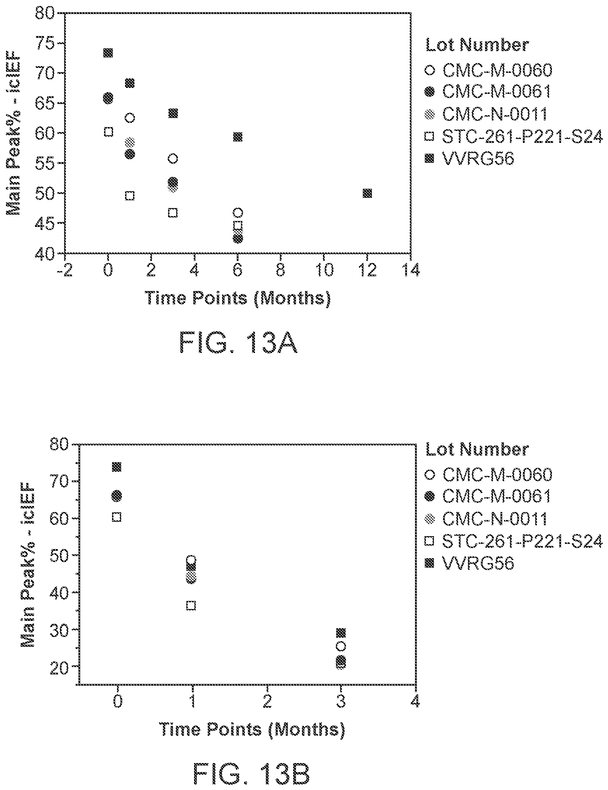

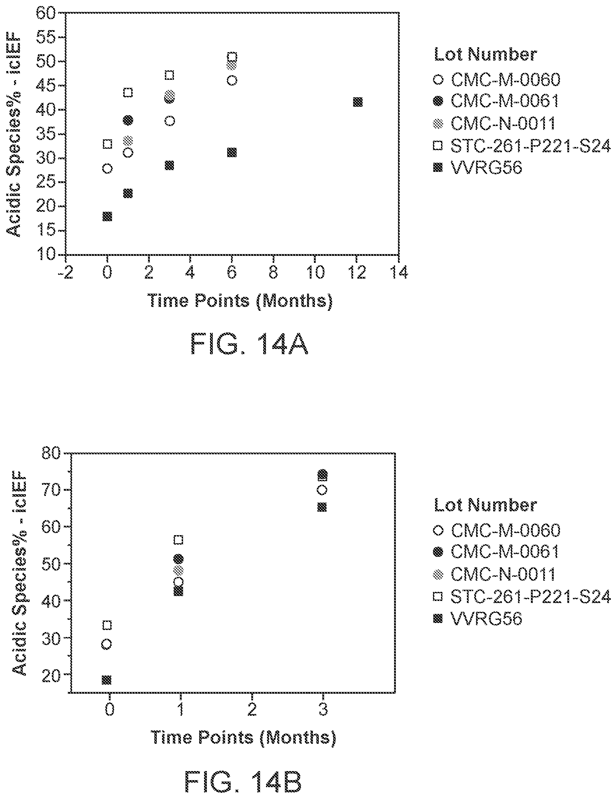

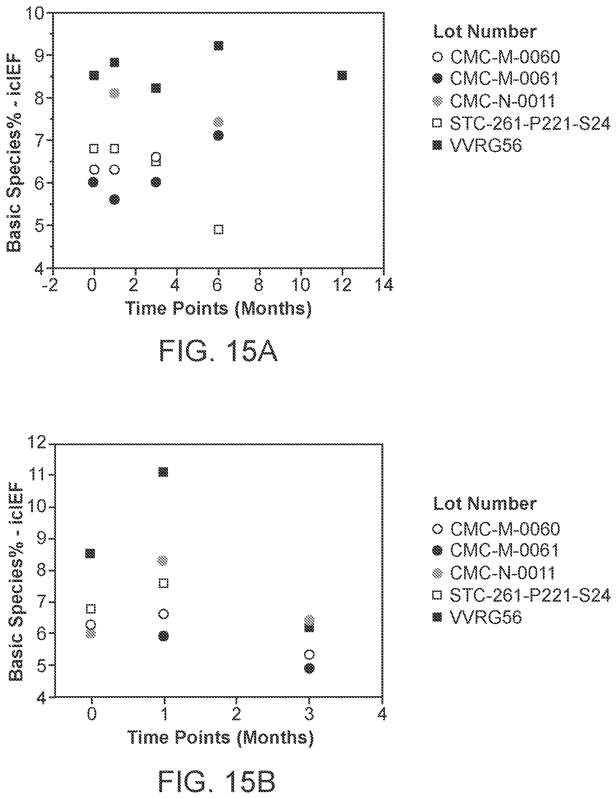

The levels of main species, acidic species, and basic species were measured by imaged capillary isoelectric focusing (icIEF). Under the accelerated condition, the main peak% decreased over time (FIG. 13A), mainly due to increase of the acidic species (FIG. 14A), as no apparent changes in basic species (FIG. 15A) was observed. Similarly, under the stress condition, the main peak% decreased (FIG. 13B) and acidic species% increased (FIG. 14B) with no apparent changes in basic species (FIG. 15B). The changes of the main species% and acidic species% appeared to be linear with some variation from lot to lot, independent of the protein concentrations (FIGs. 13A and 13B; FIGs. 14A and 14B; FIGs. 15A and 15B).

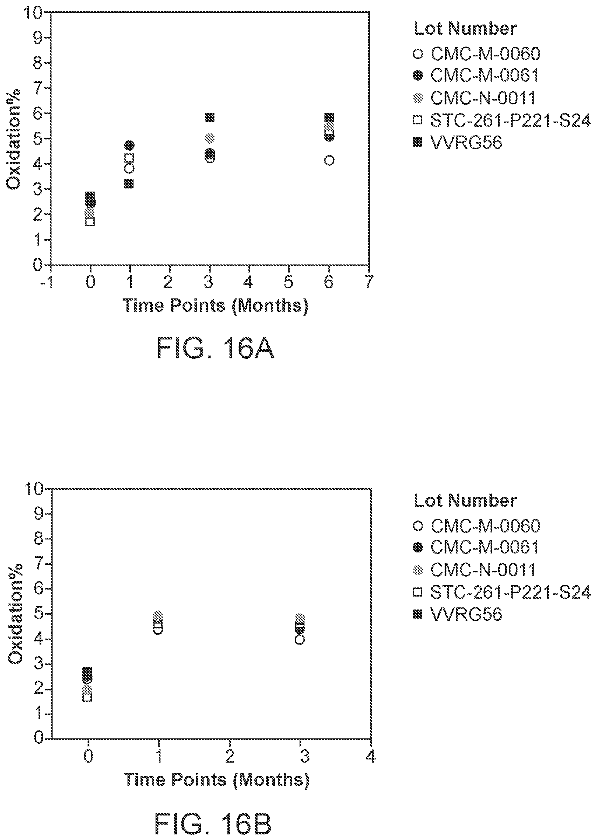

The level of oxidation was measured by reversed phase-high performance liquid chromatography (RP-HPLC). Under the accelerated condition, the oxidation% appeared to reach to a plateau at 3 months for all the lots (FIG. 16A). Similarly, under the stress condition, the oxidation% level appeared to reach to a plateau at 1 month for all the lots (FIG. 16B). The plateau level for the accelerated and stress conditions appeared to be 5-6%. Overall, this indicates that the maximum oxidation is 5-6%.

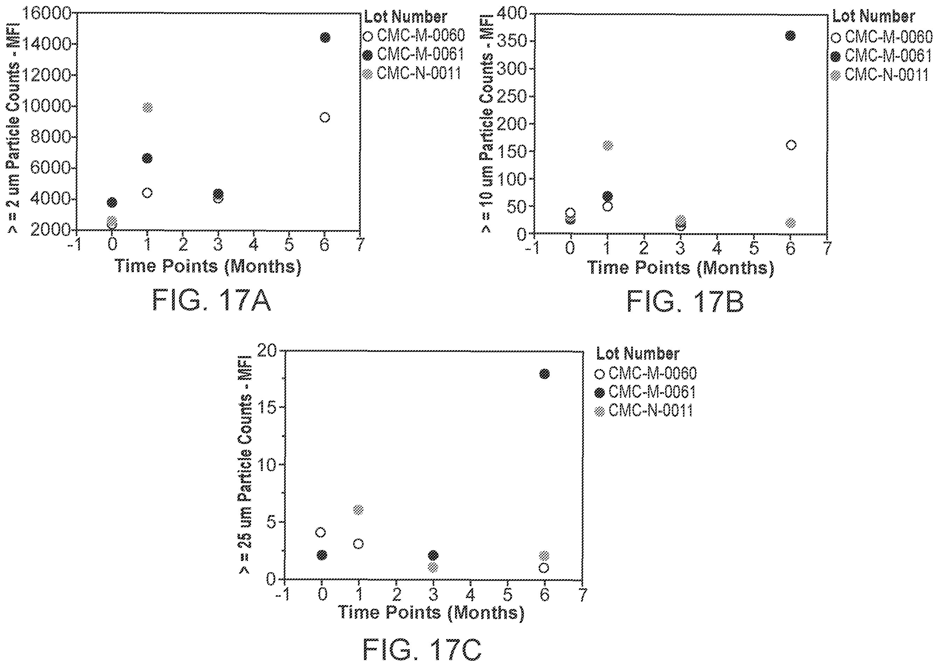

There were no apparent changes in the number of sub-visible particles tested by high accuracy fluid particle counting (HIAC) for all three clinical lots after 1 month under the accelerated condition (Tables 6 to 8). There were changes in the number of sub-visible particles tested by micro-flow imaging (MFI) for the three clinical lots over 6 months under the accelerated condition (FIGs. 17A, 17B, and 17C). However, these changes did not correlate with the protein concentrations. As a similar sub-visible particle level was also observed in the placebo lot (CMC-M-0062) at 3-6 months, some of these sub-visible particles could be due to nature of the formulation buffer components.

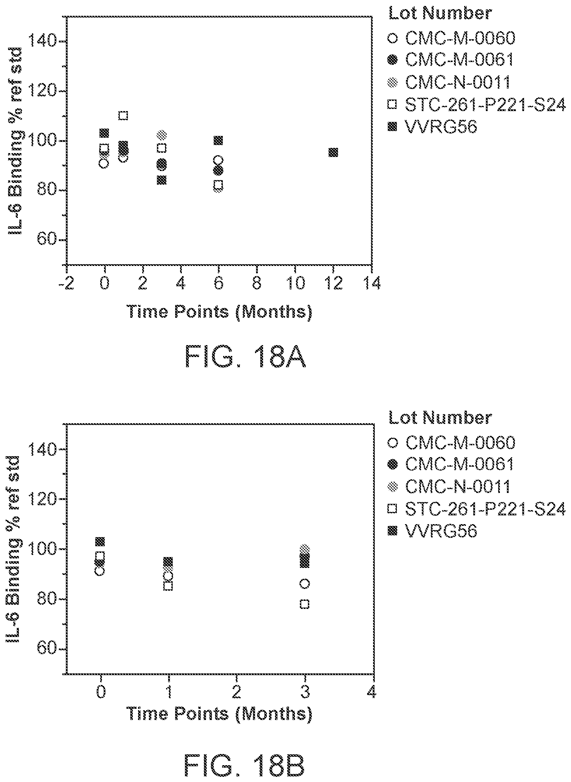

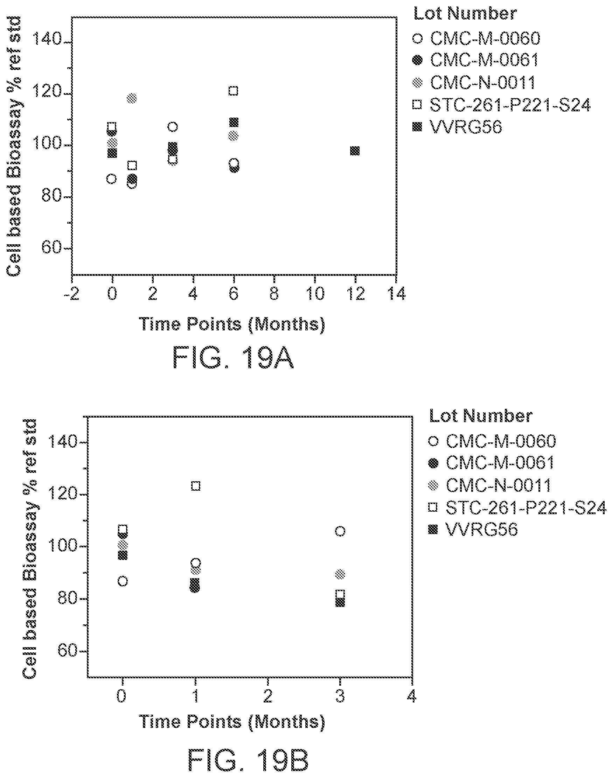

There were no apparent changes in potency tested by both IL-6 binding ELISA and HEK Blue cell-based bioassay up to 12 months under the accelerated condition and 3 months under the stress condition (FIGs. 18A and 18B; FIGs 19A and 19B).

The microbiological quality in the three clinical drug product lots was monitored by level of endotoxins, as well as container closure integrity testing (CCIT) in lieu of sterility testing for the clinical lots. After 1 month under the accelerated condition, the results all met the acceptance criteria for long term storage (Tables 6 to 8).

### B. Stability Results under the Long Term Storage Condition

The stability of each lot of COR-001 prepared in the formulation stored under the long term storage condition are summarized in the Tables 14 to 18 below, showing the results for up to 12 months. When available, the acceptance criteria of assays are listed in each table.

| **Table 14: Stability Data for COR-001 Drug Product Lot STC-261-P221-S24 (30 mg/mL) at 5 ± 3°C** | | | | | | |
|---|---|---|---|---|---|---|
| **Test** | **Acceptance Criteria** | **Time points (Months)** | | | | |
| | | **0** | **1** | **3** | **6** | **12** |
| Appearance | Report result (visible particles) | 0 | 0 | 0 | 0 | 0 |
| Clarity | Report result | < IV | < IV | < IV | < IV | < IV |
| Color | Report result | < BY7 | < BY7 | < BY7 | < BY7 | < BY7 |
| pH | 5.5 - 6.5 | 6.2 | 5.9 | 5.9 | 5.9 | 6.0 |
| Protein concentration | Report result | 29.6 | 29.4 | 28.1 | 28.1 | 29.8 |
| SEC | Monomer peak: ≥ 95.0% | 98.8 | 98.9 | 98.5 | 98.4 | 98.2 |
| | HMW species: ≤ 5.0% | 1.0 | 1.0 | 1.2 | 1.3 | 1.4 |
| | LMW species: report result | 0.2 | 0.1 | 0.4 | 0.3 | 0.4 |
| Nonreduced CE-SDS | %Main peak: Report result | 93.9 | 93.8 | 94.3 | 93.3 | 92.6 |
| | %HHL peak: Report result | 4.2 | 4.0 | 3.8 | 4.5 | 4.4 |
| Reduced CE-SDS | % LC + % HC: ≥ 95.0% | 97.8 | 98.2 | 97.7 | 98.6 | 96.8 |
| icIEF | %Main: Report result | 60.2 | 57.8 | 55.6 | 54.0 | 53.2 |
| | %Acidic: Report result | 33.1 | 35.5 | 37.9 | 40.6 | 41.2 |
| | %Basic: Report result | 6.8 | 6.6 | 6.4 | 5.4 | 5.7 |
| Oxidation | Report % oxidation | 1.7 | 2.3 | 2.9 | 4.8 | 5.3 |
| IL-6 binding ELISA | 50% - 150% of Reference Standard | 97 | 98 | 93 | 83 | 95 |
| Cell based bioassay | 50% - 150% of Reference Standard | 106 | 108 | 115 | 99 | 88 |
| Sub-visible particulates (HIAC) | Report results | | | | | |
| | #particles/mL for ≥25µm | 0 | NT^{a} | NT^{a} | NT^{a} | 0 |
| | #particles/mL for ≥10µm | 60 | | | | 15 |
| | #particles/mL for ≥2µm | 1103 | | | | 290 |

| | | | | | | |
|---|---|---|---|---|---|---|
| ^{a} NT: Not Tested | | | | | | |

| **Table 15: Stability Data for COR-001 Drug Product Lot VVRG56 (30 mg/mL) at 5 ± 3°C** | | | | | | | |
|---|---|---|---|---|---|---|---|
| **Test** | **Acceptance Criteria** | **Time points (Months)** | | | | | |
| | | **0** | **1** | **3** | **6** | **9** | **12** |
| Appearance | Report result | a | a | a | a | a | a |
| Clarity | Report result | ≤ II | ≤ III | ≤ III | ≤ II | ≤ II | ≤ III |
| Color | Report result | ≤ BY7 | ≤ BY7 | ≤ BY7 | ≤ BY7 | ≤ BY7 | ≤ BY7 |
| pH | Report result | 6.2 | 6.2 | 6.2 | 6.2 | 6.1 | 6.1 |
| Protein concentration | 30.0 ± 5 mg/mL | 30.8 | 30.8 | 30.7 | 31.0 | 30.5 | 30.5 |
| SE-UHPLC | Monomer peak: ≥ 95.0% | 98.8 | 98.8 | 98.7 | 98.6 | 98.3 | 98.4 |
| | HMW species: ≤ 5.0% | 0.7 | 0.7 | 0.7 | 0.7 | 0.7 | 0.8 |
| | LMW species: report result | 0.5 | 0.5 | 0.6 | 0.7 | 1.0 | 0.8 |
| Nonreduced CE-SDS | % IgG: ≥ 85.0% | 95.5 | 97.3 | 97.0 | 97.5 | 97.0 | 96.8 |
| | % HHL: report result | 2.7 | 1.6 | 1.8 | 1.3 | 1.5 | 1.8 |
| Reduced CE-SDS | % LC + % HC: ≥ 95.0% | 98.4 | 97.6 | 97.8 | 97.9 | 97.8 | 97.4 |
| icIEF | %Main: Report result | 73.5 | 72.7 | 73.1 | 72.8 | 70.6 | 68.9 |
| | %Acidic: Report result | 18.0 | 18.7 | 18.6 | 18.8 | 21.0 | 22.9 |
| | %Basic: Report result | 8.5 | 8.6 | 8.4 | 8.5 | 8.4 | 8.1 |
| Oxidation | Report % oxidation | 2.7 | 2.9 | 2.7 | 4.7 | 4.9 | NA° |
| IL-6 binding ELISA | 50% - 150% of Reference Standard | 103 | 94 | 85 | 92 | 98 | 98 |
| Cell based bioassay | 50% - 150% of Reference Standard | 97 | 91 | 106 | 101 | 87 | 84 |
| Sub-visible particulates (HIAC) | ≤600 particles/container for ≥25µm | 0 | NT ^{b} | NT ^{b} | 0 | NT ^{b} | 0 |
| | ≤6000 particles/container for ≥10µm | 5 | | | 9 | | 10 |
| Sub-visible particulates (MFI) | Report results | | | | | | |
| | #particles/container for ≥25 µm | 14 | NT ^{b} | NT^{b} | 1 | NT ^{b} | 6 |
| | #particles/container for ≥ 10 µm | 71 | | | 12 | | 64 |
| | #particles/container for ≥2 µm | 2378 | | | 3554 | | 4336 |
| CCIT | Pass (No blue coloration) | Pass | NT^{c} | NT^{c} | NT^{c} | NT^{c} | Pass |

| | | | | | | | |
|---|---|---|---|---|---|---|---|
| ^{a} Clear to opalescent, colorless to slightly brown yellow solution, essentially free of visible particles; ^{b} NT: Not Tested; ^{c}NA: Not Available. | | | | | | | |

| **Table 16: Stability Data for COR-001 Drug Product Lot CMC-M-0060 (30 mg/mL) at 5 ± 3°C** | | | | | | |
|---|---|---|---|---|---|---|
| **Test** | **Acceptance Criteria** | **Time points (Months)** | | | | |
| | | **0** | **1** | **3** | **6** | **9** |
| Appearance | Clear to opalescent, colorless to slightly brown yellow solution essentially free of visible particles | confor m | confor m | confor m | confor m | confor m |
| Clarity | ≤ Reference IV | ≤ III | ≤ II | ≤ III | ≤ II | ≤ II |
| Color | ≤ Reference BY4 | ≤ BY7 | ≤ BY7 | ≤ BY7 | ≤ BY7 | ≤ BY7 |
| pH | 5.7 - 6.3 | 6.1 | 6.1 | 6.2 | 6.1 | 6.1 |
| Polysorbate80 | Report result (w/v%) | 0.07 | 0.07 | 0.07 | 0.06 | 0.06 |
| Protein concentration | 30.0 ± 5 mg/mL | 30.8 | 30.4 | 30.3 | 30.5 | 30.1 |
| SE-UHPLC | Monomer peak: ≥ 95.0% | 98.3 | 98.4 | 98.1 | 98.1 | 97.7 |
| | HMW species: ≤ 5.0% | 0.9 | 0.9 | 0.9 | 1.0 | 1.2 |
| | LMW species: report result | 0.7 | 0.7 | 1.0 | 0.9 | 1.1 |
| Nonreduced CE-SDS | % IgG: ≥ 85.0% | 96.0 | 95.7 | 95.1 | 95.0 | 94.9 |
| | % HHL: report result | 2.4 | 2.4 | 2.7 | 2.9 | 2.9 |
| Reduced CE-SDS | % LC + % HC: ≥ 95.0% | 97.8 | 97.6 | 97.3 | 97.4 | 97.6 |
| icIEF | %Main: Report result | 65.7 | 67.4 | 65.7 | 61.2 | 59.9 |
| | %Acidic: Report result | 28.0 | 27.0 | 28.1 | 31.6 | 33.9 |
| | %Basic: Report result | 6.3 | 5.6 | 6.2 | 7.2 | 6.2 |
| Oxidation | Report % oxidation | 2.4 | 2.0 | 2.5 | 3.7 | 4.0 |
| IL-6 binding ELISA | 50% - 150% of Reference Standard | 91 | 94 | 92 | 95 | 89 |
| Cell based bioassay | 50% - 150% of Reference Standard | 87 | 92 | 92 | 85 | 98 |
| Sub-visible particulates (HIAC) | ≤ 600 particles/container for ≥25µm | 0 | NT^{a} | NT^{a} | 1 | NT^{a} |
| | ≤ 6000 particles/container for ≥10µm | 19 | | | 33 | |
| Sub-visible particulates (MFI) | Report results | | | | | |
| | #particles/container for ≥25 µm | 4 | NT^{a} | NT^{a} | 12 | NT^{a} |
| | #particles/container for ≥10 µm | 39 | | | 222 | |
| | #particles/container for ≥2 µm | 2322 | | | 14373 | |
| Endotoxin | ≤ 15 EU/mL | <2.5 | NT^{a} | NT^{a} | NT^{a} | NT^{a} |
| CCIT | Pass (No blue coloration) | Pass | NT^{a} | NT^{a} | NT^{a} | NT^{a} |

| | | | | | | |
|---|---|---|---|---|---|---|
| ^{a} NT = Not Tested. | | | | | | |

| **Table 17: Stability Data for COR-001 Drug Product Lot CMC-M-0061 (15 mg/mL) at 5 ± 3°C** | | | | | | |
|---|---|---|---|---|---|---|
| **Test** | **Acceptance Criteria** | **Time points (Months)** | | | | |
| | | **0** | **1** | **3** | **6** | **9** |
| Appearance | Clear to opalescent, colorless to slightly brown yellow solution essentially free of visible particles | confor m | confor m | confor m | confor m | confor m |
| Clarity | ≤ Reference IV | ≤ III | ≤ II | ≤ I | ≤ I | ≤ II |
| Color | ≤ Reference BY4 | ≤ BY7 | ≤ BY7 | ≤ BY7 | ≤ BY7 | ≤ BY7 |
| pH | 5.7 - 6.3 | 6.2 | 6.2 | 6.2 | 6.1 | 6.1 |
| Polysorbate 80 | Report result (w/v%) | 0.07 | 0.07 | 0.07 | 0.07 | 0.06 |
| Protein concentration | 30.0 ± 5 mg/mL | 15.0 | 15.2 | 15.2 | 15.2 | 15.1 |
| SE-UHPLC | Monomer peak: ≥ 95.0% | 98.3 | 98.4 | 98.2 | 98.2 | 97.8 |
| | HMW species: ≤ 5.0% | 0.9 | 0.8 | 0.8 | 0.9 | 1.0 |
| | LMW species: report result | 0.8 | 0.7 | 1.0 | 1.0 | 1.3 |
| Nonreduced CE-SDS | % IgG: ≥ 85.0% | 95.9 | 95.6 | 94.8 | 94.9 | 94.6 |
| | % HHL: report result | 2.3 | 2.4 | 2.9 | 2.9 | 3.1 |
| Reduced CE-SDS | % LC + % HC: ≥ 95.0% | 98.0 | 98.1 | 97.1 | 97.2 | 97.4 |
| icIEF | %Main: Report result | 66.2 | 67.2 | 65.1 | 58.1 | 59.0 |
| | %Acidic: Report result | 27.8 | 27.0 | 28.8 | 34.9 | 34.6 |
| | %Basic: Report result | 6.0 | 5.8 | 6.1 | 6.9 | 6.4 |
| Oxidation | Report % oxidation | 2.5 | 2.4 | 3.1 | 5.1 | 4.9 |
| IL-6 binding ELISA | 50% - 150% of Reference Standard | 96 | 94 | 96 | 97 | 85 |
| Cell based bioassay | 50% - 150% of Reference Standard | 105 | 84 | 85 | 93 | 98 |
| Sub-visible particulates (HIAC) | ≤ 600 particles/container for ≥25µm | 1 | NT^{a} | NT^{a} | 1 | NT^{a} |
| | ≤ 6000 particles/container for ≥ 10µm | 26 | | | 10 | |
| Sub-visible particulates (MFI) | Report results | | | | | |
| | #particles/container for ≥25 µm | 2 | NT^{a} | NT^{a} | 9 | NT^{a} |
| | #particles/container for ≥10 µm | 25 | | | 206 | |
| | #particles/container for ≥2 µm | 3730 | | | 10654 | |
| Endotoxin | ≤ 15 EU/mL | <2.5 | NT^{a} | NT^{a} | NT^{a} | NT^{a} |
| CCIT | Pass (No blue coloration) | Pass | NT^{a} | NT^{a} | NT^{a} | NT^{a} |

| | | | | | | |
|---|---|---|---|---|---|---|
| ^{a}NT = Not Tested. | | | | | | |

| **Table 18: Stability Data for COR-001 Drug Product Lot CMC-N-0011 (7.5 mg/mL) at 5 ± 3°C** | | | | | |
|---|---|---|---|---|---|
| **Test** | **Acceptance Criteria** | **Time points (Months)** | | | |
| | | **0** | **1** | **3** | **6** |
| Appearance | Clear to opalescent, colorless to slightly brown yellow solution essentially free of visible particles | conform | conform | conform | conform |
| Clarity | ≤ Reference IV | ≤ I | ≤ I | ≤ I | ≤ I |
| Color | ≤ Reference BY4 | ≤ BY7 | ≤ BY7 | ≤ BY7 | ≤ BY7 |
| pH | 5.7 - 6.3 | 6.2 | 6.1 | 6.1 | 6.1 |
| Polysorbate 80 | Report result (w/v%) | 0.07 | 0.07 | 0.07 | 0.07 |
| Protein concentration | 7.5 ± 1.1 mg/mL | 7.5 | 7.6 | 7.5 | 7.7 |
| SE-UHPLC | Monomer peak: ≥ 95.0% | 98.3 | 98.5 | 98.3 | 98.2 |
| | HMW species: ≤ 5.0% | 0.8 | 0.9 | 0.9 | 0.8 |
| | LMW species: report result | 0.9 | 0.6 | 0.8 | 1.0 |
| Nonreduced CE- SDS | % IgG: ≥ 85.0% | 95.5 | 95.5 | 94.9 | 95.0 |
| | % HHL: report result | 2.6 | 2.5 | 2.9 | 2.7 |
| Reduced CE-SDS | % LC + % HC: ≥ 95.0% | 97.3 | 97.0 | 97.5 | 97.0 |
| icIEF | %Main: Report result | 66.1 | 64.7 | 62.1 | 58.9 |
| | %Acidic: Report result | 27.9 | 27.9 | 31.0 | 34.2 |
| | %Basic: Report result | 6.0 | 7.4 | 6.9 | 6.9 |
| Oxidation | Report % oxidation | 2.0 | 2.6 | 2.6 | 4.2 |
| IL-6 binding ELISA | 50% - 150% of Reference Standard | 94 | 86 | 83 | 92 |
| Cell based bioassay | 50% - 150% of Reference Standard | 101 | 98 | 93 | 99 |
| Sub-visible particulates (HIAC) | ≤ 600 particles/container for ≥25µm | 0 | NT^{a} | NT^{a} | 0 |
| | ≤ 6000 particles/container for ≥ 10µm | 9 | | | 8 |
| Sub-visible particulates (MFI) | Report results | | NT^{a} | NT^{a} | |
| | #particles/container for ≥25 µm | 2 | | | 1 |
| | #particles/container for ≥10 µm | 32 | | | 11 |
| | #particles/container for ≥2 µm | 2600 | | | 2287 |
| Endotoxin | ≤ 15 EU/mL | <2.5 | NT^{a} | NT^{a} | NT^{a} |
| CCIT | Pass (No blue coloration) | Pass | NT^{a} | NT^{a} | NT^{a} |

| | | | | | |
|---|---|---|---|---|---|
| ^{a} NT = Not Tested. | | | | | |

There were no apparent changes in appearance, clarity, and color up to 12 months under the long term storage condition, and all results met the acceptance criteria (Tables 14 to 18).

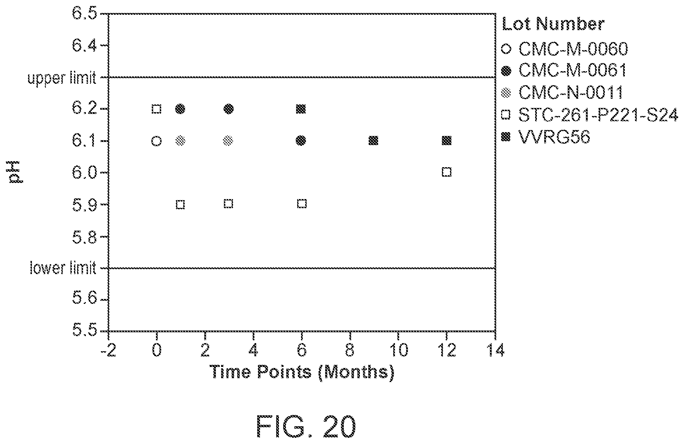

There were no apparent changes in pH up to 12 months under the long term storage condition, and all results met the acceptance criteria of 5.7 - 6.3 (FIG. 20).

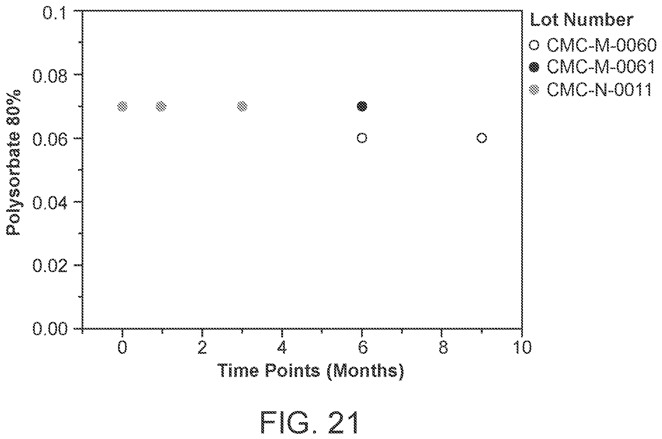

The polysorbate 80 level of the three clinical lots was monitored under the long term storage condition. FIG. 21 shows that there was a small drop from 0.07% to 0.05-0.06% at 6-9 months. Based on the accelerated and stress conditions, likely the polysorbate 80 drop under the long term storage condition will also reach to a plateau of 0.04-0.05% (w/v), which is sufficient to protect the protein from shaking or agitation based on the formulation development studies.

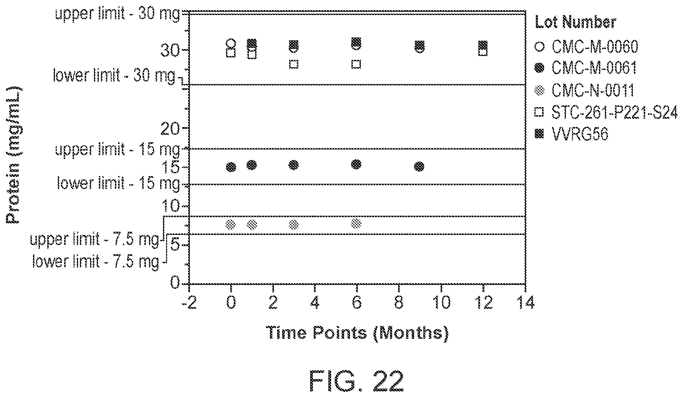

There were no apparent changes in protein concentrations up to 12 months under the long term storage condition, and all results met the acceptance criteria (FIG. 22). Some variations observed for the development lot STC-261-P221-S24 was likely due to assay variation as this lot was tested by a different method (an A280 method by sample dilution) from the other lots.

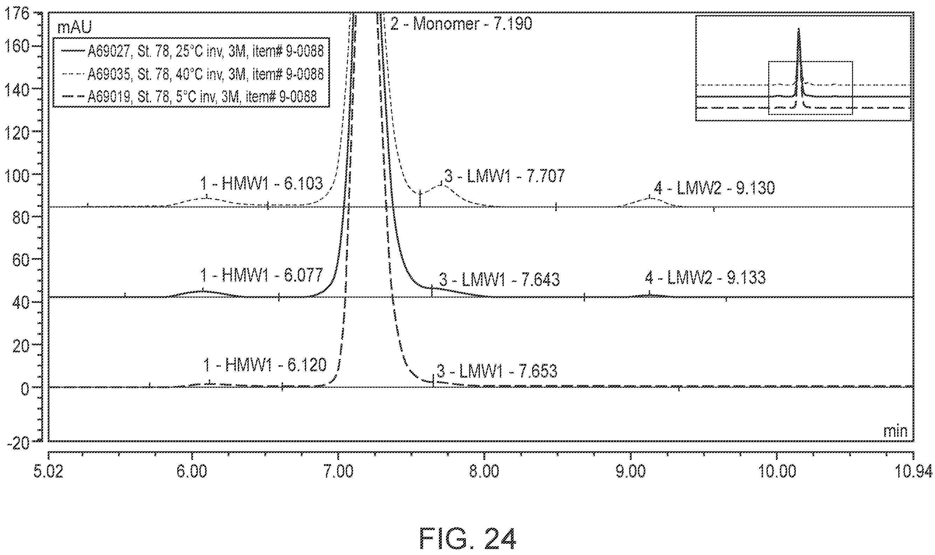

Similar to the accelerated and stress conditions, there were changes in monomer%, HMW%, and LMW% in all the lots under the long term storage condition. The monomer% decreased over time, mainly due to increase of HMW% and LMW% (FIGs 23A, 23B, and 23C). With linear kinetics prediction at 95% confidence, both monomer% and HMW% for all the lots will meet the acceptance criteria at 24 months (FIGs 23A and 23B). An overlay of chromatographic profiles of Lot CMC-M-0061 comparing the changes under the long term storage condition with changes under the accelerated condition (25°C) and the stress condition (40°C) at 3 months is presented in FIG. 24.

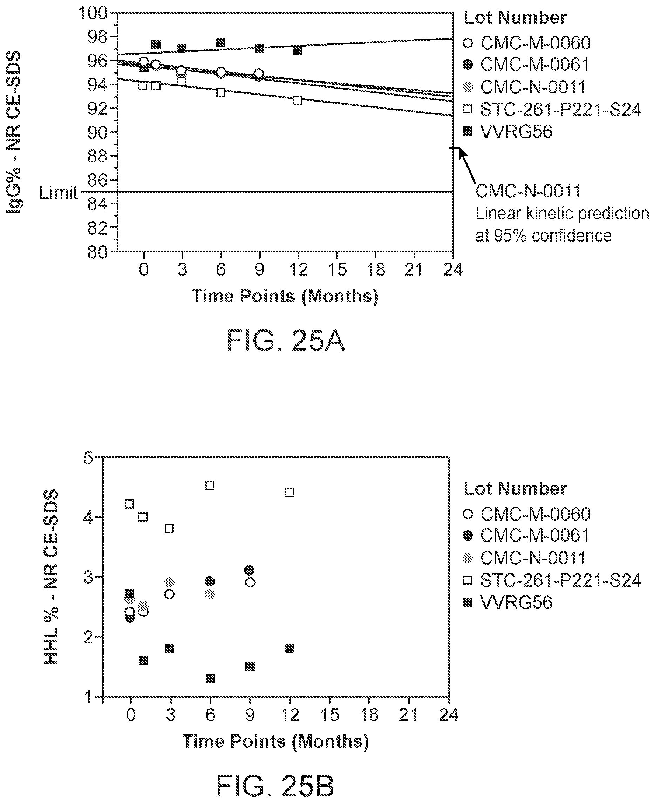

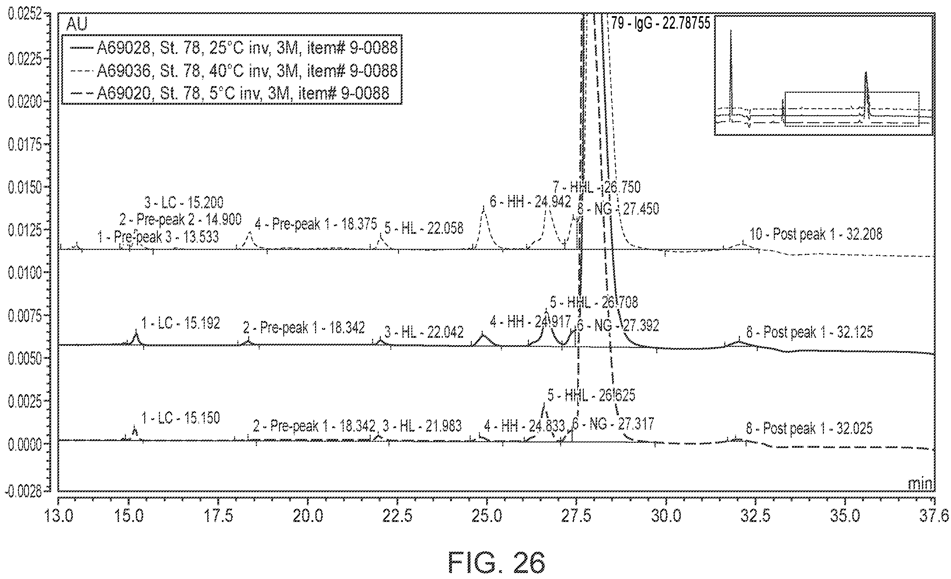

Similar to the accelerated and stress conditions, IgG% decreased under the long term storage condition for all of the lots except lot VVRG56 (FIG. 25A). With linear kinetics prediction at 95% confidence, the IgG% levels for all the lots will meet the acceptance criteria at 24 months (FIG. 25A). There was no apparent trend in change of HHL% over time (FIG. 25B), suggesting that the IgG% decrease was due to increase in other fragments. An overlay of electrophoretic profiles of Lot CMC-M-0061 comparing the changes under the long term storage condition (5°C) with changes under the accelerated condition (25°C) and the stress condition (40°C) at 3 months is presented in FIG. 26, which shows the elution locations and the potential identities of these fragments.

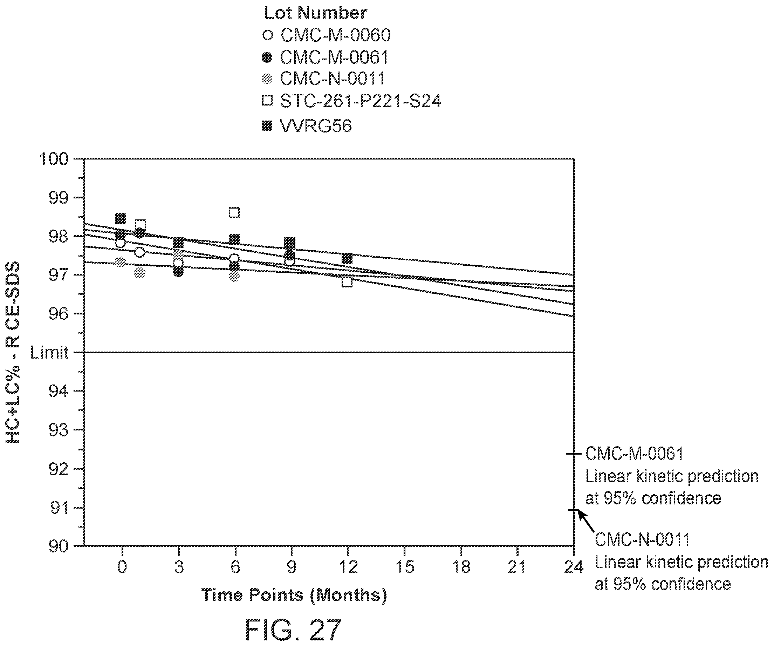

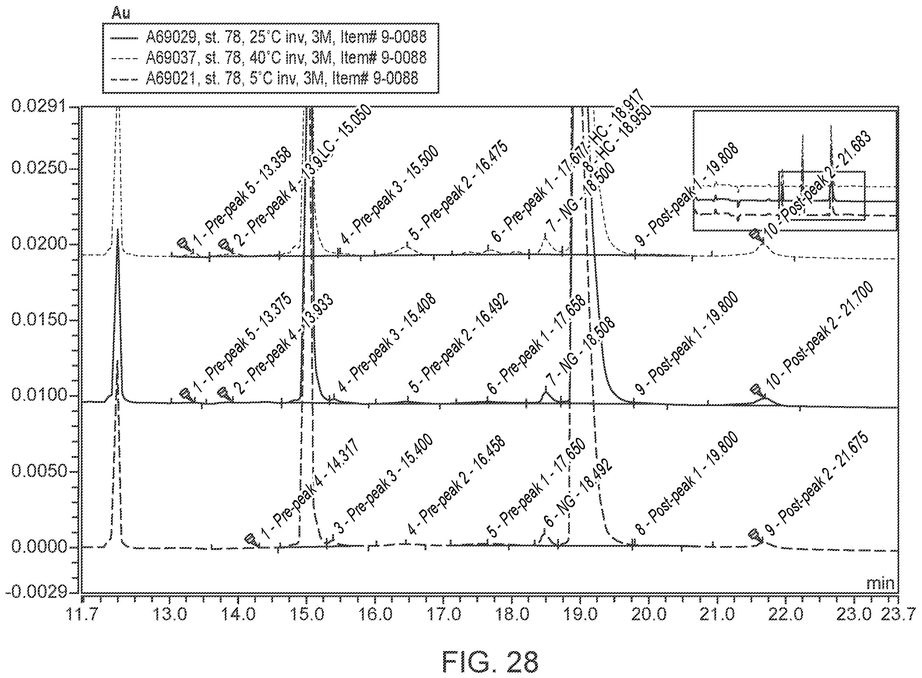

Similar to the accelerated and stress conditions, HC+LC% decreased over time in all the lots due to increase in fragments under the long term storage condition (FIG. 27). An overlay of electrophoretic profiles of Lot CMC-M-0061 comparing the changes under the long term storage condition (5°C) with changes under the accelerated condition (25°C) and the stress condition (40°C) at 3 months is presented in FIG. 28. With linear kinetics prediction at 95% confidence, the HC+LC% levels for all the lots will meet the acceptance criteria at 24 months except lots CMC-M-0061 and CMC-N-0011. Based on the limited data and relatively higher variations of the data for these two lots, the levels at 24 months from the 95% confidence predictions were overestimated, compared to the other lots. In addition, the results under the accelerated conditions (FIG. 12A) indicate that the decrease of HC+LC% reached to a plateau (96%-97%) at 3 months with no apparent change from 3 months to 12 months. Thus, it is reasonable to predict that the HC+LC% levels for lots CMC-M-0061 and CMC-N-0011 will meet the acceptance criteria at 24 months when stored under the long term storage condition.

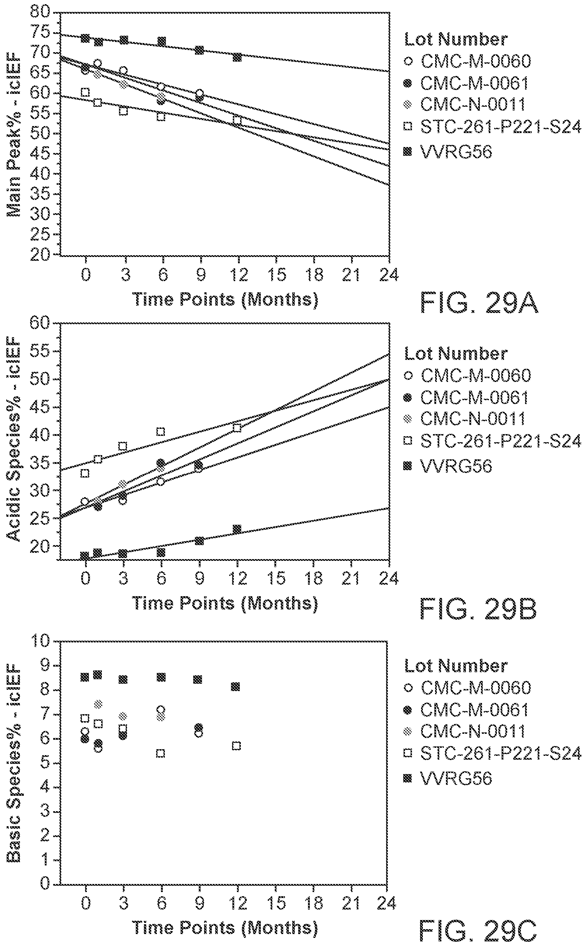

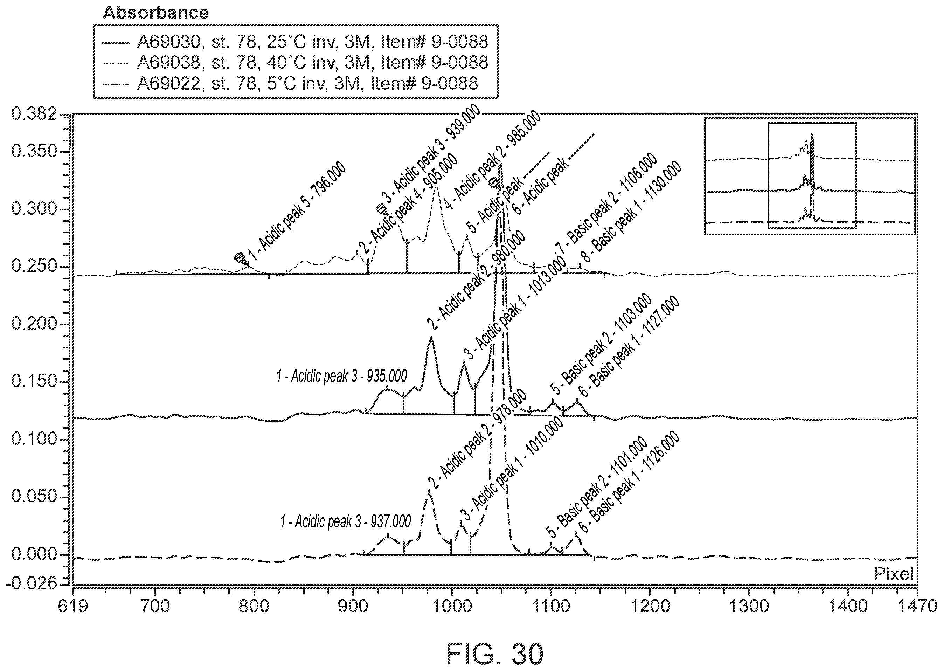

Similar to the accelerated and stress conditions, there were decreases in the main species% (FIG. 29A) and increases in the acidic species% (FIG. 29B) with no apparent changes in the basic species% (FIG. 29C) of all the lots under the long term storage condition. An overlay of electrophoretic profiles of Lot CMC-M-0061 comparing the changes under the long term storage condition (5°C) with changes under the accelerated condition (25°C) and the stress condition (40°C) at 3 months is presented in FIG. 30. It appeared that with decreasing protein concentrations, the main species% drop rate increased, which was not observed at the accelerated or the stress condition. The predicted main species% could decrease to approximate 37% at 24 months for the lot with the fastest kinetics (FIG. 29A). The predicted level of the main species% at 24 months at long term storage condition is not expected to have any impact to the potency of COR-001.

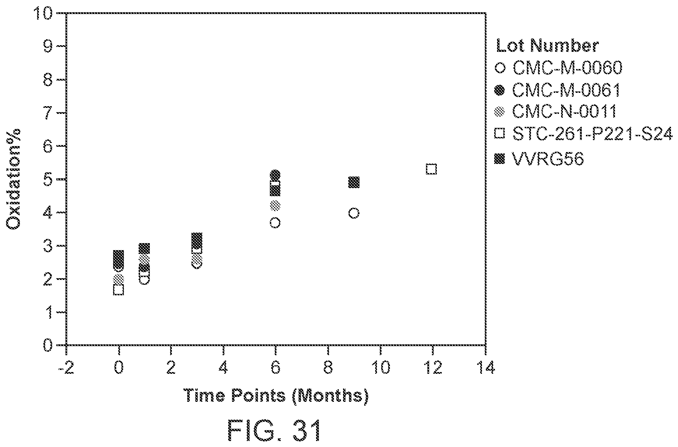

Similar to the accelerated and stress conditions, there was an increase over time in level of oxidation in all the lots under the long term storage condition, and it appeared to reach to a plateau at 6 months (FIG. 31), which is similar to the plateau level of 5-6% at the accelerated and stress conditions (FIGs 16A and 16B). Thus, it is reasonable to predict the maximum oxidation level would remain 5-6% at 24 months for the long term storage condition. No potency impact is expected for the predicted oxidation levels under the long term storage condition for 24 months.

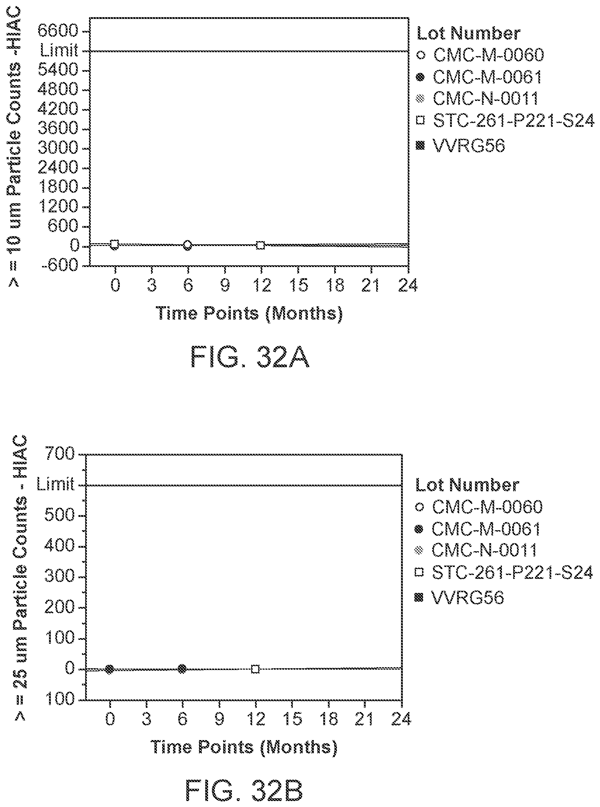

There were no apparent changes in number of sub-visible particulates up to 12 months under the long term storage condition, and all results met the acceptance criteria. With linear kinetics predictions at 95% confidence, the numbers of sub-visible particulates with size ≥ 10 micron and ≥ 25 micron will meet the acceptance criteria at 24 months (FIGs 32A and 32B).

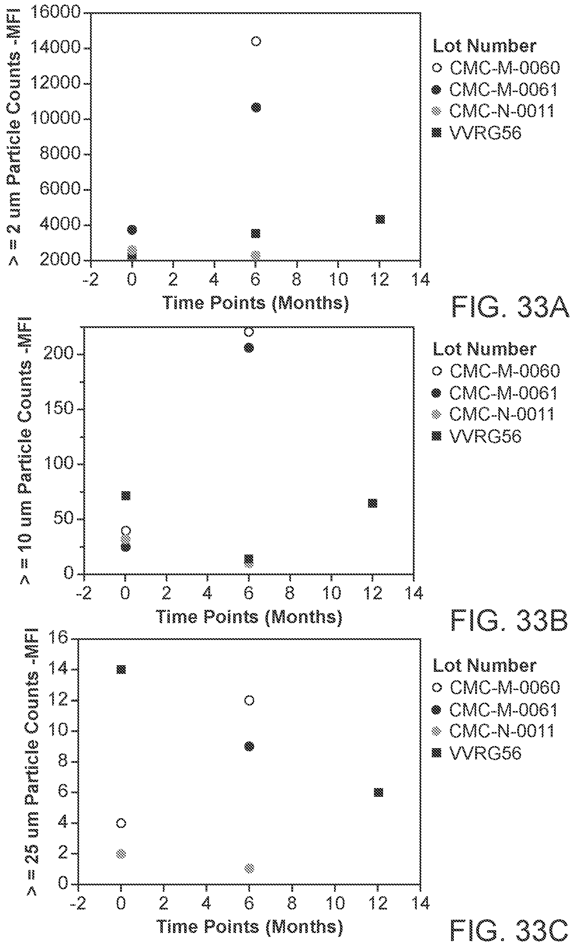

There were changes in the number of sub-visible particles tested by micro-flow imaging (MFI) under the long term storage condition (FIGs 33A, 33B, and 33C). At 6-month time point, the numbers of ≥ 2 micron particles were similar to the numbers under the accelerated condition (FIG. 17A), which suggests the increase of these particles is independent of the storage temperature. A similar level was also observed in the placebo lot (CMC-M-0062). Thus, some of these sub-visible particulates could be due to nature of the formulation buffer components.

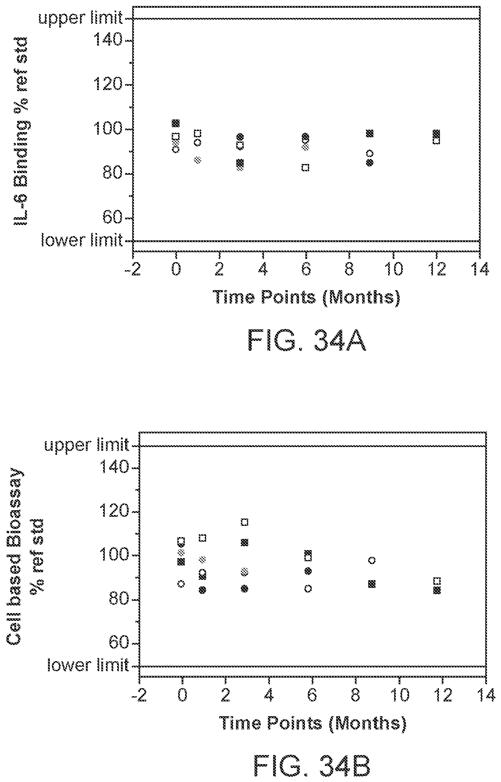

Similar to the accelerated and stress conditions, there were no apparent changes in potency of all the lots tested by IL-6 binding ELISA and HEK Blue Bioassay up to 12 months under the long term storage condition, and all results met the acceptance criteria (FIGs. 34A and 34B).

The microbiological quality in the three clinical drug product lots was monitored by level of endotoxins, as well as container closure integrity testing (CCIT) in lieu of sterility testing. No changes are expected over the long term storage (Tables 14 to 18).

## Claims

1. An antibody formulation, comprising:
a) 10 mg/mL to 50 mg/mL of an anti-IL-6 antibody and 5 mM to 15 mM methionine,
wherein the anti-IL-6 antibody has the heavy chain amino acid sequence of SEQ ID NO: 9 and the light chain amino acid sequence of SEQ ID NO: 10;
b) 5% (w/v) trehalose;
c) 0.07% (w/v) polysorbate 80;
d) 70 mM arginine;
e) 20 mM histidine;
wherein the antibody formulation has a pH of 6.0.

2. The antibody formulation of claim 1, wherein the formulation comprises 10 mg/mL to 30 mg/mL of the anti-IL-6 antibody.

3. The antibody formulation of claim 2, wherein the formulation comprises 10 mg/mL of the anti-IL-6 antibody.

4. The antibody formulation of claim 2, wherein the formulation comprises 15 mg/mL of the anti-IL-6 antibody.

5. The antibody formulation of claim 2, wherein the formulation comprises 30 mg/mL of the anti-IL-6 antibody.

6. The antibody formulation of any of the preceding claims, wherein the formulation is suitable for parenteral administration.

7. The antibody formulation of claim 6, wherein the formulation is suitable for subcutaneous administration.

8. A unit dosage form comprising the antibody formulation of any of the preceding claims.

9. The unit dosage form of claim 8, wherein the unit dosage form comprises 7.5 mg of the anti-IL-6 antibody.

10. The unit dosage form of claim 8, wherein the unit dosage form comprises 15 mg of the anti-IL-6 antibody.

11. The unit dosage form of claim 8, wherein the unit dosage form comprises 30 mg of the anti-IL-6 antibody.

## Patentansprüche

1. Antikörperformulierung, umfassend:
a) 10 mg/ml bis 50 mg/ml eines anti-IL-6-Antikörpers und 5 mM bis 15 mM Methionin, wobei der anti-IL-6-Antikörper die Aminosäuresequenz der schweren Kette von SEQ ID NO: 9 und die Aminosäuresequenz der leichten Kette von SEQ ID NO: 10 aufweist;
b) 5 % (Gew./Vol.) Trehalose;
c) 0,07 % (Gew./Vol.) Polysorbat 80;
d) 70 mM Arginin;
e) 20 mM Histidin;
wobei die Antikörperformulierung einen pH-Wert von 6,0 aufweist.

2. Antikörperformulierung nach Anspruch 1, wobei die Formulierung 10 mg/ml bis 30 mg/ml des anti-IL-6-Antikörpers umfasst.

3. Antikörperformulierung nach Anspruch 2, wobei die Formulierung 10 mg/ml des anti-IL-6-Antikörpers umfasst.

4. Antikörperformulierung nach Anspruch 2, wobei die Formulierung 15 mg/ml des anti-IL-6-Antikörpers umfasst.

5. Antikörperformulierung nach Anspruch 2, wobei die Formulierung 30 mg/ml des anti-IL-6-Antikörpers umfasst.

6. Antikörperformulierung nach einem der vorhergehenden Ansprüche, wobei die Formulierung zur parenteralen Verabreichung geeignet ist.

7. Antikörperformulierung nach Anspruch 6, wobei die Formulierung zur subkutanen Verabreichung geeignet ist.

8. Einheitsdosierungsform, umfassend die Antikörperformulierung nach einem der vorhergehenden Ansprüche.

9. Einheitsdosierungsform nach Anspruch 8, wobei die Einheitsdosierungsform 7,5 mg des anti-IL-6-Antikörpers umfasst.

10. Einheitsdosierungsform nach Anspruch 8, wobei die Einheitsdosierungsform 15 mg des anti-IL-6-Antikörpers umfasst.

11. Einheitsdosierungsform nach Anspruch 8, wobei die Einheitsdosierungsform 30 mg des anti-IL-6-Antikörpers umfasst.

## Revendications

1. Formulation d'anticorps, comprenant :
a) 10 mg/ml à 50 mg/ml d'un anticorps anti-IL-6 et de 5 mM à 15 mM de méthionine, dans lequel l'anticorps anti-IL-6 a la séquence d'acides aminés de chaîne lourde de SEQ ID NO : 9 et la séquence d'acides aminés de chaîne légère de SEQ ID NO : 10 ;
b) 5 % (p/v) de tréhalose ;
c) 0,07 % (p/v) de polysorbate 80 ;
d) Arginine 70 mM ;
e) 20 mM d'histidine ;
dans lequel la formulation d'anticorps a un pH de 6,0.

2. Formulation d'anticorps selon la revendication 1, dans laquelle la formulation comprend 10 mg/ml à 30 mg/ml de l'anticorps anti-IL-6.

3. Formulation d'anticorps selon la revendication 2, dans laquelle la formulation comprend 10 mg/ml de l'anticorps anti-IL-6.

4. Formulation d'anticorps selon la revendication 2, dans laquelle la formulation comprend 15 mg/ml de l'anticorps anti-IL-6.

5. Formulation d'anticorps selon la revendication 2, dans laquelle la formulation comprend 30 mg/ml de l'anticorps anti-IL-6.

6. Formulation d'anticorps selon l'une quelconque des revendications précédentes, dans laquelle la formulation est appropriée pour une administration parentérale.

7. Formulation d'anticorps selon la revendication 6, dans laquelle la formulation est appropriée pour une administration sous-cutanée.

8. Forme posologique unitaire comprenant la formulation d'anticorps selon l'une quelconque des revendications précédentes.

9. Forme posologique unitaire selon la revendication 8, dans laquelle la forme posologique unitaire comprend 7,5 mg de l'anticorps anti-IL-6.

10. Forme posologique unitaire selon la revendication 8, dans laquelle la forme posologique unitaire comprend 15 mg de l'anticorps anti-IL-6.

11. Forme posologique unitaire selon la revendication 8, dans laquelle la forme posologique unitaire comprend 30 mg de l'anticorps anti-IL-6.
